Europäisches Patentamt

European Patent Office

Office européen des brevets

(19)

(11)  **EP 1 548 129 A1**

(12)  # EUROPEAN PATENT APPLICATION
published in accordance with Art. 158(3) EPC

(43) Date of publication:
**29.06.2005 Bulletin 2005/26**

(51) Int Cl.⁷: **C12Q 1/68**, G01N 33/53

(21) Application number: **03798530.6**

(86) International application number:
**PCT/JP2003/012361**

(22) Date of filing: **26.09.2003**

(87) International publication number:
**WO 2004/029293 (08.04.2004 Gazette 2004/15)**

(84) Designated Contracting States:
**AT BE BG CH CY CZ DE DK EE ES FI FR GB GR
HU IE IT LI LU MC NL PT RO SE SI SK TR**

(30) Priority:  **27.09.2002  JP  2002284351
25.09.2003  JP  2003334170**

(71) Applicants:
• **Japan Science and Technology Agency
Kawaguchi-shi, Saitama 332-0012 (JP)**
• **National University Corporation Niigata
University
Niigata-shi, Niigata 950-2181 (JP)**

(72) Inventors:
• **Nawa, Hiroyuki
Niigata-shi, Niigata 951-8104 (JP)**
• **SOMEYA, Toshiyuki
Niigata-shi, Niigata 950-2045 (JP)**
• **MURATAKE, Tatsuyuki
Niigata-shi, Niigata 951-8131 (JP)**
• **KAWAMURA, Meiko
Niigata-shi, Niigata 950-2012 (JP)**

(74) Representative: **Bannerman, David Gardner et al
Withers & Rogers LLP
Goldings House,
2 Hays Lane
London SE1 2HW (GB)**

(54)  **METHOD OF DIAGNOSING INTEGRATION DYSFUNCTION SYNDROME USING BLOOD**

(57)  An object of the present invention is to provide an objective method for diagnosis of schizophrenia using gene expression in mononuclear cells of peripheral blood as an index, and this invention provide a method for diagnosing whether a test subject suffers from schizophrenia or not. The method according to this invention is a method for diagnosing whether a test subject suffers from schizophrenia or not, the method comprising the steps of; obtaining mononuclear cells in blood containing nucleic acid from said subject, measuring the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and determining alteration of the quantified level(s) of the gene(s) in said test subject is statistically significant in comparison with the quantified level(s) of said nucleic acid(s) defining gene(s) exhibiting altered expression by occurrence of schizophrenia or said nucleic acid(s) defining gene(s) exhibiting altered expression by progression of schizophrenia in healthy subjects or schizophrenic patients, thereby diagnosing whether said subject is suffering from schizophrenia or not.

EP 1 548 129 A1

**EP 1 548 129 A1**

**Description**

<u>Background of the Invention</u>

1. Field of the invention

The present invention relates to an objective method for diagnosis of schizophrenia using gene expression in blood as an index.

2. Prior art

Schizophrenia is a mental disorder and about 0.8% of the population suffers from schizophrenia during their youth. For it takes a long time to recover from schizophrenia, social loss caused by schizophrenia is immensely large. Therefore, enthusiastic investigations have been made in many laboratories all over the world to develop therapies and diagnoses for schizophrenia. In particular, significant progress has been made on therapies since development of dopamine receptor antagonists such as chlorpromazine. In contrast, diagnosis of schizophrenia is still classified based on psychological symptoms such as paranoid type, disorganized type, catatonic type and a type incapable to be classified, even in the latest US diagnostic reference "DSMIV". Therefore, diagnosis of schizophrenia finally relies upon subjective diagnosis made by the doctor in attendance, hence, diagnostic accuracy of schizophrenia has not been sufficient. Under such circumstances, chromosomal mapping of gene responsible for schizophrenia and identification of the gene have been made enthusiastically. However, definitive reports have been not made on such gene yet.
In occurrence of schizophrenia, it is known that considerable genetic backgrounds are underlying as risk factors. According to recent progress in genetic analysis, it is revealed that plural genes are involved in schizophrenia (Chiu YF et al., Mol Psychiatry, 2002; 7(6): 658-664; Reference 1). Moreover, from above-mentioned pathognomy, the disease itself is considered to be a complex disease comprising plural disorders (Kirkpatrick B et al., Arch Gen Psychiatry, 2001; 58(2): 165-171; Reference 2).
Recently, it has been recognized that schizophrenia is not only a local disease involved in cerebral nerve system, but a systemic disease involved in immune system etc. Actually, it has been revealed that components of peripheral serum such as epidermal growth factor (Nawa H et al., Mol Psychiatry, 2002; 7(7): 673-682; Reference 3), brain-derived neurotrophic factor (Nawa H et al., Mol Psychiatry, 2002; 110(3): 249-257; Reference 4) and interleukin-8 (Akiyama et al., Schizophr Res., 1995; 37(1): 97-106; Reference 5) exhibit alteration in their expression levels in accordance with occurrence of the disease. Therefore, though patent properties on theses components have been acquired as a diagnostic method for the disease, the accuracy of determination remained to be low.
Moreover, as prior references concerning pathologic diagnosis by multi-measurement using a DNA micro-array or a DNA chip, references of following (6), (7) and (8) can be listed. In addition, literatures on analysis using brain tissue (not using peripheral blood like this invention) by a DNA micro-array or a DNA chip, references of following (9) and (10) can be listed. Furthermore, as prior arts involved in diagnosis of schizophrenia using proteins in blood, references of following (11), (12) and (13) can be listed. Furthermore, in addition to above-mentioned references, Patent Publication No.JP 2001-235470, Patent Publication JP 2001-245661, Patent Application JP 2000-331742, Patent Application JP 2001-228038 (Patent Publication JP 2003-038198) and Patent Application JP 2002-036937 (Patent Publication JP 2003-235557) can be listed.
Reference List

(1) Chiu YF et al., Mol Psychiatry, 2002; 7(6): 658-664.
(2) Kirkpatrick B et al., Arch Gen Psychiatry, 2001; 58(2): 165-171.
(3) Nawa H et al., Mol Psychiatry, 2002; 7(7): 673-682.
(4) Nawa H et al., Mol Psychiatry, 2002; 110(3): 249-257.
(5) Akiyama et al., Schizophr Res., 1995; 37(1): 97-106.
(6) Bertucci F, Viens P, Hingamp P, Nasser V, Houlgatte R, Birnbaum D., Int. J Cancer. 2003; 103(5): 565-571.
(7) Staudt LM.. N. Engl. J. Med., 2003; 348(18): 1777-1785
(8) Bertucci F, Houlgatte R, Nguyen C, Viens P, Jordan BR, Birnbaum D.,Lancet Oncol. 2001; 2(11): 674-682.
(9) Hakak, Y. et al., Proc Natl Acad Sci USA. 2001; 98: 4746-4751
(10)Mirnics, K. et al., Neuron. 2000; 28: 53-67.
(11)Toyooka, K. et al. Neurosci Res., 2003; 46: 299-307.
(12)Futamura, T. et al. Mol Psychiatry., 2002; 7: 673-682.
(13) Toyooka, K. et al. Psychiatry Res., 2002; 110: 249-257.

## Summary of the Invention

The present invention was performed to solve aforementioned problems. An object of the present invention is to provide a reliable method for diagnosis of schizophrenia using small amount of blood as a sample, without forcing risk or pain to a patient.

To solve said problems, the present invention provides a method for diagnosing whether a test subject suffers from schizophrenia or not, the method comprising the steps of;

obtaining mononuclear cells in blood containing nucleic acid from said subject,

measuring the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and

determining alteration of the quantified level(s) of the gene(s) in said test subject is statistically significant in comparison with the quantified level(s) of said nucleic acid(s) defining gene(s) exhibiting altered expression by occurrence of schizophrenia or said nucleic acid(s) defining gene(s) exhibiting altered expression by progression of schizophrenia in healthy subjects or schizophrenic patients, thereby diagnosing whether said subject is suffering from schizophrenia or not.

Moreover, the present invention provides a method for diagnosing whether a test subject suffers from schizophrenia or not, the method comprising the steps of;

obtaining protein derived from mononuclear cells in blood from said test subject,

measuring the content of at least one protein selected from the group consisting of protein(s) (containing its fragment) encoded by nucleic acid(s) defining gene(s) exhibiting altered expression by occurrence of schizophrenia or protein(s) (containing its fragment) encoded by nucleic acid(s) defining gene(s) exhibiting altered expression by progression of schizophrenia in said mononuclear cells, and

determining alteration of the quantified level of the protein(s) in said test subject is statistically significant in comparison with the quantified level(s) of said protein(s) encoded by nucleic acid(s) defining gene(s) exhibiting altered expression by occurrence of schizophrenia or said protein(s) encoded by nucleic acid(s) defining gene(s) exhibiting altered expression by progression of schizophrenia in healthy subjects or schizophrenic patients, thereby diagnosing whether said subject is suffering from schizophrenia or not.

According to the method of this invention, one can diagnose whether a test subject suffers from schizophrenia or not objectively without invasion. The method according to present invention provides a scientific and reliable method compared with conventional subjective methods for diagnosis of schizophrenia, and reinforces conventional diagnostic methods based on psychological symptoms.

Hereafter, the present invention is explained in detail. However, these detailed description of preferred embodiments and examples do not mean any restriction or limitation of the scope of the present invention.

## Brief Description of the Drawings

Fig. 1 is a figure showing distribution of healthy subjects, chronic patients and acute patients in the Mahalanobis cluster analysis.

Fig. 2 is a graph showing the discrimination points analyzed by mRNA levels of kinases/phoaphatases.

Fig. 3 is a graph showing the result of Mahalanobis cluster analysis using kinases/phoaphatases.

## Detailed Description of Preferred Embodiments

The present invention was achieved based on the knowledge obtained by the present inventors that the mRNA expression levels of 132 kinds of genes listed in Table 1 described below are altered by occurrence of schizophrenia with statistical significance. Moreover, mRNAs expression levels of 34 kinds of genes listed in Table 2 described below are altered by progression of schizophrenia with statistical significance. As described in detail in the following examples, the present inventors found 152 genes exhibiting altered expression accompanied with this disease, by comparing the expression levels of about 12000 kinds of genes from peripheral mononuclear cells from acute and chronic schizophrenic patients in hospital with those from normal individuals. Moreover, statistical values measured on these genes are shown in Table 3 described below.

Moreover, among genes listed in Table 1, 24 genes exhibited altered expression in acute non-treated acute schizophrenic patients compared with normal individuals. Furthermore, 111 genes exhibited altered expression in chronic schizophrenic patients in hospital. Meanwhile, 3 genes exhibited altered expression in both patient groups redundantly.

Incidentally, "nucleic acid(s) defining gene(s) exhibiting altered expression by occurrence of schizophrenia" in this specification means nucleic acid(s) described in Table 1, that is defined by the gene name, the protein name which is a gene product and the nucleic acid sequence name, as well as GenBank Accession Nos. described with the names. Among the genes listed in Table 1, No.1 to No.99 and No.129 to No.132 are included in a group of genes exhibiting decreased expression by schizophrenia. Whereas, among the genes listed in Table 1, No.99 to No.128 are included in a group of genes exhibiting increased expression by schizophrenia.

Furthermore, "nucleic acid(s) defining gene(s) exhibiting altered expression by progression of schizophrenia" in this specification means nucleic acid(s) described in Table 2, that is defined by the gene name, the protein name which is s gene product and the nucleic acid sequence name, as well as GenBank Accession Nos. described with the names. As described in the following Example, the genes described in Table 1 and Table 2 consisting of 152 genes in total are useful for the purpose of the invention to diagnose schizophrenia. Meanwhile, statistical values of the 152 genes described in Tables 1 and 2 are disclosed in Table 3, and the genes attached with the mark "NS" in Table 3 are those exhibiting altered expression by progression of schizophrenia and corresponds to genes listed in Table 3.

The method according to this invention is also useful for diagnose whether or not a test animal other than human, especially mammals, suffers from schizophrenia. In the following, a test animal means an animal other than human being, preferably mammals.

That is, this invention also provides a method for diagnosing whether a test animal suffers from schizophrenia or not, the method comprising the steps of;

obtaining mononuclear cells in blood containing nucleic acid from said test animal,

measuring the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and

determining alteration of the quantified level(s) of the gene(s) in said test animal is statistically significant in comparison with the quantified level(s) of said nucleic acid(s) defining gene(s) exhibiting altered expression by occurrence of schizophrenia or said nucleic acid(s) defining gene(s) exhibiting altered expression by progression of schizophrenia in healthy animals or schizophrenic animals, thereby diagnosing whether said animal is suffering from schizophrenia or not.

Moreover, this invention provides a method for diagnosing whether a test animal suffers from schizophrenia or not, the method comprising the steps of;

obtaining protein derived from mononuclear cells in blood from said test animal,

measuring the content of at least one protein selected from the group consisting of protein(s) (containing its fragment) encoded by nucleic acid(s) defining gene(s) exhibiting altered expression by occurrence of schizophrenia or protein(s) (containing its fragment) encoded by nucleic acid(s) defining gene(s) exhibiting altered expression by progression of schizophrenia in said mononuclear cells, and

determining alteration of the quantified level of the protein(s) in said test animal is statistically significant in comparison with the quantified level(s) of said protein(s) encoded by nucleic acid(s) defining gene(s) exhibiting altered expression by occurrence of schizophrenia or said protein(s) encoded by nucleic acid(s) defining gene(s) exhibiting altered expression by progression of schizophrenia in healthy animals or schizophrenic animals, thereby diagnosing whether said animal is suffering from schizophrenia or not.

Table 1

| No. | Genbank | Gene Name |
|---|---|---|
| 1 | AI677689 | Homo sapiens cDNA, 3 end/clone=IMAGE-2329930, EST wd33c06.x1 |
| 2 | Z23115 | Bcl-Xl |
| 3 | X69115 | ZNF37A mRNA for zinc finger |
| 4 | X07024 | HSCCG1 Human X chromsome mRNA for CCG1 protein inv.in cell proliferation |
| 5 | L42243 | Interferon receptor, alternatively spliced interferon receptor (IFNAR2) |
| 6 | HG960-HT960 | Guanine Nucleotide Exchange Factor 1 |
| 7 | Z12173 | Glucosamine-6-sulphatase precursor |
| 8 | X98176 | MACH-beta-1 protein (Caspase 8) |
| 9 | W25921 | 15a11 Homo sapiens cDNA /gb=W25921 /gi =1306044 /ug=Hs.164036 /len=723 |
| 10 | Z35102 | Ndr protein kinase |

Table 1   (continued)

| No. | Genbank | Gene Name |
|---|---|---|
| 11 | U28964 | 14-3-3 protein |
| 12 | X74262 | RbAp48 mRNA encoding retinoblastoma binding protein |
| 13 | Y09568 | SNAP23B protein |
| 14 | AF038960 | SKD1 homolog |
| 15 | AI955897 | Homo sapiens cDNA, 3 end/clone=IMAGE-2509049 ETS wt31b09.×1 |
| 16 | X69086 | Utrophin |
| 17 | M80629 | Cdc2-related protein kinase (CHED) |
| 18 | U12022 | Calmodulin type 1 (CALM1) |
| 19 | X74594 | Rb2/p130 protein |
| 20 | M64174 | Protein-tyrosine kinase JAK1 |
| 21 | M28212 | GTP-binding protein RAB6 |
| 22 | U94333 | Clq/MBL/SPA receptor C1qR(p) |
| 23 | U13948 | Zinc finger/leucine zipper protein (AF10) |
| 24 | U96919 | Inositol polyphosphate 4-phosphatase type I-beta |
| 25 | U26398 | Inositol polyphosphate 4-phosphatase |
| 26 | AF068836 | Cytohesin binding protein HE |
| 27 | L43821 | CAS like protein for enhancer of filamentation (HEF1) |
| 28 | U17032 | Rho GTPase activated protein type 5 (p190-B) |
| 29 | AB022017 | AMP-activated protein kinase alpha-1 |
| 30 | AF038897 | Syntaxin 16 |
| 31 | HG846-HT846 | Cyclophilin-Related Protein |
| 32 | L04288 | Natural killer-tumor recognition sequence |
| 33 | S66213 | Integrin alpha 6B (CD49f) |
| 34 | AF052160 | Homo sapiens clone 24629 mRNA sequence |
| 35 | AB015982 | Protein kinase C Nu (EPK2), EPK2 mRNA for serine/threonine kinase |
| 36 | S79325 | mRNA for SYT.SSX1 translocational target region of human synovial inducible sarcomas [Partial Mutant, 3' genes, 585nt],[synovial sarcomas, mRNA Partial Mutant, 3 genes, 585 nt] |
| 37 | M55536 | Glucose transporter pseudogene |
| 38 | X97674 | Nuclear receptor intermediary activation factor 2 (TIF2), transcriptional intermediary factor 2 |
| 39 | U16028 | CRE-BP1 transcription factor |
| 40 | M27504 | Topoisomerase type II beta (Topo II) |
| 41 | U13044 | Nuclear respiratory factor-2 subunit alpha |
| 42 | AC004990 | PAC clone DJ1185I07 from 7q11.23-q21 |
| 43 | AF048732 | Cyclin T2b |
| 44 | AF061261 | Zinc finger protein type C3H (MBLL) |
| 45 | U29671 | MEK kinase (Mekk) |
| 46 | AB023967 | Rod1 |

Table 1   (continued)

| No. | Genbank | Gene Name |
|---|---|---|
| 47 | U07794 | HSTXK Human tyrosine kinase (TXK) |
| 48 | U48736 | Serine/threonine-protein kinase PRP4h (PRP4h) |
| 49 | AJ001810 | Pre-mRNA cleavage factor I subunit Im |
| 50 | AI961669 | Homo sapiens cDNA, 3 end/clone=IMAGE-2512364 EST wt65e11×1 |
| 51 | Z48579 | Disintegrin-metalloprotease |
| 52 | AF047432 | ADP-ribosylation factor no.6 (ARF6) |
| 53 | U50553 | Helicase like protein 2 containing DEAD/H box, helicase like protein 2 (DDX14) |
| 54 | U57317 | P300/CBP-associated factor (P/CAF) |
| 55 | U08316 | Ribosomal protein S6 kinase, insulin-stimulated protein kinase 1 (ISPK-1) |
| 56 | X77794 | Cyclin G1 |
| 57 | U43083 | Guanine binding protein type q, G alpha-q (Gaq) |
| 58 | AF094481 | Trinucleotide repeat CGG-DNA binding protein p20-CGGBP (CGGBP) |
| 59 | L12002 | Integrin alpha 4 subunit |
| 60 | AB002450 | Chromosome 5q21-22, clone-A3-A |
| 61 | X77723 | Unknown protein of uterine endometrium |
| 62 | M97935 | Transcription factor ISGF-3 (STAT91) |
| 63 | AC002086 | Human PAC clone DJ525N14 from Xq23 |
| 64 | AF100539 | SH2 domain protein 1A isoform B (SH2D1A) |
| 65 | AJ001683 | Killer cell lectin-like receptor NKG2F (NKG2F) |
| 66 | U13896 | Human homolog of Drosophila discs gene, isoform 2 (hdlg-2) |
| 67 | U57452 | Human SNF1-like protein kinase |
| 68 | X14798 | Human DNA for c-ets-1 proto-oncogene |
| 69 | D16815 | EAR-1r |
| 70 | L22075 | Guanine nucleotide regulatory protein (G alpha 13) |
| 71 | L49229 | Retinoblastoma susceptibility protein (RB1) |
| 72 | D13540 | SH-PTP3 for protein-tyrosine phosphatase |
| 73 | W25874 | EST 14e9 Homo sapiens cDNA |
| 74 | AF007111 | MDM2-like p53-binding protein (MDMX) |
| 75 | AA013087 | Homo sapiens cDNA, 5 end/clone=IMAGE-360208 EST ze27c09.r1 |
| 76 | J04101 | Erythroblastosis virus oncogene homolog 1 (ets-1) |
| 77 | X74837 | HUMM9, Man9-mannosidase alpha, class 1A |
| 78 | X65873 | Kinesin heavy chain 5B |
| 79 | U50648 | Interferon-inducible RNA-dependent protein kinase (Pkr) |
| 80 | X15949 | Interferon regulatory factor-2 (IRF-2) |
| 81 | AI189226 | Homo sapiens cDNA, 3 end/clone=IMAGE-1722789 EST qd04h11.×1 |
| 82 | D32039 | Chondroitin sulfate proteoglycan PG-M (bursicon), proteoglycan PG-M(V3) |
| 83 | AL049962 | Homo sapiens mRNA; cDNA DKFZp564P0823 (from clone DKFZp564P0823) |
| 84 | W27675 | 36b3 Homo sapiens cDNA |

Table 1   (continued)

| No. | Genbank | Gene Name |
|---|---|---|
| 85 | AW006742 | Homo sapiens cDNA, 3 end/clone=IMAGE-2489058 EST wr28g10.×1 |
| 86 | AA457029 | Homo sapiens cDNA, 3 end/clone=IMAGE-815515 EST aa 38b10.s1 |
| 87 | J03069 | c-myc proto-oncogene (MYCL2) |
| 88 | Z24459 | Mature T cell proliferation factor c6.1B gene; MTCP1 gene |
| 89 | AB018340 | Homo sapiens mRNA for KIAA0797 protein |
| 90 | X02751 | N-ras |
| 91 | U94747 | WD repeat protein HAN11 |
| 92 | AB028971 | Homo sapiens mRNA for KIAA1048 protein |
| 93 | AB007923 | Homo sapiens mRNA for KIAA0454 protein |
| 94 | AB026891 | Cystine/glutamate transporter |
| 95 | U04735 | Microsomal stress 70 protein ATPase core (stch) |
| 96 | X07767 | cAMP-dependent protein kinase catalytic subunit type alpha (EC 2.7.1.37) |
| 97 | AA058762 | Homo sapiens cDNA, 5 end/clone=IMAGE-487691 |
| 98 | M69177 | Monoamine oxidase B (MAOB) |
| 99 | M20560 | Lipocortin-III (annexins A3) |
| 100 | AB007977 | Homo sapiens chromosome 1 specific transcript KIAA0508 |
| 101 | D10202 | Platelet-activating factor receptor |
| 102 | AL048308 | DKFZp586A2224_s1 Homo sapiens cDNA |
| 103 | X66363 | PCTAIRE-1 for serine/threonine protein kinase |
| 104 | M94345 | Gelsolin; macrophage capping protein; villin |
| 105 | W27466 | 31c9 Homo sapiens cDNA |
| 106 | Y15909 | Diaphanous type 2 isoform 12C protein, dia-156 protein (DIA-156) |
| 107 | X02160 | Insulin receptor precursor |
| 108 | L41827 | Heregulin type 1; sensory and motor neuron-derived factor (HRG alpha) |
| 109 | AF026548 | Branched chain alpha-ketoacid dehydrogenase kinase precursor (BCKD kinase) |
| 110 | X71129 | Electron transfer flavoprotein beta subunit |
| 111 | U88153 | P160 |
| 112 | U08015 | Calciceurin dependently activated T cell nuclear factor (NF-Atc) |
| 113 | AB011135 | Homo sapiens mRNA for KIAA0563 protein |
| 114 | X13839 | Vascular smooth muscle alpha-actin |
| 115 | AF076838 | Rad17-like protein (RAD17) |
| 116 | AI762213 | Homo sapiens cDNA, 3 end/clone=IMAGE-2394055 EST wi54d04.×1 |
| 117 | L77213 | Phosphomevalonate kinase |
| 118 | D17530 | Drebrin E |
| 119 | D64109 | Tob family transducer ERBB2,2 |
| 120 | AB016816 | MASL1 |
| 121 | AB023211 | Homo sapiens mRNA for KIAA0994 protein |
| | 122 AA521060 | Homo sapiens cDNA, 3 end/clone=IMAGE-826408 EST aa71e09.s1 |

Table 1  (continued)

| No. | Genbank | Gene Name |
|-----|---------|-----------|
| 123 | X77094 | Neutrophil cytoplasmic factor type 4 (P40phox) |
| 124 | HG2689-HT2785 | Mucin 5b, Tracheobronchial |
| 125 | AC004893 | Homo sapiens PAC clone DJ0808A01 from 7q21.1-q31.1 |
| 126 | AB028973 | Homo sapiens mRNA for KIAA1050 protein |
| 127 | AI148772 | Homo sapiens cDNA, 3 end/clone=IMAGE-1714897 EST qc69b01.×1 |
| 128 | AL109724 | Homo sapiens mRNA full length insert cDNA clone EUROIMAGE 417629 |
| 129 | L12691 | Defensins alpha 3 (neutrophil peptide-3) |
| 130 | M34379 | Elastase/medullasin |
| 131 | AF002224 | Angelman Syndrome Gene, E6-AP ubiquitin protein ligase 3A (UBE3A) |
| 132 | X69089 | Skeletal muscle 165kD protein |

Table 2

| No. | Genbank | Gene name |
|-----|---------|-----------|
| 1 | AI677689 | Homo sapiens cDNA, 3 end/clone=IMAGE-2329930, EST wd33c06.x1 |
| 2 | X74837 | HUMM9,Man9-mannosidase alpha, class1A |
| 3 | D32039 | Chondroitin sulfate proteoglycan PG-M (bursicon), proteoglycan PG-M(V3) |
| 4 | Z24459 | Mature T cell proliferation factor c6.1B gene; MTCP1 gene |
| 5 | X07767 | cAMP-dependent protein kinase catalytic subunit type alpha (EC 2.7.1.37) |
| 6 | AW003733 | Homo sapiens cDNA, 3 end/clone=IMAGE-2497327 |
| 7 | M20560 | Lipocortin-III (annexins A3) |
| 8 | X66363 | PCTAIRE-1 for serine/threonine protein kinase |
| 9 | AI762213 | Homo sapiens cDNA, 3 end/clone=IMAGE-2394055 EST wi54d04.×1 |
| 10 | AA522537 | Homo sapiens cDNA, 3 end/clone=IMAGE-979142 EST ni38e08.s1 |
| 11 | U66359 | Human T54 protein (T54) |
| 12 | Z80345 | acyl-CoA dehydrogenase; SCAD gene |
| 13 | D17530 | Drebrin E |
| 14 | L36645 | Receptor protein-tyrosine kinase EphA4 (HEK8) |
| 15 | D64109 | Tob family transducer BRBB2,2 |
| 16 | AI039144 | Homo sapiens cDNA, 3 end/clone=IMAGE-1657913 EST ox31b09.s1 |
| 17 | AF000573 | Homogentisate 1,2-dioxygenase |
| 18 | AB016816 | MASL1 |
| 19 | AA528252 | Homo sapiens cDNA, 3 end/clone=IMAGE-965972 EST nh92c11.s1 |
| 20 | M14648 | Cell adhesion protein (vitronectin) receptor alpha subunit (CD51) |
| 21 | AL049435 | Cluster Incl AL049435:Homo sapiens mRNA; cDNA DKFZp586B0220 (from clone DKFZp586B0220) |
| 22 | L40392 | Homo sapiens (clone S164) mRNA, 3 end of cds /cds |
| 23 | AB023226 | Homo sapiens mRNA for KIAA1009 protein |

Table 2   (continued)

| No. | Genbank | Gene name |
|---|---|---|
| 24 | AB018259 | Homo sapiens mRNA for KIAA0716 protein |
| 25 | AJ132099 | Vanin-like gene; vnn1 gene; VNN1 protein |
| 26 | AC004893 | Homo sapiens PAC clone DJ0808A01 from 7q21.1-q31.1 |
| 27 | AF001549 | Human Chromosome 16 BAC clone CIT987SK-A-270G1 |
| 28 | X55544 | Transcriptional factor TREB protein |
| 29 | AB011120 | Homo sapiens mRNA for KIAA0548 protein |
| 30 | U01877 | P300; transcriptional adaptor protein; E1A-binding protein |
| 31 | L25851 | Integrin alpha E precursor (CD103) |
| 32 | AI148772 | Homo sapiens cDNA, 3 end/clone=IMAGE-1714897 EST qc69h01.×1 |
| 33 | L12691 | Defensins alpha 3 (neutrophil peptide-3) |
| 34 | AL036554 | Homo sapiens cDNA, 5 end/clone=DKFZp564J2262·1 |

Table 3

| No. | Genbank | | Healthy subject group | | Threshold of healthy subject group | | Patient group | | Threshold of patient group | | Total of patient group | Welch T test | | | | |
|---|---|---|---|---|---|---|---|---|---|---|---|---|---|---|---|---|
| | | | Mean | S.D. | 5% threshold | 1% threshold | Mean vaue | Standard deviation | 5% threshold | 1% threshold | Ratio of mean value | P Comparison with acute patient group | Signifi-cance | P Comparison with chronic patient group | Signifi-cance | |
| 1 | AI677689 | decrease | 1.12 | 0.81 | -0.18 | -0.76 | 0.92 | 0.61 | 1.90 | 2.33 | 0.82 | 0.049 | CN | 0.947 | | NS |
| 2 | Z23115 | decrease | 1.72 | 0.93 | 0.23 | -0.44 | 0.82 | 0.79 | 2.08 | 2.63 | 0.47 | 0.027 | CN | 0.051 | | |
| 3 | X69115 | decrease | 2.19 | 1.20 | 0.27 | -0.60 | 1.07 | 1.18 | 2.96 | 3.78 | 0.49 | 0.404 | | 0.02 | CS | |
| 4 | X07024 | decrease | 1.64 | 0.74 | 0.46 | -0.07 | 0.76 | 0.44 | 1.47 | 1.78 | 0.47 | 0.043 | CN | 0.008 | CS | |
| 5 | L42243 | decrease | 1.61 | 0.62 | 0.62 | 0.17 | 0.83 | 0.35 | 1.39 | 1.63 | 0.51 | 0.044 | | 0.003 | CS | |
| 6 | HG960-HT960 | decrease | 1.53 | 0.48 | 0.75 | 0.40 | 0.79 | 0.48 | 1.55 | 1.89 | 0.52 | 0.082 | | 0.001 | CS | |
| 7 | Z12173 | decrease | 1.97 | 0.77 | 0.75 | 0.19 | 0.85 | 0.60 | 1.82 | 2.24 | 0.43 | 0.184 | | 0.001 | CS | |
| 8 | X98176 | decrease | 1.83 | 0.58 | 0.91 | 0.49 | 0.80 | 0.53 | 1.64 | 2.01 | 0.44 | 0.056 | | 0 | CS | |
| 9 | W25921 | decrease | 1.48 | 0.47 | 0.74 | 0.40 | 0.81 | 0.43 | 1.51 | 1.81 | 0.55 | 0.209 | | 0.001 | CS | |
| 10 | Z35102 | decrease | 1.53 | 0.36 | 0.95 | 0.69 | 0.80 | 0.36 | 1.37 | 1.63 | 0.52 | 0.048 | | 0 | CS | |
| 11 | U28964 | decrease | 1.57 | 0.51 | 0.75 | 0.38 | 0.84 | 0.33 | 1.36 | 1.59 | 0.53 | 0.046 | | 0.001 | CS | |
| 12 | X74262 | decrease | 1.47 | 0.45 | 0.75 | 0.43 | 0.83 | 0.39 | 1.45 | 1.72 | 0.56 | 0.196 | | 0.001 | CS | |
| 13 | Y09568 | decrease | 1.56 | 0.41 | 0.91 | 0.62 | 0.86 | 0.41 | 1.51 | 1.79 | 0.55 | 0.163 | | 0 | CS | |
| 14 | AF038960 | decrease | 1.61 | 0.60 | 0.66 | 0.22 | 0.83 | 0.48 | 1.61 | 1.95 | 0.52 | 0.247 | | 0.001 | CS | |
| 15 | AI955897 | decrease | 1.88 | 0.64 | 0.85 | 0.39 | 1.00 | 0.52 | 1.83 | 2.19 | 0.53 | 0.255 | | 0.001 | CS | |
| 16 | X69086 | decrease | 1.64 | 0.62 | 0.65 | 0.21 | 0.91 | 0.44 | 1.61 | 1.92 | 0.56 | 0.276 | | 0.003 | CS | |
| 17 | M80629 | decrease | 1.72 | 0.57 | 0.81 | 0.40 | 0.92 | 0.43 | 1.61 | 1.90 | 0.54 | 0.215 | | 0.001 | CS | |
| 18 | U12022 | decrease | 1.61 | 0.48 | 0.84 | 0.49 | 0.80 | 0.36 | 1.38 | 1.63 | 0.50 | 0.041 | | 0 | CS | |
| 19 | X74594 | decrease | 1.77 | 0.47 | 1.02 | 0.68 | 0.91 | 0.48 | 1.68 | 2.01 | 0.51 | 0.1 | | 0 | CS | |
| 20 | M64174 | decrease | 1.76 | 0.46 | 1.01 | 0.68 | 0.92 | 0.40 | 1.56 | 1.84 | 0.52 | 0.058 | | 0 | CS | |
| 21 | M28212 | decrease | 1.72 | 0.43 | 1.04 | 0.73 | 0.87 | 0.46 | 1.61 | 1.94 | 0.51 | 0.137 | | 0 | CS | |
| 22 | U94333 | decrease | 1.49 | 0.44 | 0.79 | 0.48 | 0.81 | 0.47 | 1.56 | 1.89 | 0.55 | 0.331 | | 0 | CS | |
| 23 | U13948 | decrease | 1.41 | 0.29 | 0.95 | 0.74 | 0.80 | 0.37 | 1.38 | 1.64 | 0.56 | 0.07 | | 0 | CS | |
| 24 | U96919 | decrease | 1.72 | 0.86 | 0.34 | -0.28 | 0.86 | 0.46 | 1.61 | 1.93 | 0.50 | 0.198 | | 0.008 | CS | |
| 25 | U26398 | decrease | 1.39 | 0.47 | 0.64 | 0.31 | 0.70 | 0.42 | 1.38 | 1.67 | 0.51 | 0.037 | | 0.001 | CS | |
| 26 | AF068836 | decrease | 1.66 | 0.53 | 0.81 | 0.43 | 0.88 | 0.49 | 1.68 | 2.02 | 0.53 | 0.093 | | 0.002 | CS | |
| 27 | L43821 | decrease | 1.74 | 0.61 | 0.77 | 0.33 | 1.02 | 0.49 | 1.80 | 2.14 | 0.58 | 0.421 | | 0.002 | CS | |
| 28 | U17032 | decrease | 1.63 | 0.79 | 0.37 | -0.20 | 0.94 | 0.61 | 1.91 | 2.34 | 0.58 | 0.497 | | 0.014 | CS | |
| 29 | AB022017 | decrease | 1.90 | 0.99 | 0.32 | -0.39 | 0.74 | 0.51 | 1.55 | 1.91 | 0.39 | 0.029 | CN | 0.006 | CS | |
| 30 | AF038897 | decrease | 1.97 | 0.75 | 0.77 | 0.23 | 0.80 | 0.57 | 1.71 | 2.10 | 0.41 | 0.083 | | 0.001 | CS | |

EP 1 548 129 A1

EP 1 548 129 A1

| No. | Genbank | | Healthy subject group | | Threshold of healthy subject group | | Patient group | | Threshold of patient group | | Total of patient group | Welch T test | | | |
|---|---|---|---|---|---|---|---|---|---|---|---|---|---|---|---|
| | | | Mean | S.D. | 5% threshold | 1% threshold | Mean vaue | Standard deviation | 5% threshold | 1% threshold | Ratio of mean value | P Comparison with acute patient group | Signifi-cance | P Comparison with chronic patient group | Signifi-cance |
| 31 | HG846-HT846 | decrease | 1.92 | 0.88 | 0.51 | −0.12 | 0.84 | 0.54 | 1.70 | 2.08 | 0.43 | 0.152 | | 0.003 | CS |
| 32 | L04288 | decrease | 2.39 | 1.48 | 0.02 | −1.05 | 0.92 | 0.85 | 2.28 | 2.88 | 0.39 | 0.117 | | 0.017 | CS |
| 33 | S66213 | decrease | 2.08 | 1.14 | 0.25 | −0.57 | 0.93 | 0.54 | 1.79 | 2.17 | 0.45 | 0.134 | | 0.009 | CS |
| 34 | AF052160 | decrease | 1.86 | 1.14 | 0.03 | −0.79 | 0.91 | 0.65 | 1.95 | 2.40 | 0.49 | 0.222 | | 0.026 | CS |
| 35 | AB015982 | decrease | 1.64 | 0.64 | 0.62 | 0.16 | 0.91 | 0.58 | 1.84 | 2.25 | 0.55 | 0.418 | | 0.003 | CS |
| 36 | S79325 | decrease | 3.05 | 1.47 | 0.70 | −0.35 | 0.92 | 1.13 | 2.72 | 3.51 | 0.30 | 0.232 | | 0.001 | CS |
| 37 | M55536 | decrease | 2.89 | 1.96 | −0.25 | −1.67 | 0.88 | 1.16 | 2.74 | 3.56 | 0.30 | 0.274 | | 0.007 | CS |
| 38 | X97674 | decrease | 2.64 | 1.56 | 0.15 | −0.98 | 0.91 | 0.98 | 2.47 | 3.15 | 0.34 | 0.238 | | 0.005 | CS |
| 39 | U16028 | decrease | 1.93 | 1.04 | 0.26 | −0.49 | 0.85 | 0.70 | 1.96 | 2.45 | 0.44 | 0.3 | | 0.007 | CS |
| 40 | M27504 | decrease | 2.47 | 1.43 | 0.18 | −0.84 | 1.01 | 1.03 | 2.66 | 3.38 | 0.41 | 0.425 | | 0.006 | CS |
| 41 | U13044 | decrease | 2.47 | 1.72 | −0.29 | −1.53 | 1.14 | 0.87 | 2.53 | 3.14 | 0.46 | 0.478 | | 0.022 | CS |
| 42 | AC004990 | decrease | 2.57 | 1.17 | 0.70 | −0.14 | 0.88 | 1.06 | 2.57 | 3.32 | 0.34 | 0.276 | | 0.001 | CS |
| 43 | AF048732 | decrease | 2.36 | 1.33 | 0.23 | −0.72 | 0.97 | 0.77 | 2.20 | 2.74 | 0.41 | 0.22 | | 0.006 | CS |
| 44 | AF061261 | decrease | 1.95 | 1.14 | 0.12 | −0.70 | 0.87 | 0.74 | 2.06 | 2.59 | 0.45 | 0.384 | | 0.01 | CS |
| 45 | U29671 | decrease | 2.18 | 1.19 | 0.28 | −0.58 | 1.06 | 0.81 | 2.36 | 2.93 | 0.49 | 0.438 | | 0.01 | CS |
| 46 | AB023967 | decrease | 2.07 | 1.34 | −0.07 | −1.04 | 1.11 | 0.63 | 2.12 | 2.56 | 0.53 | 0.354 | | 0.039 | CS |
| 47 | U07794 | decrease | 1.78 | 0.90 | 0.34 | −0.31 | 0.94 | 0.71 | 2.07 | 2.57 | 0.53 | 0.49 | | 0.01 | CS |
| 48 | U48736 | decrease | 1.94 | 1.20 | 0.02 | −0.85 | 1.01 | 0.74 | 2.19 | 2.71 | 0.52 | 0.57 | | 0.02 | CS |
| 49 | AJ001810 | decrease | 1.70 | 0.88 | 0.29 | −0.35 | 0.98 | 0.72 | 2.14 | 2.64 | 0.58 | 0.943 | | 0.009 | CS |
| 50 | AI961669 | decrease | 1.73 | 0.89 | 0.31 | −0.33 | 0.93 | 0.57 | 1.84 | 2.24 | 0.54 | 0.514 | | 0.009 | CS |
| 51 | Z48579 | decrease | 1.76 | 0.92 | 0.29 | −0.38 | 0.77 | 0.65 | 1.81 | 2.27 | 0.44 | 0.259 | | 0.005 | CS |
| 52 | AF047432 | decrease | 1.82 | 0.78 | 0.58 | 0.01 | 0.84 | 0.70 | 1.95 | 2.44 | 0.46 | 0.298 | | 0.002 | CS |
| 53 | U50553 | decrease | 1.61 | 0.63 | 0.59 | 0.14 | 0.92 | 0.69 | 2.03 | 2.51 | 0.57 | 0.554 | | 0.005 | CS |
| 54 | U57317 | decrease | 2.30 | 1.12 | 0.51 | −0.30 | 0.97 | 0.72 | 2.12 | 2.62 | 0.42 | 0.144 | | 0.005 | CS |
| 55 | U08316 | decrease | 1.91 | 0.94 | 0.40 | −0.28 | 0.89 | 0.47 | 1.64 | 1.97 | 0.47 | 0.12 | | 0.007 | CS |
| 56 | X77794 | decrease | 1.82 | 0.77 | 0.60 | 0.05 | 1.01 | 0.58 | 1.93 | 2.34 | 0.55 | 0.348 | | 0.005 | CS |
| 57 | U43083 | decrease | 1.94 | 0.90 | 0.50 | −0.15 | 0.91 | 0.53 | 1.76 | 2.13 | 0.47 | 0.193 | | 0.004 | CS |
| 58 | AF094481 | decrease | 1.63 | 0.68 | 0.54 | 0.05 | 0.92 | 0.41 | 1.58 | 1.86 | 0.57 | 0.243 | | 0.005 | CS |
| 59 | L12002 | decrease | 1.70 | 0.85 | 0.34 | −0.28 | 0.86 | 0.44 | 1.57 | 1.88 | 0.51 | 0.195 | | 0.009 | CS |

| No. | Genbank | | Healthy subject group | | Threshold of healthy subject group | | Patient group | | Threshold of patient group | | Total of patient group | Welch T test | | | | |
|---|---|---|---|---|---|---|---|---|---|---|---|---|---|---|---|---|
| | | | Mean | S.D. | 5% threshold | 1% threshold | Mean value | Standard deviation | 5% threshold | 1% threshold | Ratio of mean value | P Comparison with acute patient group | Signifi-cance | P Comparison with chronic patient group | Signifi-cance | |
| 60 | AB002450 | decrease | 1.81 | 1.17 | −0.06 | −0.90 | 0.93 | 0.64 | 1.96 | 2.41 | 0.51 | 0.316 | | 0.037 | CS | |
| 61 | X77723 | decrease | 1.81 | 0.72 | 0.66 | 0.14 | 0.93 | 0.79 | 2.19 | 2.74 | 0.51 | 0.593 | | 0.001 | CS | |
| 62 | M97935 | decrease | 2.69 | 1.76 | −0.12 | −1.39 | 1.06 | 0.99 | 2.65 | 3.34 | 0.39 | 0.321 | | 0.012 | CS | |
| 63 | AC002086 | decrease | 2.08 | 1.07 | 0.36 | −0.41 | 0.85 | 0.58 | 1.78 | 2.18 | 0.41 | 0.139 | | 0.004 | CS | |
| 64 | AF100539 | decrease | 1.89 | 0.88 | 0.48 | −0.15 | 0.90 | 0.59 | 1.85 | 2.26 | 0.48 | 0.092 | | 0.007 | CS | |
| 65 | AJ001683 | decrease | 1.86 | 0.82 | 0.54 | −0.05 | 1.07 | 1.01 | 2.67 | 3.38 | 0.57 | 0.756 | | 0.007 | CS | |
| 66 | U13896 | decrease | 1.71 | 0.78 | 0.45 | −0.11 | 0.96 | 0.87 | 2.35 | 2.96 | 0.56 | 0.61 | | 0.012 | CS | |
| 67 | U57452 | decrease | 3.12 | 2.13 | −0.29 | −1.83 | 0.95 | 0.65 | 1.98 | 2.44 | 0.30 | 0.041 | CN | 0.013 | CS | |
| 68 | X14798 | decrease | 2.40 | 1.46 | 0.07 | −0.98 | 0.92 | 0.63 | 1.93 | 2.38 | 0.38 | 0.11 | | 0.01 | CS | |
| 69 | D16815 | decrease | 2.36 | 1.40 | 0.11 | −0.90 | 1.01 | 0.83 | 2.33 | 2.91 | 0.43 | 0.274 | | 0.011 | CS | |
| 70 | L22075 | decrease | 4.79 | 4.19 | −1.92 | −4.94 | 0.87 | 1.40 | 3.10 | 4.08 | 0.18 | 0.1 | | 0.016 | CS | |
| 71 | L49229 | decrease | 3.39 | 2.63 | −0.82 | −2.72 | 1.10 | 1.78 | 3.94 | 5.19 | 0.32 | 0.44 | | 0.016 | CS | |
| 72 | D13540 | decrease | 3.60 | 2.27 | −0.02 | −1.65 | 0.99 | 2.09 | 4.34 | 5.81 | 0.27 | 0.399 | | 0.004 | CS | |
| 73 | W25874 | decrease | 2.38 | 1.33 | 0.25 | −0.71 | 0.70 | 0.99 | 2.28 | 2.98 | 0.29 | 0.146 | | 0.002 | CS | |
| 74 | AF007111 | decrease | 2.10 | 1.22 | 0.15 | −0.73 | 1.00 | 0.96 | 2.54 | 3.21 | 0.48 | 0.424 | | 0.016 | CS | |
| 75 | AA013087 | decrease | 2.94 | 1.18 | 1.05 | 0.20 | 0.65 | 0.99 | 2.24 | 2.93 | 0.22 | 0.115 | | 0 | CS | |
| 76 | J04101 | decrease | 3.05 | 1.70 | 0.33 | −0.90 | 0.72 | 1.04 | 2.38 | 3.11 | 0.24 | 0.089 | | 0.001 | CS | |
| 77 | X74837 | decrease | 1.93 | 1.09 | 0.18 | −0.61 | 0.79 | 0.48 | 1.56 | 1.90 | 0.41 | 0.144 | | 0.006 | CS | NS |
| 78 | X65873 | decrease | 1.80 | 0.63 | 0.80 | 0.34 | 0.79 | 0.53 | 1.63 | 2.00 | 0.44 | 0.054 | | 0 | CS | |
| 79 | U50648 | decrease | 1.68 | 0.73 | 0.51 | −0.02 | 0.86 | 0.35 | 1.42 | 1.66 | 0.51 | 0.113 | | 0.004 | CS | |
| 80 | X15949 | decrease | 1.50 | 0.55 | 0.63 | 0.23 | 0.79 | 0.39 | 1.42 | 1.70 | 0.53 | 0.095 | | 0.003 | CS | |
| 81 | AI189226 | decrease | 1.70 | 0.93 | 0.20 | −0.47 | 0.85 | 0.43 | 1.54 | 1.84 | 0.50 | 0.047 | | 0.027 | CS | |
| 82 | D32039 | decrease | 1.30 | 0.38 | 0.69 | 0.42 | 0.83 | 0.58 | 1.76 | 2.16 | 0.64 | 0.878 | | 0.002 | CS | NS |
| 83 | AL049962 | decrease | 1.46 | 0.51 | 0.63 | 0.26 | 0.83 | 0.47 | 1.57 | 1.90 | 0.57 | 0.346 | | 0.002 | CS | |
| 84 | W27675 | decrease | 1.34 | 0.24 | 0.96 | 0.79 | 0.65 | 0.53 | 1.49 | 1.86 | 0.48 | 0.131 | | 0 | CS | |
| 85 | AW006742 | decrease | 1.50 | 0.41 | 0.85 | 0.55 | 0.77 | 0.55 | 1.64 | 2.03 | 0.51 | 0.157 | | 0.001 | CS | |

| No. | Genbank | | Healthy subject group | | Threshold of healthy subject group | | Patient group | | Threshold of patient group | | Total of patient group | Welch T test | | | | |
|---|---|---|---|---|---|---|---|---|---|---|---|---|---|---|---|---|
| | | | Mean | S.D. | 5% threshold | 1% threshold | Mean vaue | Standard deviation | 5% threshold | 1% threshold | Ratio of mean value | P Comparison with acute patient group | Signifi-cance | P Comparison with chronic patient group | Signifi-cance | |
| 86 | AA457029 | decrease | 1.36 | 0.34 | 0.81 | 0.56 | 0.80 | 0.42 | 1.47 | 1.77 | 0.59 | 0.386 | | 0 | CS | |
| 87 | J03069 | decrease | 1.43 | 0.47 | 0.68 | 0.33 | 0.86 | 0.50 | 1.67 | 2.02 | 0.60 | 0.492 | | 0.002 | CS | |
| 88 | Z24459 | decrease | 1.43 | 0.30 | 0.95 | 0.74 | 0.87 | 0.38 | 1.49 | 1.76 | 0.61 | 0.675 | | 0 | CS | NS |
| 89 | AB018340 | decrease | 1.13 | 0.17 | 0.85 | 0.73 | 0.72 | 0.40 | 1.36 | 1.63 | 0.64 | 0.89 | | 0 | CS | |
| 90 | X02751 | decrease | 1.75 | 0.97 | 0.19 | −0.51 | 0.81 | 0.72 | 1.96 | 2.46 | 0.46 | 0.186 | | 0.017 | CS | |
| 91 | U94747 | decrease | 1.44 | 0.79 | 0.17 | −0.40 | 0.72 | 0.50 | 1.52 | 1.88 | 0.50 | 0.084 | | 0.025 | CS | |
| 92 | AB028971 | decrease | 1.22 | 0.24 | 0.84 | 0.67 | 0.72 | 0.47 | 1.47 | 1.80 | 0.59 | 0.025 | CN | 0.014 | | |
| 93 | AB007923 | decrease | 1.54 | 0.56 | 0.64 | 0.23 | 0.82 | 0.41 | 1.47 | 1.75 | 0.53 | 0.027 | | 0.003 | CS | |
| 94 | AB026891 | decrease | 1.73 | 0.99 | 0.14 | −0.58 | 0.96 | 0.60 | 1.92 | 2.34 | 0.55 | 0.4 | | 0.028 | CS | |
| 95 | U04735 | decrease | 1.70 | 0.63 | 0.70 | 0.25 | 0.95 | 0.43 | 1.63 | 1.93 | 0.56 | 0.164 | | 0.003 | CS | |
| 96 | X07767 | decrease | 1.38 | 0.76 | 0.16 | −0.39 | 0.83 | 0.70 | 1.94 | 2.42 | 0.60 | 0.908 | | 0.031 | CS | NS |
| 97 | AW003733 | decrease | 1.08 | 0.36 | 0.51 | 0.25 | 0.84 | 0.63 | 1.85 | 2.29 | 0.77 | 0.147 | | 0.037 | | NS |
| 98 | AA058762 | decrease | 1.38 | 0.73 | 0.21 | −0.31 | 0.66 | 0.63 | 1.67 | 2.12 | 0.47 | 0.202 | | 0.017 | CS | |
| 99 | M69177 | decrease | 3.57 | 2.74 | −0.81 | −2.79 | 1.32 | 1.83 | 4.25 | 5.53 | 0.37 | 0.35 | | 0.025 | CS | |
| 100 | M20560 | increaase | 1.97 | 1.48 | 4.34 | 5.41 | 2.20 | 2.70 | −2.12 | −4.01 | 1.11 | 0.037 | CN | 0.391 | | NS |
| 101 | AB007977 | increaase | 0.69 | 0.73 | −1.33 | −1.00 | 1.65 | 1.14 | −0.18 | −0.97 | 2.39 | 0.566 | | 0.007 | CS | |
| 102 | D10202 | increaase | 0.58 | 0.65 | −1.37 | −0.93 | 2.09 | 1.67 | −0.58 | −1.74 | 3.60 | 0.12 | | 0.011 | CS | |
| 103 | AL048308 | increaase | 0.66 | 0.39 | −0.39 | −0.25 | 1.24 | 0.61 | 0.27 | −0.15 | 1.89 | 0.362 | | 0.003 | CS | |
| 104 | X66363 | increaase | 0.34 | 0.64 | 2.14 | −1.15 | 2.32 | 1.98 | −0.84 | −2.23 | 6.86 | 0.271 | | 0.001 | CS | NS |
| 105 | M94345 | increaase | 0.78 | 0.28 | −0.23 | 0.13 | 1.30 | 0.48 | 0.53 | 0.20 | 1.66 | 0.035 | CN | 0.015 | | |
| 106 | W27466 | increaase | 0.76 | 0.24 | −0.19 | 0.20 | 1.28 | 0.43 | 0.60 | 0.30 | 1.69 | 0.003 | CN | 0.012 | | |
| 107 | Y15909 | increaase | 0.68 | 0.39 | −0.37 | −0.22 | 1.27 | 0.45 | 0.55 | 0.24 | 1.87 | 0.022 | CN | 0.008 | | |
| 108 | X02160 | increaase | 0.57 | 0.42 | −0.49 | −0.40 | 1.02 | 0.29 | 0.56 | 0.36 | 1.78 | 0.002 | CN | 0.037 | | |
| 109 | L41827 | increase | 0.70 | 0.37 | −0.35 | −0.16 | 1.40 | 0.61 | 0.42 | −0.01 | 2.00 | 0.008 | CN | 0.011 | | |
| 110 | AF026548 | increaase | 0.54 | 0.41 | −0.49 | −0.41 | 1.28 | 0.51 | 0.47 | 0.12 | 2.36 | 0.054 | | 0.001 | CS | |
| 111 | X71129 | increaase | 0.70 | 0.44 | −0.46 | −0.33 | 1.45 | 0.80 | 0.18 | −0.38 | 2.09 | 0.144 | | 0.008 | CS | |
| 112 | U88153 | increaase | 0.70 | 0.41 | −0.41 | −0.26 | 1.28 | 0.65 | 0.24 | −0.22 | 1.84 | 0.211 | | 0.009 | CS | |
| 113 | U08015 | increase | 0.80 | 0.24 | −0.19 | 0.25 | 1.62 | 0.83 | 0.29 | −0.29 | 2.02 | 0.078 | | 0.007 | CS | |
| 114 | AB011135 | increase | 0.71 | 0.34 | −0.31 | −0.09 | 1.27 | 0.58 | 0.34 | −0.07 | 1.79 | 0.44 | | 0.002 | CS | |
| 115 | X13839 | increase | 0.65 | 0.41 | −0.42 | −0.30 | 1.53 | 0.81 | 0.24 | −0.33 | 2.36 | 0.239 | | 0.001 | CS | |

| No. | Genbank | | Healthy subject group | | Threshold of healthy subject group | | Patient group | | Threshold of patient group | | Total of patient group | Welch T test | | | | |
|---|---|---|---|---|---|---|---|---|---|---|---|---|---|---|---|---|
| | | | Mean | S.D. | 5% threshold | 1% threshold | Mean vaue | Standard deviation | 5% threshold | 1% threshold | Ratio of mean value | P Comparison with acute patient group | Signifi-cance | P Comparison with chronic patient group | Signifi-cance | |
| 116 | AF076838 | increaase | 0.65 | 0.53 | -0.66 | -0.57 | 1.37 | 0.67 | 0.31 | -0.16 | 2.10 | 0.127 | | 0.005 | CS | |
| 117 | AI762213 | increaase | 0.81 | 0.22 | -0.16 | 0.31 | 1.35 | 0.86 | -0.03 | -0.63 | 1.66 | 0.391 | | 0.006 | CS | NS |
| 118 | AA522537 | increaase | 0.89 | 0.42 | -0.38 | -0.10 | 1.35 | 0.94 | -0.15 | -0.81 | 1.51 | 0.6 | | 0.052 | | NS |
| 119 | U66359 | increaase | 0.76 | 0.42 | -0.40 | -0.21 | 1.00 | 0.54 | 0.13 | -0.25 | 1.30 | 0.18 | | 0.06 | | NS |
| 120 | Z80345 | increaase | 1.08 | 1.03 | -1.60 | -1.32 | 1.06 | 0.60 | 0.11 | -0.31 | 0.98 | 0.121 | | 0.575 | | NS |
| 121 | L77213 | increaase | 0.68 | 0.55 | -0.69 | -0.59 | 1.26 | 0.96 | -0.28 | -0.96 | 1.86 | 0.016 | CN | 0.168 | | |
| 122 | D17530 | increaase | 1.05 | 0.62 | -0.62 | -0.39 | 0.89 | 0.64 | -0.13 | -0.58 | 0.85 | 0.015 | CN | 0.848 | | NS |
| 123 | L36645 | increaase | 0.78 | 0.46 | -0.46 | -0.29 | 1.06 | 0.55 | 0.17 | -0.21 | 1.36 | 0.473 | | 0.043 | | NS |
| 124 | D64109 | increaase | 0.60 | 0.52 | -0.71 | -0.61 | 1.41 | 1.11 | -0.36 | -1.14 | 2.35 | 0.771 | | 0.01 | CS | NS |
| 125 | AI039144 | increaase | 1.11 | 0.82 | -0.95 | -0.79 | 1.40 | 1.29 | -0.67 | -1.57 | 1.26 | 0.188 | | 0.241 | | |
| 126 | AF000573 | increaase | 1.27 | 0.72 | -0.70 | -0.41 | 1.33 | 1.02 | -0.30 | -1.02 | 1.05 | 0.046 | | 0.426 | | NS |
| 127 | AB016816 | increaase | 0.66 | 0.78 | -1.90 | -1.16 | 1.54 | 1.66 | -1.12 | -2.29 | 2.34 | 0.205 | | 0.019 | CS | NS |
| 128 | AB023211 | increaase | 0.72 | 0.34 | -0.30 | -0.06 | 1.29 | 0.80 | 0.01 | -0.55 | 1.79 | 0.044 | CN | 0.149 | | |
| 129 | AA521060 | increaase | 0.99 | 0.31 | -0.24 | 0.28 | 1.37 | 0.68 | 0.28 | -0.19 | 1.38 | 0.005 | CN | 0.565 | | |
| 130 | X77094 | increaase | 0.90 | 0.41 | -0.36 | -0.05 | 1.64 | 1.11 | -0.13 | -0.91 | 1.83 | 0.035 | CN | 0.179 | | |
| 131 | HG2689-HT27 | increaase | 0.72 | 0.48 | -0.51 | -0.39 | 1.32 | 0.72 | 0.17 | -0.33 | 1.84 | 0.005 | CN | 0.135 | | |
| 132 | AA528252 | increaase | 1.15 | 0.48 | -0.41 | 0.02 | 1.07 | 0.62 | 0.08 | -0.36 | 0.93 | 0.013 | | 0.161 | | NS |
| 133 | M14648 | increaase | 1.07 | 0.42 | -0.35 | 0.09 | 1.02 | 0.63 | 0.01 | -0.42 | 0.95 | 0.08 | | 0.064 | | NS |
| 134 | AL049435 | increaase | 1.07 | 0.34 | -0.26 | 0.29 | 1.15 | 0.64 | 0.13 | -0.32 | 1.07 | 0.036 | | 0.119 | | NS |
| 135 | L40392 | increaase | 1.13 | 0.22 | -0.16 | 0.61 | 1.05 | 0.64 | 0.02 | -0.43 | 0.93 | 0.08 | | 0.008 | | NS |
| 136 | AB023226 | increaase | 1.05 | 0.41 | -0.34 | 0.09 | 0.96 | 0.61 | -0.01 | -0.43 | 0.92 | 0.044 | | 0.106 | | NS |
| 137 | AB018259 | increaase | 0.84 | 0.69 | -0.90 | -0.77 | 1.03 | 0.52 | 0.20 | -0.17 | 1.23 | 0.012 | | 0.791 | | NS |
| 138 | AJ132099 | increaase | 1.22 | 0.54 | -0.47 | -0.04 | 1.08 | 0.72 | -0.07 | -0.57 | 0.88 | 0.016 | | 0.027 | | NS |
| 139 | AC004893 | increaase | 0.88 | 0.29 | -0.23 | 0.21 | 1.16 | 0.70 | 0.04 | -0.45 | 1.31 | 0.034 | CN | 0.977 | | NS |
| 140 | AB028973 | increase | 0.73 | 0.47 | -0.49 | -0.36 | 1.15 | 0.52 | 0.32 | -0.05 | 1.57 | 0.013 | CN | 0.225 | | |
| 141 | AF001549 | increaase | 1.04 | 0.58 | -0.55 | -0.30 | 1.02 | 0.78 | -0.22 | -0.77 | 0.98 | 0.135 | | 0.294 | | NS |
| 142 | X55544 | increaase | 1.05 | 0.42 | -0.35 | 0.06 | 0.89 | 0.54 | 0.01 | -0.37 | 0.85 | 0.11 | | 0.082 | | NS |
| 143 | AB011120 | increaase | 0.96 | 0.40 | -0.34 | 0.04 | 0.97 | 0.65 | -0.07 | -0.52 | 1.01 | 0.005 | | 0.117 | | NS |
| 144 | U01877 | increaase | 1.28 | 0.94 | -1.08 | -0.89 | 1.36 | 0.99 | -0.23 | -0.93 | 1.06 | 0.095 | | 0.492 | | NS |
| 145 | L25851 | increaase | 1.05 | 0.40 | -0.33 | 0.12 | 1.10 | 0.80 | -0.17 | -0.73 | 1.05 | 0.001 | | 0.477 | | NS |

EP 1 548 129 A1

| No. | Genbank | | Healthy subject group | | Threshold of healthy subject group | | Patient group | | Threshold of patient group | | Total of patient group | Welch T test | | | |
|---|---|---|---|---|---|---|---|---|---|---|---|---|---|---|---|
| | | | Mean | S.D. | 5% threshold | 1% threshold | Mean vaue | Standard deviation | 5% threshold | 1% threshold | Ratio of mean value | P Comparison with acute patient group | Signifi-cance | P Comparison with chronic patient group | Signifi-cance |
| 146 | AI148772 | increaase | 0.70 | 0.54 | -0.65 | -0.55 | 1.23 | 1.00 | -0.37 | -1.08 | 1.75 | 0.006 | CN | 0.891 | NS |
| 147 | AL109724 | increaase | 0.79 | 0.73 | -1.09 | -0.91 | 1.45 | 1.13 | -0.37 | -1.16 | 1.84 | 0.003 | CN | 0.316 | NS |
| 148 | L12691 | decrease | 1.64 | 1.07 | -0.08 | -0.85 | 1.84 | 2.30 | 5.52 | 7.13 | 1.12 | 0.007 | CN | 0.325 | NS |
| 149 | AL036554 | decrease | 1.26 | 0.82 | -0.05 | -0.64 | 1.72 | 2.30 | 5.39 | 7.00 | 1.36 | 0.016 | CN | 0.202 | |
| 150 | M34379 | decrease | 1.71 | 1.01 | 0.08 | -0.65 | 2.25 | 3.42 | 7.72 | 10.12 | 1.32 | 0.007 | CN | 0.278 | |
| 151 | AF002224 | decrease | 9.96 | 11.12 | -7.83 | -15.84 | 2.53 | 6.68 | 13.22 | 17.90 | 0.25 | 0.613 | | 0.04 | CS |
| 152 | X69089 | decrease | 5.03 | 4.83 | -2.70 | -6.18 | 1.87 | 2.35 | 5.63 | 7.27 | 0.37 | 0.578 | | 0.041 | CS |
| | | | 253.80 | 77.84 | | | 161.02 | 54.81 | | | | | | | |

It was determined that the genes listed in Table 1 are particularly useful as an index for diagnosis of schizophrenia, in consideration of all of the following criteria on a DNA chip:

(1) having reliable signal intensity,
(2) exhibiting gene-expression alteration ratio of more than two-folds or less than half, wherein the gene-expression alteration ratio is determined by either one of "comparison of the average expression levels between the non-treated acute patient group and the healthy subject group" or "comparison of the average expression levels between the chronic patient group in hospital and the healthy subject group", (3) exhibiting p-value of 0.05 or less obtained from test of difference in the average level of gene expression between the patient group and the normal group. Note that the term "p-value" is the probability of measuring a certain statistical value according to null hypothesis. Therefore Table 1 consists of sum of the list of genes obtained by two kinds of statistic comparison as follows: (1) comparison of the average expression levels of genes between the non-treated acute patient group and the healthy subject group, and (2) comparison of the average expression levels of genes between the chronic patient group in hospital and the healthy subject group. Therefore, when the patient group was defined as the sum of the non-treated acute patient group and the chronic patient group in hospital, comparison between the average expression levels of genes in the patient group and that in the healthy subject group does not necessarily result in the p value (significance in probability) of less than 0.05.

It was shown that the genes listed in Table 2 can satidfiy all of the following criteria on a DNA chip:

(1) having reliable signal intensity,
(2) exhibiting gene-expression alteration ratio of more than two-folds or less than half, wherein the gene-expression alteration ratio is determined by "comparison of the average expression levels between the non-treated acute patient group the chronic patient group in hospital",
(3) exhibiting p-value of 0.05 or less obtained from test of difference in the average level of gene expression, when the non-treated acute patient group was compared with the chronic patient group in hospital. The genes exhibit alteration with progression of schizophrenia, and thus assumed to reflect the pathology of schizophrenia itself. Therefore, when only the average expression levels of genes were compared between the patient group and the healthy subjects, the comparison does not necessarily result in the p value of less than 0.05.

However, for the purpose to improve accuracy of diagnosis and to discriminate the acute phase and chronic phase of schizophrenia (in details, refer to the following Example) the "gene(s) exhibiting altered expression by progression of schizophrenia " can be selected for the purpose of this diagnosis, then the accuracy of diagnosis can be increased. The genes and proteins to be used as the index may be selected from these genes or proteins, in the simplest may be selected based upon the p value alone or the gene-expression alteration ratio alone, otherwise may be selected based upon the standard deviation of the normal group, theta of non-treated acute patient group or that of chronic patients group in hospital.

When the index gene is selected on the basis of p value alone as the criteria, the index gene may preferably have the p value of 0.2 or less, 0.15 or less, more preferably 0.10 or less and more 0.05 or less. Further preferably, the index gene may have the p value of 0.02 or less, 0.01 or less, 0.005 or less, 0.025 or less, 0.002 or less, 0.001 or less.

When the index gene is selected on the basis of the gene-expression alteration ratio alone as the criteria, the index gene may have the gene-expression alteration ratio of 2.0 or more, preferably the gene-expression alteration ratio of 2.1 or more, 2.2 or more, 2.25 or more, 2.5 or more, 3 or more, 4 or more, 5 or more, 6 or more, 7 or more, 7.5 or more, 8 or more, 9 or more, 10 or more.

Moreover, the method of the present invention can be utilized for the purpose to diagnose objectively whether a test subject suffers from schizophrenia or not, using the expression of the gene or fragment thereof and/or the protein encoded by the gene or fragment thereof satisfying the aforementioned criteria.

According to this specification, the term "schizophrenia" includes any type of schizophrenia such as paranoid schizophrenia, disorganized schizophrenia, catatonic schizophrenia, and a type of schizophrenia incapable to be classified. As to genes involved in schizophrenia, genetic polymorphism of these genes has been known. Therefore, by using a DNA chip, a method capable of capturing expression of larger number of gene is identical, then a method to diagnose "schizophrenia" based upon plural criteria on gene expression is developed as described in this specification. Thus among genes listed in Table 1 and Table 2 described above, gene expression may measured on one gene, preferably 2 genes, more preferably 5 genes, 10 genes, 20 genes, 30 genes, 50 genes, 100 genes, and determination may be performed comprehensively.

According to the method of the present invention, at least one nucleic acid or its fragment and/or at least one protein or its fragment encoded by the nucleic acid selected from the group consisting of the genes represented by the Genbank accession no. described in Table 1 and Table 2 may be quantified. In some cases, plural protein names or plural Genbank accession nos. may be registered or become to be popular for one gene, a nucleic acid specified as such is also included within the scope of this invention.

The "nucleic acid(s) complementary to the nucleic acid(s) defining gene(s) exhibiting altered expression by occurrence of schizophrenia" typically means mRNAs and cDNAs of genes represented by the Genbank accession no. described in Table 1. Moreover, the "nucleic acid(s) defining gene(s) exhibiting altered expression by progression of schizophrenia" typically means mRNAs and cDNAs of genes represented by the Genbank accession no. described in Table 2. Furthermore, any polynucleotides, such as regulatory sequences or polyadenyl sequences, may be included in the terminal ends of the translation region and/or inside of these mRNA or cDNA.

The "fragment of a nucleic acid defining gene(s) exhibiting altered expression by occurrence of schizophrenia" means a polynucleotide consisting of a part of nucleic acid(s) defining gene(s) represented by the Genbank accession no. described in Table 1 while retaining its biological function. Typically, it may be a restriction fragment of a mRNA or a cDNA corresponding to the gene represented by the Genbank accession no. described in Table 1. In the same manner, the "fragment of a protein encoded by a nucleic acid(s) defining gene(s) exhibiting altered expression by occurrence of schizophrenia" means a polypeptide consisting of a part of protein(s) encoded by the nucleic acid(s) defining gene represented by the Genbank accession no. described in Table 1 while retaining its biological function.

Moreover, the "fragment of a nucleic acid defining gene(s) exhibiting altered expression by progression of schizophrenia" means a polynucleotide consisting of a part of nucleic acid(s) defining gene(s) represented by the Genbank accession no. described in Table 2 while retaining its biological function. Typically, it may be a restriction fragment of a mRNA or a cDNA corresponding to the gene represented by the Genbank accession no. described in Table 2. In the same manner, the "fragment of a protein encoded by a nucleic acid(s) defining gene(s) exhibiting altered expression by progression of schizophrenia" means a polypeptide consisting of a part of protein(s) encoded by the nucleic acid(s) defining gene represented by the Genbank accession no. described in Table 2 while retaining its biological function.

The term "nucleic acid" used in this specification may include any polynucleotide consisting of simple nucleotides and/or modified nucleotides such as cDNA, mRNA, total RNA and hnRNA. The term "modified nucleotides" may include phosphoric esters such as inosine, acetylcytidine, methylcytidine, methyladenosine and methyl guanosine, as well as other postnatal nucleotides which may be produced by the effect of ultraviolet rays or chemical substances.

Mononuclear cell in blood is also called monocyte, which is a cell having single nuclear in blood and corresponds to large lymphocyte. Macrophage in inflammatory sites is included herein and such vell exhibits strong phagocytic effect. Erythrocyte may removed from total blood cell fraction treated by anti-coagulant under non-isotonic condition, and blood cell fraction thus obtained may be purified by classification according to cell volume using sucrose density-gradient centrifugation method or ficoll centrifugation method.

In general, to achieve quantification of nucleic acids, a sample may be obtained from a test subject, which is succeeded by extraction of ribonucleic acid from the sample. Extraction of the nucleic acid from a component of living body may be achieved by any extraction method such as phenol extraction and ethanol precipitation. To achieve extraction of mRNA, the sample may be passed through an oligo-dT column.

In the case where the amount of the nucleic acid is not large, the nucleic acid may be amplified, if necessary. The nucleic acid may be amplified by polymerase chain reaction (hereinafter, simply referred to as "PCR"), for example, by reverse transcriptase PCR (RT-PCR). Furthermore, as described in the following description, the amplification may be performed as a quantitative operation or the quantitative operation may be combined with other operations.

After the extraction procedure and/or the amplification procedure (if necessary) may be achieved, at least one nucleic acid or fragment thereof selected from the group consisting of nucleic acids defining genes represented by GenBank Accession nos. listed in Table 1 or 2, may be quantified. Otherwise, at least one protein or fragment thereof encoded by the nucleic acid may be quantified.

The nucleic acid may be quantified by any conventional known method, such as quantitative PCR, Northern blotting, RNase protection mapping, or a combination of such methods. In the case the kinds of genes to be quantified is small, these methods of quantitative PCR, Northern blotting, RNase protection mapping may be effective. In the Example, a DNA chip is utilized, however, above-mentioned procedures are simpler and lower in price. However, the embodiment of the present invention is not limited to that utilizing a DNA chip.

The internal nucleotides of the amplified products may be labeled in the quantitative PCR, typically by using radio-labeled nucleotides (e.g.,$^{32}$P). Alternatively, the amplified product may be endo-labeled by using radio-labeled primers. Free radio-labeled nucleotides or radio-labeled primers may be separated from the labeled amplified products, by using some known methods including gel filtration, alcohol precipitation, trichloroacetic acid precipitation and physical absorption using a glass filter. Thereafter, procedures such as electrophoresis and hybridization may be performed (or may not be performed) and the amplified products may be quantified by using liquid scintillation, autoradiography, and imaging plate Bio-Imaging Analyzer (BAS; Fuji Photo Film Co., Ltd.). Instead of such radioactive substance, a fluorescent substance or a luminescent substance may be used as a labeling substance, and the amplified product may be quantified by means of spectrofluorometer, fluoromicro plate reader or CCD camera. Furthermore, in the case where incorporation of the labeling substance into the amplified product is not performed during the PCR operation, an intercalate fluorescent pigment such as ethidium bromide, SYBR Green I™, PicoGreen™ (manufactured and sold by Molecular Probes) may be used to detect the amplified product.

In the case where the quantitative PCR may not be performed, the sample containing nucleic acid may be subjected to electrophoresis, and then analyzed by Southern blotting or Northern blotting, thereafter quantification may be achieved by using a probe labeled with a detectable marker.

In the case where many kinds of nucleic acids are to be quantified simultaneously, DNA chip or DNA microarray may be used together with or instead of the aforementioned techniques. In this case, many kinds of nucleic acids can be quantified and analyzed at one procedure, such means is useful for multiplication of probabilities and for linear discriminant analysis. Moreover, a DNA chip or a DNA micro-array on which the "nucleic acid(s) defining gene(s) exhibiting altered expression by progression of schizophrenia" is immobilized may be prepared for the purpose to be applied in diagnosis of schizophrenia and a kit may be also prepared. Various methods can be adopted to prepare a DNA or a DNA micro-array on which a nucleic acid is immobilized, such methods are well known to those skilled in the art. In concrete, DNA may be arranged onto a substrate at extremely high density and the arranged DNA may be analyzed, otherwise, DNA may be synthesized directly on a substrate. Arrangement of DNA on a substrate at extremely high density may be achieved by utilizing a commercially available spotter.

Instead of quantification of the nucleic acid or together with quantification of the nucleic acid, the amount of gene expression may be indirectly determined by quantifying the amount of protein produced from the nucleic acid (gene). When schizophrenia is diagnosed according to the method of the present invention, in many cases, the indirect method of quantifying protein(s) encoded by nucleic acid(s) may be more useful than the direct method of quantifying nucleic acid(s). Therefore, in the case a nucleic acid is to be quantified indirectly by quantification of a protein, it is preferred to investigate on the extent of the alteration of the protein to be quantified observed in a schizophrenic patient in comparison with healthy subjects (non-mental patient).

For the method of protein extraction from cells and for the method of protein quantification, any methods known in this field may be used. The proteins (gene products) produced in many blood cells are released into blood in many cases, therefore, proteins in blood, plasma or serum can be used as a sample of a test subject. Examples of methods for protein quantification may include Western blotting method and enzyme-linked immunosorbent assay method including solid-phase enzyme-linked immunosorbent assay, immunocytochemistry, and immunohistochemistry. Alternatively, in the case an antibody toward the target protein is available, cells may be labeled by fluorescence by means of immunocytochemistry and the fluorescent intensity of the cells may be quantified by cell sorter, thereby determination may be achieved.

Meanwhile, only summary of the conventional procedures are schematically exemplified in this specification, however, such description should not be interpret to limit the range of this invention. Therefore, modified or alternative methods of the aforementioned methods can be also utilized.

Extraction, amplification, isolation, and quantification of the nucleic acid can be performed automatically by using an automatic operation device currently on the market, in which an electrophoresis device and a PCR device and the like are combined, therefore, utilization of such device may be preferred. By using such an automatic machine, diagnosis of schizophrenia can be achieved in the same manner as routine clinical tests.

After quantification of a prescribed nucleic acid, whether a test subject suffers from schizophrenia or not may be determined, using the quantified value as the index. In the case diagnosis is made by using quantitative value of a singular nucleic acid as the index, the threshold value may be determined appropriately with reference to a normal value. Then, if the quantified value is higher or lower than the threshold value, it is highly possible that the test subject suffers from schizophrenia. In the group of genes listed in Table 1, the genes described in No.1 to No.98 and No.129 to No.132 exhibit decreased expression by occurrence of schizophrenia. Therefore, if the quantified value is lower than the predetermined threshold of healthy subjects, the test subject can be diagnosed to be schizophrenic at high probability.

Meanwhile, in the group of genes listed in Table 1, the genes described in No.99 to No.128 exhibit increased expression by occurrence of schizophrenia. Therefore, if the quantified value is higher than the predetermined threshold of healthy subjects, the test subject can be diagnosed to be schizophrenic at high probability. Moreover, in the group of genes shown in Table 3, 24 genes attached with "CN" mark are included in a gene group obtained by the comparison between the healthy subjects and non-treated patients, whereas 111 genes attached with "CS" mark are included in a gene group obtained by comparison between healthy subjects and chronic patients

Conversely, as to genes described in No.1 to No.98 and No.129 to No.132 of Table 1, when the quantified values of these nucleic acids obtained from a test subject are higher than the known threshold of overall schizophrenic group, the test subject is not included in the patient group. That is, the test subject is diagnosed to be "normal". In the same manner, as to genes described in No.99 to No.128 of Table 1, when the quantified values of these nucleic acids obtained from a test subject are lower than the known threshold of acute and chronic schizophrenic groups, the test subject is not included in the patient group. That is, the test subject is diagnosed to be "normal".

At present, schizophrenia is considered to be a syndrome consisting of plural diseases (paranoid type schizophrenia, disorganized type, catatonic type schizophrenia and a type of schizophrenia incapable to be classified). Therefore, it is desired to diagnose in comprehensive manner by combination of plural genes applicable for determination of schizophrenia at high probability, rather than diagnosis based upon only singular gene.

The threshold value may be selected depending upon the accurately of the diagnosis required, as shown below.

When distribution on the amount of gene expression is elucidated on both the non-schizophrenic group (hereinafter, referred to as normal group) and the schizophrenic group (hereinafter, simply referred to as patient group) groups, the upper threshold or the lower threshold may be selected in such a manner that an individual (from which the nucleic acids to be determined have been obtained) belongs to the normal group with probability of 10%, 5%, or 1%.

When distribution on the amount of gene-expression is elucidated for only the normal group, it can be hypothesized that an individual (from which the nucleic acids to be determined have been obtained) belongs to the normal group. Under this hypothesis, the threshold (the amount or concentration of nucleic acid) may be determined so as to such quantified value can be obtained with a probability (hereinafter, referred to as p-value, one-sided probability may be also utilized for the direction of the alteration is known in advance) of 10%, preferably 5%, more preferably 1%. For example, null hypothesis can be verified using the value of the gene expression level obtained from a DNA chip, in accordance with the 5% threshold value or the 1% threshold value represented in Table 3, that is; the sample having the determined value is included in the normal group or patient group.

In a method for determination of nucleic acid not using a DNA chip, when a part of genes or plural genes described in Table 1 and Table 2 are adopted to determine whether a subject is "normal" or "abnormal", the distribution and variance of the gene may preferably be investigated on healthy subjects and schizophrenic patients in advance. In that case, when distribution of the gene expression has been elucidated only one of the healthy subject group and the patient group, analysis can be made using identical statistical method. The p-value can be calculated by a statistical method such as t-test or non-parametric test.

To elucidate statistical distribution of gene-expression on the normal group and/or the patient group, it is generally required that at least 5 individuals, preferably 10 individuals, more preferably 20 individuals, more preferably 30 individuals, further preferably 50 individuals and the most preferably 100 individuals are to be measured.

Moreover, depending on the correlation with schizophrenia or reliability of the determination, the plural genes selected may be treated by addition at different weights, and the multiplication or mathematical conversion may be performed on the values of expression levels of individual genes to increase the accuracy of determination. It is also possible to determine whether a test subject suffers from schizophrenia or not with higher accurately by using arbitrary statistic methods of various kinds, thus a diagnostic method using such statistic methods should be included within the scope of the present invention.

In the case where diagnosis is made using the quantified value of a singular nucleic acid as the index, as described in detail in the following Examples, the singular nucleic acid may preferably satisfy following criteria; (1) the amount of expression in either of the normal group or the patient group is high (2) the absolute gene-expression alteration ratio between the normal group and patient group is 2.0 or more (refer to "Examples"), and (3) the p-value in the test of mean-values difference is 5% or less.

In the case where diagnosis is made using the quantified values of plural nucleic acids, appropriate thresholds should be determined on each of the nucleic acids. Then diagnosis can be made in the same manner when a singular nucleic acid is used as the index, by examining whether the amount of gene expression is higher or lower than the threshold for individual genes.

If one quantified value of the nucleic acid(s) is higher or lower than the threshold in accordance with the accuracy required, the test subject may be diagnosed to be schizophrenic. If more than two quantified values of the nucleic acid(s) are higher or lower than the thresholds, the test subject may be diagnosed to be schizophrenic at higher possibility. In the case confirmed diagnosis is required, when more numbers of the quantified values of nucleic acids are above or below compared with the threshold, the diagnosis of schizophrenia can be made with more accurately. It is also possible to make determination using the amount of expression of these plural genes through statistic calculation such as mathematical formula, multiplication of probabilities and weighted linear addition.

The diagnostic method of the present invention can be used together with the conventional subjective diagnostic method. Moreover, if quantified data on the amount of nucleic acid(s) can be collected from patients clearly suffering from schizophrenia (determined by some means) and such data is applicable as the index for the diagnostic method of the present invention, it is possible to make confirmed diagnosis by the method of the present invention alone.

The subject of the present invention is to provide a method for objective diagnosis for schizophrenia, therefore, not to provide particular individual procedures for extraction, amplification and analysis described concretely in this specification. Hence, it should be noted that diagnostic method utilizing other than above-mentioned procedures are also include in the scope of present invention.

As described in the above, according to the method of the present invention, objective diagnosis can be made on whether a test subject suffers from schizophrenia or not, by using the amount of expression of nucleic acid (gene) as an index.

Moreover, the method of this invention is also useful for diagnosis of transition of schizophrenia; i.e. from acute phase to chronic phase of schizophrenia. In concrete, the genes described in Table 2 are included in a group of genes exhibiting difference in the expression between the acute patient group and the chronic patient group, therefore, these genes reflect pathologic alteration of schizophrenia. Therefore, using the genes listed in Table 2 as an index, objective diagnosis can be made on whether the pathology of a test subject is at the acute phase of schizophrenia or the pathology of test subject is changing to the chronic schizophrenia.

The diagnostic method of the present invention can be applied to a psychiatric assessment for the purpose to examine whether a subject is legally responsible or not, and to a psychiatric assessment performed for other purposes.

Furthermore, the method of this invention can be utilized in development of a medicine for treatment of schizophrenia. In such case, a candidate compound to be screened may be administrated to a model animal of schizophrenia. If the model animal recovers from schizophrenia, it may be determined that said candidate substance is effective as an anti-schizophrenic agent. In concrete, for example, when the quantified level of the nucleic acid(s) defining gene (s) exhibiting decreased expression by schizophrenia (Table 1, No.1-98 and No.129-132) approaches to the control level with significance compared with the level of the nucleic acid(s) measured prior to administration of said candidate compound, said candidate compound may be effective as an anti-schizophrenic agent. For example, if the level of the nucleic acid(s) defining gene(s) exhibiting altered expression with significance in non-treated acute patients described in Table 3 or gene products thereof is quantified and the quantified level approaches to the normal level with respect to a chronic patient in hospital who received treatment a medicine, such alteration the quantified value(s) of the gene (s) is assumed to reflect the effect of the medicine. In concrete, No.1 (Homo sapiens cDNA, 3 end/clone=IMAGE-2329930 EST wd33c06.x1: Genbank No. AI677689), No.100 (lipocortin-III: Genbank No. M20560), No.146 (Homo sapiens cDNA, 3 end/clone=IMAGE-1714897 EST qc69h01.x1: Genbank No. AI1487729), No.139 (Homo sapiens PAC clone DJ0808A01 from 7q21.1-q31.1: Genbank No. AC004893) and defensin alpha 3: Genbank No. L12691) are included within such gene.

Furthermore in the genes listed in Table 1 or Table 2, determination of the expression levels of genes (the amount of mRNA) involved in protein phosphorylation or de-phosphorylation is particularly useful in diagnosis of schizophrenia. In concrete, by linear discriminant analysis on the expression levels of the genes encoding following 16 kinds of proteins, it is possible to discriminate schizophrenic patients from healthy subjects; (1) Ndr protein kinase (Genbank No.Z35102), (2) protein-tyrosine kinase JAK1 (Genbank No.M64174), (3) inositol polyphosphate 4-phosphatase type I-beta (Genbank No.U96919), (4) AMP-activated protein kinase alpha-1 (Genbank No.AB022017), (5) Protein C kinase Nu (EPK2) : EPK2 mRNA for serine/threonine kinase (Genbank No.AB015982), (6) MEK kinase (Mekk) (Genbank No. U29671), (7) HSTXK Human tyrosine kinase (TXK) (Genbank No.U07794), (8) serine/threonine-protein kinase PRP4h (PRP4h) (Genbank No.U48736), (9) ribosome protein S6 kinase, insulin-stimulated protein kinase 1 (ISPK-1) (Genbank No.U08316), (10) Human SNF1-like protein kinase (Genbank No.U57452), (11) SH-PTP3 for protein-tyrosine phosphatase (Genbank No.D13540), (12) interferon-inducible RNA-dependent protein kinase (Pkr) (Genbank No.U50648), (13) cAMP-dependent protein kinase catalytic subunit type alpha (EC 2.7.1.37) (Genbank No.X07767), (14) PCTAIRE-1 for serine/threonine protein kinase (Genbank No.X66363), (15) branched chain alpha-ketoacid dehydrogenase kinase precursor (BCKD kinase) (Genbank No.AF026548) and (16) Phosphomevalonate kinase (Genbank No.L77213).

The levels of these genes are not altered in depressive patients and in panic disorders compared with control, then the diagnostic method according to this method is effective to discriminate schizophrenia from these other psychiatric diseases. Moreover, in the embodiment where plural genes are combined in the diagnosis, above-mentioned 16 genes may preferably be included within the target genes to be measured for the purpose to achieve higher accuracy.

Now, the present invention will be further explained in detail with reference to Experimental Examples, which will not limits the scope of the present invention in any sense.

<u>Examples</u>

In this Embodiment, explanation will be provided on the genes identified by the present inventors and available as a marker for diagnosis of schizophrenia.

In this study, blood sample was obtained from (1) acute schizophrenic patients (non-treated, samples N1 to N5), (2) chronic schizophrenia patients in hospital (treated by medicine, samples S1 to S12), and (3) normal volunteers without psychiatric disease (samples C1 to C9), in the presence of anticoagulant using a Venoject vacuum blood collection tube. Then mononuclear cells were separated and purified from the blood and RNA was extracted using an Isogen nucleic acid extraction kit (Nippon Gene).

According to the protocol provided by Affynmetrix Co., cDNA was synthesized by transcriptase using an oligo dT primer with T7 promoter, and double strand DNA was prepared using E.coli DNA polymerase. The DNA was purified, and cRNA was transcribed by T7 RNA synthase using a biotin UTP as a substrate. The obtained cRNA was fragmented by the treatment with a solution containing magnesium acetate and potassium acetate. A 30 µg of the cRNA was hybridized with Genechip-U95A (version 2, Affymetrix), then expression of the gene was measured all together and patterning (molecular expression profiling) was performed. The hybridization signal was visualized by fluorescence using avidin/R-Phycoerythrin. The signal was read with a fluorescent reader (Gene array scanner G2500A, Hewlett-Packard). Signals from gene spots were analyzed using a Microarray Suite program (Affymetrix). The signal intensities were determined from the sum of the signals from perfect matches (PM) oligo probes, by subtracting the sum of signals from miss match (MM) oligo probes. To compensate for the variation among different DNA chips, the result was calibrated by representing the values as the ratios toward the median of the positive genes on each of the DNA chips to give normalized signal intensities.

To confirm genes exhibiting widespread alteration in plural schizophrenic patients, two comparisons were performed separately, i.e. the comparison between the acute schizophrenic group (samples N1 to N5) and the normal volunteer group (samples C1 to C9), the comparison between the chronic schizophrenic group (samples S1 to S12) and the normal volunteer group (samples C1 to C9). Table 4 shows the data obtained from the normal volunteers C1-C9. Table 4-1 shows the results from the individual subjects, and Table 4-2 shows averages and standard deviations (S.D.) obtained from the data. Table 5 shows the data from non-treated patients N1-N5 and data from treated chronic patients S1-S12. Table 5-1 shows the values on the non-treated patients, Table 5-2 shows the values on the treated patients respectively, and Table 5-3 shows averages and standard deviations obtained from the data.

Table 4—1

| Genbank | Individual Gene No. | Healthy subject C1 | Healthy subject C2 | Healthy subject C3 | Healthy subject C4 | Healthy subject C5 | Healthy subject C6 | Healthy subject C7 | Healthy subject C8 | Healthy subject C9 |
|---|---|---|---|---|---|---|---|---|---|---|
| U26398 | 25 | 1.01 | 1.25 | 1.27 | 0.99 | 2.33 | 1.83 | 1.61 | 1.40 | 0.85 |
| J04101 | 76 | 0.37 | 4.55 | 1.09 | 4.95 | 3.33 | 5.32 | 2.83 | 2.96 | 2.05 |
| AW006742 | 85 | 1.71 | 1.36 | 1.01 | 1.73 | 1.82 | 1.57 | 2.02 | 0.73 | 1.59 |
| AB028971 | 92 | 1.05 | 1.60 | 0.99 | 1.23 | 1.26 | 1.49 | 1.33 | 0.83 | 1.24 |
| AW003733 | 97 | 1.36 | 0.82 | 1.65 | 1.02 | 0.45 | 1.44 | 1.05 | 0.97 | 0.97 |
| D10202 | 102 | 0.50 | 10.00 | 1.12 | 6.54 | 3.10 | 2.88 | 10.00 | 0.90 | 2.53 |
| L41827 | 109 | 1.05 | 1.61 | 1.14 | 7.85 | 0.75 | 2.85 | 1.08 | 2.65 | 1.37 |
| U08015 | 113 | 1.21 | 1.32 | 1.53 | 0.85 | 3.18 | 1.01 | 1.03 | 1.33 | 1.24 |
| AB028973 | 140 | 0.90 | 2.74 | 2.67 | 1.53 | 6.77 | 1.79 | 1.10 | 1.33 | 0.59 |
| AL036554 | 149 | 0.36 | 1.54 | 0.00 | 1.46 | 2.65 | 1.00 | 0.77 | 1.60 | 1.99 |
| AF002224 | 151 | 1.29 | 1.90 | 12.03 | 4.89 | 26.43 | 27.89 | 15.20 | 0.00 | 0.00 |
|  |  | −7.00 | 4.00 | 3.00 | 3 | 7.00 | 7.00 | −1.00 | −3.00 | 0.00 |

Table 4-2

| Genbank | Mean | Standard Deviation |
|---|---|---|
| U26398 | 1.39 | 0.47 |
| J04101 | 3.05 | 1.70 |
| AW006742 | 1.50 | 0.41 |
| AB028971 | 1.22 | 0.24 |
| AW003733 | 1.08 | 0.36 |
| D10202 | 1.73 | 1.53 |
| L41827 | 1.43 | 2.69 |
| U08015 | 1.24 | 4.15 |
| AB028973 | 1.37 | 2.13 |
| AL036554 | 1.26 | 0.82 |
| AF002224 | 9.96 | 11.12 |

Table 5-1

| Genbank | Gene No. | Non-treated patient N1 | Non-treated patient N2 | Non-treated patient N3 | Non-treated patient N4 | Non-treated patient N5 |
|---|---|---|---|---|---|---|
| U26398 | 25 | 0.67 | 1.31 | 1.05 | 0.93 | 0.73 |
| J04101 | 76 | 1.57 | 3.16 | 0.00 | 2.45 | 0.12 |
| AW006742 | 85 | 1.76 | 1.52 | 0.71 | 0.34 | 0.79 |
| AB028971 | 92 | 1.01 | 0.00 | 0.79 | 0.83 | 0.14 |
| AW003733 | 97 | 1.59 | 1.60 | 1.37 | 0.83 | 1.50 |
| D10202 | 102 | 0.40 | 3.71 | 0.35 | 0.44 | 2.31 |
| L41827 | 109 | 0.62 | 1.21 | 0.53 | 0.62 | 0.74 |
| U08015 | 113 | 0.56 | 1.49 | 0.44 | 0.61 | 1.00 |
| AB028973 | 140 | 0.92 | 0.90 | 0.60 | 0.64 | 0.46 |
| AL036554 | 149 | 0.00 | 0.57 | 0.00 | 1.00 | 0.00 |
| AF002224 | 151 | 5.52 | 27.27 | 0.00 | 0.00 | 0.00 |
| | | -7.00 | 1 | -9.00 | -11.00 | -9.00 |

Table 5-2

| Genbank | Gene No. | Chronic treated patient S1 | Chronic treated patient S2 | Chronic treated patient S3 | Chronic treated patient S4 | Chronic treated patient S5 | Chronic treated patient S6 | Chronic treated patient S7 | Chronic treated patient S8 | Chronic treated patient S9 | Chronic treated patient S10 | Chronic treated patient S11 | Chronic treated patient S12 |
|---|---|---|---|---|---|---|---|---|---|---|---|---|---|
| U26398 | 25 | 0.42 | 0.80 | 1.20 | 0.00 | 1.06 | 0.46 | 0.00 | 0.00 | 1.08 | 0.51 | 0.71 | 1.05 |
| J04101 | 76 | 0.00 | 0.00 | 0.24 | 0.00 | 0.00 | 0.91 | 0.00 | 0.00 | 2.30 | 1.15 | 0.00 | 0.36 |
| AW006742 | 85 | 1.09 | 0.57 | 1.65 | 0.29 | 0.99 | 0.14 | 0.21 | 0.90 | 0.36 | 0.15 | 0.31 | 1.35 |
| AB028971 | 92 | 0.32 | 0.71 | 0.62 | 0.57 | 0.18 | 1.37 | 1.10 | 1.06 | 0.81 | 1.12 | 1.57 | 0.00 |
| AW003733 | 97 | 0.08 | 0.98 | 1.06 | 0.78 | 0.06 | 0.00 | 0.00 | 0.19 | 0.15 | 1.09 | 1.70 | 1.24 |
| D10202 | 102 | 0.32 | 0.26 | 0.47 | 4.80 | 0.28 | 0.76 | 0.39 | 0.16 | 0.39 | 1.61 | 10.00 | 1.25 |
| L41827 | 109 | 0.66 | 0.61 | 0.36 | 1.14 | 0.95 | 1.16 | 0.58 | 0.59 | 0.45 | 2.45 | 0.84 | 2.00 |
| U08015 | 113 | 0.67 | 0.27 | 0.36 | 0.49 | 1.22 | 1.00 | 1.12 | 0.91 | 0.91 | 1.10 | 0.55 | 0.38 |
| AB028973 | 140 | 2.89 | 0.70 | 0.61 | 0.74 | 0.83 | 2.02 | 1.23 | 1.22 | 1.67 | 0.71 | 0.66 | 3.19 |
| AL036554 | 149 | 3.80 | 0.00 | 3.74 | 1.33 | 7.19 | 0.00 | 6.82 | 0.72 | 0.85 | 1.36 | 1.14 | 0.71 |
| AF002224 | 151 | 1.37 | 0.00 | 2.46 | 0.00 | 0.00 | 0.00 | 0.00 | 0.00 | 0.00 | 0.00 | 0.00 | 6.46 |
| | | -9.00 | -11.00 | -9.00 | -9.00 | -9.00 | -7.00 | -9.00 | -11.00 | -9.00 | -3.00 | -5.00 | -5.00 |

Table 5-3

| Genbank | Mean | Standard deviation | Ratio of Mean value |
| --- | --- | --- | --- |
| U26398 | 0.70 | 0.42 | 0.51 |
| J04101 | 0.72 | 1.04 | 0.24 |
| AW006742 | 0.77 | 0.55 | 0.51 |
| AB028971 | 0.72 | 0.47 | 0.59 |
| AW003733 | 0.84 | 0.63 | 0.77 |
| D10202 | 0.48 | 0.60 | 0.28 |
| L41827 | 0.72 | 1.63 | 0.50 |
| U08015 | 0.62 | 1.20 | 0.50 |
| AB028973 | 0.87 | 1.92 | 0.64 |
| AL036554 | 1.72 | 2.30 | 1.36 |
| AF002224 | 2.53 | 6.68 | 0.25 |

In this Example, the genes described in Table 1 were obtained by screening 12,000 genes that satisfy following three criteria:

(1) having reliable signal intensity by PRESENCE Call on DNA chip,
(2) exhibiting gene-expression alteration ratio of more than two-folds or less than half, wherein the gene-expression alteration ratio is determined by either one of "comparison between the average expression level of the non-treated acute patient group and the average expression level of the healthy subjects", "comparison between average expression level of the chronic patient group in hospital and the average expression level of the healthy subjects", or "comparison between the average expression level of the non-treated acute patient group and the average expression level of the chronic patient group in hospital", and
(3) exhibiting p-value of 0.05 or less when the difference on the average gene expression level is examined by Welch's t-test for significant difference on the following groups; the difference between the patient group (either one of the non-treated group (CN) or treated chronic group (CS)) and the normal group, or the difference between the non-treated acute patient group and the treated chronic group (NS). Twenty-four genes were identified by comparing the average expression levels between the non-treated acute patient group and the normal group; 111 genes were identified by comparing the average expression levels between the treated chronic patient group and the normal group; and 34 genes were identified by comparing the average expression levels between the non-treated acute patient group and the treated chronic patient group. These genes exhibited significant alteration by acute or chronic schizophrenia.

Accordingly, these genes described in Tables 1 and 2 selected according to the above criteria are particularly useful as an index for diagnosis of schizophrenia. The data on the averages, distributions and probabilities (described in Table 3) obtained from the DNA chips of the following Examples are useful for creating criteria for practical diagnosis of schizophrenia.

Example 1: Singular determination

In this Example, description will be given about a diagnostic method for schizophrenia based upon one gene, using peripheral blood from a patient and adopting the expression level of mRNA for erythroblastosis virus oncogene homolog 1 (ETS-1) protein (v-ets avian erythroblastosis virus E26 oncogene homolog 1, Genbank Accession No. J04101) as a criteria.

First, mononuclear cells were isolated from the peripheral blood of the patient, and pure RNA was extracted by acid-phenol extraction. The RNA was treated according to the protocol provided by Affimetrix, and cDNA, dDNA and cRNA were prepared, then they were fragmented and hybridized with a DNA chip (U34Human). The amount of mRNA for ETS-1 protein (GenBank Accession No.J04101) was determined, and the result was represented by the ratio relative to the median obtained from all genes that gave significant signals on the DNA chip for normalization of the results.

From the data listed in Tables 4 and 5, the distributions on the control group and the patient group are as follows;

(1) Distribution of control group (N = 9): Average 3.05; Standard deviation 1.70; 5% lower limit threshold, 0.33
(2) Distribution of patient groups (N = 17): Average 0.72; Standard deviation 1.04; 5% upper limit threshold 2.38; 1% upper limit threshold 3.13

As seen from Tables 3 and 4, the values of the total 26 samples actually used for the study were; (C1) 0.37, (C2) 4.55, (C3) 1.09, (C4) 4.95, (C5) 3.33, (C6) 5.32, (C7) 2.83, (C8) 2.96, (C9) 2.05, (N1) 1.57, (N2) 3.16, (N3) 0.00, (N4) 2.45, (N5) 0.12, (S1) 0.00, (S2) 0.00, (S3) 0.24, (S4) 0.00, (S5) 0.00, (S6) 0.91, (S7) 0.00, (S8) 0.00, (S9) 2.30, (S10) 1.15, (S11) 0.00, and (S12) 0.36. The values from four subjects (C2, C4, C5 and C6) were higher than the 1% upper limit threshold of the distribution of the patient group (3.13), and they were determined to be not included in the schizophrenic group at 99% reliability. That is, they were judged to be "normal."

In contrast, the expression levels of the gene obtained from ten patients (N3, N5, S1, S2, S3, S4, S5, S7, S8 and S11) were lower than the 5% lower limit threshold of the distribution of the control group (0.33). Therefore, they were determined to be not included in the non-schizophrenic (normal) group at 95% reliability. That is, they were determined to be "abnormal." As to the remaining 12 subjects, diagnosis could not be made on them. Therefore, as long as the above criteria were used, false diagnosis such as taking a subject included in the control group as "abnormal" or taking a subject included in the patient groups as "normal" could be avoided.

Example 2: Combined determination

In this Example, description will be given on diagnostic method with higher accuracy. In this method, mononuclear cells from peripheral blood of a patient were used, and the expression level of one gene was determined; i.e. mRNA for erythroblastosis virus oncogene homolog 1 (ETS-1) protein (v-ets avian erythroblastosis virus E26 oncogene homolog 1, GenBank Accession No.J04101), and the expression level was combined with the expression levels of two genes; i.e. gene for KIAA1048 protein (GenBank Accession No.AB028971) and gene for NCI_CGAP_Kid11 Homo sapiens cDNA clone IMAGE:2497327 3' similar to SW:RHOD_HUMAN 000212 RHO-RELATED GTP-BINDING PROTEIN RHOD (Genbank Accession No.AW003733).

As described in Example 1, DNA chips (U95A, version 2) were used to assay the expression level of the gene for KIAA1048 protein (GenBank Accession No.AB028971). The 1% lower threshold of gene for KIAA1048 protein (GenBank Accession No.AB028971) was 0.66 for the distribution of the control group and the 1% upper threshold of the gene was 1.80 for the distribution of the patient group, and the level of the sample normalized by median was compared with these thresholds, thereby singular determination can be performed in the same manner as Example 1. In the total of 26 samples shown in Tables 4 and 5 (sum of groups C, N and S), no sample had the value of higher than 1.80 and determined to be "normal". In contrast, seven subjects (N2, N5, S1, S3, S4, S5 and S12) were determined to be "abnormal" for having the values of lower than 0.66. Therefore, as long as the above thresholds are used, false diagnosis such as taking a subject included in the control group as "abnormal" or taking a subject included in the patient groups as "normal" could be avoided.

The same singular determination as in Example 1 was performed on the gene for NCI_CGAP_Kid11 Homo sapiens cDNA clone IMAGE:2497327 3' similar to SW:RHOD_HUMAN 000212 RHO-RELATED GTP-BINDING PROTEIN RHOD (Genbank Accession No.AW003733). The 1% lower threshold of this gene was 0.25 for the distribution of the control group and the 5% upper threshold of the gene was 1.82 for the distribution of the patient group, then value from an unknown sample was normalized to the median and compared with these thresholds. In total of 26 samples shown in Tables 4 and 5 (sum of groups C, N and S), no sample had the value of higher than 1.82 and determined to be "normal". In contrast, six subjects (S1, S5, S6, S7, S8 and S9) were determined to be "abnormal" for having the value of lower than 0.25. Therefore, as long as the above thresholds are used, false diagnosis such as taking a subject included in the control group as "abnormal" or taking a subject included in the patient groups as "normal" could be avoided.

Using the expression levels on gene for EST-1 protein (GeneBank Accession No.J04101), tKIAA1048 protein (GenBank Accession No.AB028971) and NCI_CGAP_Kid11 Homo sapiens cDNA clone IMAGE:2497327 3' similar to SW:RHOD_HUMAN 000212 RHO-RELATED GTP-BINDING PROTEIN RHOD (GenBank Accession No.AW003733), determinations based on these three genes were combined and any of these determinations was taken as the final determination. As the result, in total of 9 subjects in the normal group, four subjects (C2, C4, C5 and C6) were determined to be "normal." In the patient group, 14 subjects in 17 subjects were determined to be "abnormal," except for three subjects (N1, N4 and S10). Therefore, as long as the above criteria are used, false diagnosis such as taking the control group as "abnormal" or taking the patient groups as "normal" could be avoided.

Thus, if the amount of expression in any gene exhibits significant difference (even in a singular gene), a patient can be diagnosed to be schizophrenic. Therefore, diagnosis of schizophrenia can be achieved with higher accuracy.

Example 3: Comprehensive probability determination

In this Example, the description will be given about an attempt to provide a more reliable method for diagnosis of schizophrenia. In this Example, plural genes exhibiting abnormal expression level (not within the normal range) in the patient group were by combined and statistical distributions of the expression levels of the genes in the normal control group were taken the into account.

In this Example, the distributions and deviations of the expression levels in normal subjects (unit; the ratio to the median of the DNA chips) are represented for the following 11 genes. Incidentally, since the expression levels of following three genes: 6) platelet-activating factor receptor (GenBank Accession No.D10202), 7) neuregulin 1 isoform HRG-alpha (GenBank Accession No.L41827), and 8) cytosolic component of the nuclear factor of activated T cells (GenBank Accession No.U08015) increased accompanied with schizophrenia, the distributions of the reciprocal numbers were represented for the three genes. The averages, standard deviations, and thresholds of the expression levels in the normal control group as well as the data of samples obtained on the individual genes are represented in Tables 3 and 4.

1) inositol polyphosphate-4-phosphatase, type 1, isoform b (GenBank Accession No.U26398), average $\pm$ standard deviation = $1.39 \pm 0.47$

2) v-ets avian erythroblastosis virus E26 oncogene homolog 1 (GenBank Accession No.J04101), average $\pm$ standard deviation = $3.05 \pm 1.70$

3) NCI_CGAP_Pr28 Homo sapiens cDNA clone IMAGE:2489058 3' similar to TR:Q15810 Q15810 CLONE 137308 ORF1 (GenBank Accession No.AW006742), average $\pm$ standard deviation = $1.50 \pm 0.41$

4) Source: Homo sapiens mRNA for KIAA1048 protein, complete cds, KIAA1048 protein (GenBank Accession No. AB028971), average $\pm$ standard deviation = $1.22 \pm 0.24$

5) NCI_CGAP_Kid11 Homo sapiens cDNA clone IMAGE:2497327 3' similar to SW:RHOD_HUMAN 000212 RHO-RELATED GTP-BINDING PROTEIN RHOD (GenBank Accession No.AW003733), average $\pm$ standard deviation = $1.08 \pm 0.36$

6) platelet-activating factor receptor (GenBank Accession No.D10202), average $\pm$ standard deviation = $1.73 \pm 3.75$

7) neuregulin 1 isoform HRG-alpha (GenBank Accession No.L41827), average $\pm$ standard deviation = $1.43 \pm 2.22$

8) cytosolic component of the nuclear factor of activated T cells, cytoplasmic, calcineurin-dependent 1 (GenBank Accession No.U08015), average $\pm$ standard deviation = $1.24 \pm 0.69$

9) Homo sapiens mRNA for KIAA1050 protein (GenBank Accession No.AB028973), average $\pm$ standard deviation = $0.73 \pm 0.47$

10) Homo sapiens cDNA clone DKFZp564J2262 (GenBank Accession No.AL036554), average $\pm$ standard deviation = $1.26 \pm 0.82$

11) Angelman Syndrome gene, E6-AP ubiquitin protein ligase 3A (GenBank Accession No.AF002224), average $\pm$ standard deviation = $9.96 \pm 11.12$

As described in Tables 4 and 5, +1 is given if the amount of gene expression to be tested is higher than the amount of average expression in the normal control, whereas -1 is given if the amount of expression is lower than that in the normal control. For example, in the case of N1, +1 is given on the nine genes of 1), 2), 4), 6), 7), 8), 9), 10) and 11) to give the score of +9, -1 is given on two genes of 3) and 5) to give the score of -2, therefore the total score results in +7. Moreover, in the case of N2, +1 is given on the six genes of 2), 3), 5), 6), 8) and 11) to give the score of +6, -1 is given on five genes of 1), 4), 7), 9) and 10) to give the score of -5, therefore the total score results in +1.

As described above, the comparison and the addition of the scores were performed on the 11 genes described above. In the case the total score of a subject was -8 or lower, the subject was determined to be "suffering from schizophrenia (abnormal)" ($P < (1/2)^8 < 0.004$), whereas in the case the score was between -5 and -7 ($P = 0.03$ to 0.008), the subject was determined to be "pseudo-positive (suspected to be abnormal)." As a result, among 17 patients, it was found that 11 patients (N3, N4, N5, S1, S2, S3, S4, S5, S7, S8 and S9) were determined to be "positive for schizophrenia (abnormal)", 4 patients (N1, S6, S11 and S12) were determined to be "pseudo-positive for schizophrenia (suspected to be abnormal)", and two patients (N2, S10) were determined to be "not confirmed", respectively. Thus, it was demonstrated that a diagnosis could be made on a subject as to whether the subject is suffering from schizophrenia or not with high reliably. To achieve it, the expression levels (including the distribution and the variance) of plural genes were compared with those of the normal control, and the multiplicative values of the probabilities were calculated respectively. As seen from above, simultaneous measurement on the levels of gene expression can be achieved for many sample, use of DNA chips is advantageous.

Example 4: Linear discriminant analysis

In this Example, plural genes exhibiting altered expression between the chronic patient group and the acute patient group were used, and the values measured on the chronic and acute patients were treated by weighted linear discriminant, thereby diagnosis of schizophrenia was achieved with higher reliability. The explanation will be made on the Example described in Table 6.
Table 6

|  |  | Discriminant score Y |
|---|---|---|
| Healthy subject | C1 | 8.87 |
| Healthy subject | C2 | 15.02 |
| Healthy subject | C3 | 3.54 |
| Healthy subject | C4 | 12.05 |
| Healthy subject | C5 | 13.00 |
| Healthy subject | C6 | 11.19 |
| Healthy subject | C7 | 12.67 |
| Healthy subject | C8 | 7.07 |
| Healthy subject | C9 | 7.98 |
| Acute patient | N1 | -2.80 |
| Acute patient | N2 | -8.65 |
| Acute patient | N3 | -10.74 |
| Acute patient | N4 | -11.45 |
| Acute patient | N5 | -12.55 |
| Chronic patient | S1 | -12.81 |
| Chronic patient | S2 | -5.24 |
| Chronic patient | S3 | -5.12 |
| Chronic patient | S4 | -16.51 |
| Chronic patient | S5 | -11.67 |
| Chronic patient | S6 | -10.66 |
| Chronic patient | S7 | -4.67 |
| Chronic patient | S8 | -4.52 |
| Chronic patient | S9 | -6.30 |
| Chronic patient | S10 | -17.77 |
| Chronic patient | S11 | -11.84 |
| Chronic patient | S12 | -19.31 |

In this Example, the expression levels of mRNAs of the following genes were measured using the mononuclear cells in peripheral blood derived from a test subject, that is, seven genes (genes 1, 2, 3, 7 and 9) exhibiting altered expression by acute schizophrenia; three genes (genes 4, 8 and 10) exhibiting altered expression by chronic schizophrenia; and two genes (genes 5 and 6) exhibiting altered expression by transition from acute phase to chronic phase of schizophrenia.

1) inositol polyphosphate-4-phosphatase, type 1, isoform b (GenBank Accession No.U26398) The mRNA level of the gene on the Gene Chip calibrated by median was represented by parameter X1.
2) v-ets avian erythroblastosis virus E26 oncogene homolog 1 (GenBank Accession No.J04101). The mRNA level of the gene on the Gene Chip calibrated by median was represented by parameter X2.

3) NCI_CGAP_Pr28 Homo sapiens cDNA clone IMAGE:2489058 3' similar to TR:Q15810 Q15810 CLONE 137308 ORF1; ESTwr 28gIO X1 (GenBank Accession No.AW006742). The mRNA level of the gene on the Gene Chip calibrated by median was represented by parameter X3.

4) Source—Homo sapiens mRNA for KIAA1048 protein, complete cds, KIAA1048 protein (GenBank Accession No.AB028971). The mRNA level of the gene on the Gene Chip calibrated by median was represented by parameter X4.

5) NCI_CGAP_Kid11 Homo sapiens cDNA clone IMAGE:2497327 3' similar to SW:RHOD_HUMAN 000212 RHO-RELATED GTP-BINDING PROTEIN RHOD (GenBank Accession No.AW003733). The mRNA level of the gene on the Gene Chip calibrated by median was represented by parameter X5.

6) Homo sapiens cDNA clone DKFZp564J2262 (GenBank Accession No.AL036554). The mRNA level of the gene on the Gene Chip calibrated by median was represented by parameter X6.

7) Angelman Syndrome gene, E6-AP ubiquitin protein ligase 3A (GenBank Accession No.AF002224). The mRNA level of the gene on the Gene Chip calibrated by median was represented by parameter X7.

8) neuregulin 1 isoform HRG-alpha (GenBank Accession No.L41827). The mRNA level of the gene on the Gene Chip calibrated by median was represented by parameter X8.

9) cytosolic component of the nuclear factor of activated T cells (GenBank Accession No.U08015). The mRNA level of the gene on the Gene Chip calibrated by median was represented by parameter X9.

10) Homo sapiens mRNA for KIAA1050 protein (GenBank Accession No.AB028973). The mRNA level of the gene on the Gene Chip calibrated by median was represented by parameter X10.

The expression levels of above-mentioned genes represented by parameters X1 to X10 were used to calculate a discriminant score Y according to the following formula (Formula 1), which was optimized by linear discriminant analysis in advance.

[Formula 1]

$$Y = 9.35 \times (X1) - 0.15 \times (X2) + 7.50 \times (X3) + 8.46 \times (X4) + 0.99 \times (X5) - 0.66 \times (X6)$$

$$- 0.42 \times (X7) - 5.59 \times (X8) - 3.17 \times (X9) - 1.89 \times (X10) - 13.03.$$

As an exemplary trail, the discriminant score Y was calculated for the 26 samples used in the study.

(1) Y = 8.87 for normal subject C1; 15.02 for normal subject C2; 3.54 for normal subject C3; 12.0 for normal subject C4; 13.00 for normal subject C5; 11.19 for normal subject C6; 12.67 for normal subject C7; 7.07 for normal subject C8; and 7.98 for normal subject C9.

(2) Y = -2.80 for acute patient N1; -8.65 for acute patient N2; -10.74 for acute patient N3; -11.45 for acute patient N4; and -12.55 for acute patient N5.

(3) Y = -12.81 for chronic patient S1; -5.24 for chronic patient S2; -5.12 for chronic patient S3; -16.51 for chronic patient S4; -11.67 for chronic patient S5; -10.66 for chronic patient S6; -4.67 for chronic patient S7; -4.52 for chronic patient S8; -6.30 for chronic patient S9; -17.77 for chronic patient S10; -11.84 for chronic patient S11; and -19.31 for chronic patient S12.

Then in the case the discriminant score Y used as a criteria gave a negative value in a subject, the subject is determined to be schizophrenic; while if discriminant score Y gave a positive value, the subject is determined to be not schizophrenic. Thus, expression levels on plural genes (including those exhibiting altered expression by transition from acute phase to chronic phase of schizophrenia) were measured and the results were treated by weighted linear discriminant analysis. Thereby, diagnosis on whether or not a subject is suffering from schizophrenia could be achieved by with high reliability.

Example 5: Mahalanobis discriminant analysis

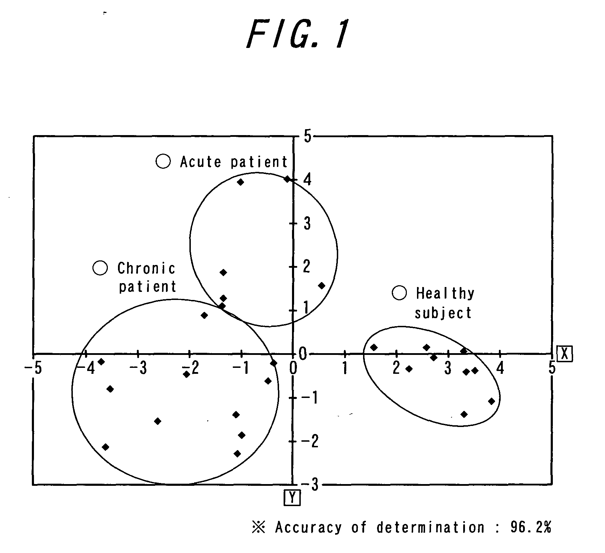

In this Example, plural genes exhibiting altered expression between the chronic patient group and the acute patient group were used, and the individual expression levels measured on the chronic and acute patients were treated by linear addition with different weights by two manners and the obtained results were plotted in two dimensional coordinate system. Using this method the acute phase and the chronic phase of a patient could be distinguished at once. The explanation will be made using Table 7 and Figure 1.

Table 7

| Caronical discriminant function coefficient | | |
|---|---|---|
| | Function | |
| | 1 | 2 |
| X1 | 1.887 | -.842 |
| X2 | .135 | .604 |
| X3 | 1.652 | -.179 |
| X4 | 1.380 | -1.930 |
| X5 | .581 | 1.272 |
| X6 | -.092 | -.284 |
| X7 | -.091 | .016 |
| X8 | -.853 | 1.489 |
| X9 | -.967 | -.887 |
| X10 | -.090 | 1.215 |
| (constant) | -2.467 | -.783 |
| non standarized coefficient | | |

In this Embodiment, the expression levels of mRNAs of the following genes were measured using the mononuclear cells in peripheral blood derived from a test subject, that is, seven genes (genes 1, 2, 3, 7 and 9) exhibiting altered expression by acute schizophrenia; three genes (genes 4, 8 and 10) exhibiting altered expression by chronic schizophrenia; and two genes (genes 5 and 6) exhibiting altered expression by transition from acute phase to chronic phase of schizophrenia.

1) inositol polyphosphate-4-phosphatase, type 1, isoform b (GenBank Accession No.U26398) The mRNA level of the gene on the Gene Chip calibrated by median was represented by parameter X1.
2) v-ets avian erythroblastosis virus E26 oncogene homolog 1 (GenBank Accession No.J04101). The mRNA level of the gene on the Gene Chip calibrated by median was represented by parameter X2.
3) NCI_CGAP_Pr28 Homo sapiens cDNA clone IMAGE:2489058 3' similar to TR:Q15810 Q15810 CLONE 137308 ORF1; ESTwr 28gIO X1 (GenBank Accession No.AW006742). The mRNA level of the gene on the Gene Chip calibrated by median was represented by parameter X3.
4) Source-Homo sapiens mRNA for KIAA1048 protein, complete cds, KIAA1048 protein (GenBank Accession No. AB028971). The mRNA level of the gene on the Gene Chip calibrated by median was represented by parameter X4.
5) NCI_CGAP_Kid11 Homo sapiens cDNA clone IMAGE:2497327 3' similar to SW:RHOD_HUMAN 000212 RHO-RELATED GTP-BINDING PROTEIN RHOD (GenBank Accession No.AW003733). The mRNA level of the gene on the Gene Chip calibrated by median was represented by parameter X5.
6) Homo sapiens cDNA clone DKFZp564J2262 (GenBank Accession No.AL036554). The mRNA level of the gene on the Gene Chip calibrated by median was represented by parameter X6.
7) Angelman Syndrome gene, E6-AP ubiquitin protein ligase 3A (GenBank Accession No.AF002224). The mRNA level of the gene on the Gene Chip calibrated by median was represented by parameter X7.
8) neuregulin 1 isoform HRG-alpha (GenBank Accession No.L41827). The mRNA level of the gene on the Gene Chip calibrated by median was represented by parameter X8.
9) cytosolic component of the nuclear factor of activated T cells (GenBank Accession No.U08015). The mRNA level of the gene on the Gene Chip calibrated by median was represented by parameter X9.
10) Homo sapiens mRNA for KIAA1050 protein (GenBank Accession No.AB028973). The mRNA level of the gene on the Gene Chip calibrated by median was represented by parameter X10.

The expression levels of above-mentioned genes represented by parameters X1 to X10 were used and the position of the test subject in a coordinate system (X,Y) was calculated according to the following formulas (Formula 2, Formula 3), which was optimized by linear discriminant analysis in advance.

[Formula 2]

$$X = 1.187x(X1) + 0.135x(X2) + 1.652x(X3) + 1.380x(X4) + 0.581x(X5) -$$

$$0.092x(X6) - 0.091x(X7) - 0.853x(X8) - 0.967x(X9) - 0.090x(X10) - 2.467$$

[Formula 3]

$$Y = -0.842x(X1) + 0.604x(X2) - 0.179x(X3) - 1.930x(X4) + 1.272x(X5) -$$

$$0.284x(X6) + 0.016x(X7) + 1.489x(X8) - 0.887x(X9) + 1.215x(X10) - 0.783$$

As an exemplary trial, calculation was performed for the 26 samples used in the study. As the result, as shown in Fig.1, it was revealed that the plots from each groups, i.e. the normal subject group, the acute schizophrenic patient group and the chronic schizophrenic patient group, gathered at certain regions of the coordinate system. Therefore, by adopting following criteria, determination of "acute schizophrenia," "chronic schizophrenia," and "non-schizophrenia" could be made for all of the samples. Meanwhile, those in other region could not be determined.

-1.5 < X < 1 & 1.1 < Y < 5 : acute schizophrenia
-4 < X <0 & -3 < Y < 1.1 : chronic schizophrenia
1 < X < 4 & -2 < Y < 2 : non-schizophrenia

Thus, the expression levels of plural genes (including those exhibiting altered expression at transition from acute phase to chronic phase of schizophrenia) were measured and results were treated by weighted linear addition according to plural manners. Thereby, diagnosis on whether or not a subject is suffering from schizophrenia could be achieved by with high reliability.

Example 6: Analysis by mRNA level of kinase/phosphatase

The genes represented in Table 1 were those exhibiting altered mRNA expression by schizophrenia in mononuclear cells of patients and the functions of the genes were classified. As the result, it was revealed that the largest part of the genes was identified to be kinase/phosphatase, which are responsible for phosphorylation or de-phosphorylation of moleculars such as proteins. From this phenomenon, it was suggested that there may be some abnormalities on phosphorylation state of the mononuclear cells in schizophrenia. Paying attention to this phenomenon, the inventors selected the sixteen genes involved in phosphorylation reactions (kinase/phosphatase) described below. Then mRNA level alteration was investigated for the genes in patients.

1) Ndr serine threonine protein kinase (GenBank Accession No.Z35102). The mRNA level of the gene on the Gene Chip calibrated by median was represented by parameter X1.
inositol polyphosphate-4-phosphatase, type 1, isoform b (GenBank Accession No.U26398). The mRNA level of the gene on the Gene Chip calibrated by median was represented by parameter X2.
2) janus kinase 1 (GenBank Accession No.M64174). Its mRNA level on the gene chip based on the corrected median is represented by parameter X2.
3) inositol polyphosphate-4-phosphatase, type 1, isoform b (GenBank Accession No.U96919). The mRNA level of the gene on the Gene Chip calibrated by median was represented by parameter X3.
4) protein kinase, AMP-activated, alpha 1 catalytic subunit (GenBank Accession No.AB022017). The mRNA level of the gene on the Gene Chip calibrated by median was represented by parameter X4.
5) protein kinase C, nu (GenBank Accession No.AB015982). The mRNA level of the gene on the Gene Chip calibrated by median was represented by parameter X5.
6) MEK kinase (GenBank Accession No.U29671). The mRNA level of the gene on the Gene Chip calibrated by median was represented by parameter X6.
7) tyrosine kinase (GenBank Accession No.U07794). The mRNA level of the gene on the Gene Chip calibrated by median was represented by parameter X7.
8) serine/threonine-protein kinase PRP4 homolog (GenBank Accession No.U48736). The mRNA level of the gene on the Gene Chip calibrated by median was represented by parameter X8.
9) ribosomal protein S6 kinase, 90kD, polypeptide 3 (GenBank Accession No.U08316). The mRNA level of the gene on the Gene Chip calibrated by median was represented by parameter X9.
10) SNF1-like protein kinase (GenBank Accession No.U57452). The mRNA level of the gene on the Gene Chip calibrated by median was represented by parameter X10.
11) protein-tyrosine phosphatase (GenBank Accession No.D13540). The mRNA level of the gene on the Gene Chip calibrated by median was represented by parameter X11.

12) interferon-inducible RNA-dependent protein kinase (GenBank Accession No.U50648). The mRNA level of the gene on the Gene Chip calibrated by median was represented by parameter X12.

13) protein kinase, cAMP-dependent, catalytic, alpha (GenBank Accession No.X07767). The mRNA level of the gene on the Gene Chip calibrated by median was represented by parameter X13.

14) PCTAIRE protein kinase 1 (GenBank Accession No.X66363). The mRNA level of the gene on the Gene Chip calibrated by median was represented by parameter X14.

15) branched chain alpha-ketoacid dehydrogenase kinase (GenBank Accession No.AF026548). The mRNA level of the gene on the Gene Chip calibrated by median was represented by parameter X15.

16) phosphomevalonate kinase (GenBank Accession No.L77213). The mRNA level of the gene on the Gene Chip calibrated by median was represented by parameter X16.

In this Example, linear discriminant analysis was performed on an acute patient group including six patients (N1-N6), chronic patient group including 12 patients (S1-S12), and normal group including 12 normal subjects (C1-C12). Then alteration of the mRNA level was determined on the above-mentioned enzymes. In concrete, the mRNA signal intensities of the genes on the Gene Chip were assigned as parameters X1 to X16, and a discriminant score Y was calculated according to the following formula (Formula 4), which was optimized by linear discriminant analysis in advance.

[Formula 4]

$$Y = -4.60X1 - 5.77X2 + 7.74X3 - 13.9X4 + 24.2X5 + 4.11X6 + 3.20X7 -$$

$$7.41X8 + 5.76X9 - 1.06X10 + 34.1X11 + 0.15X12 + 4.16X13 - 2.51X14 + 14.9X15$$

$$- 2.85X16 + 3.50$$

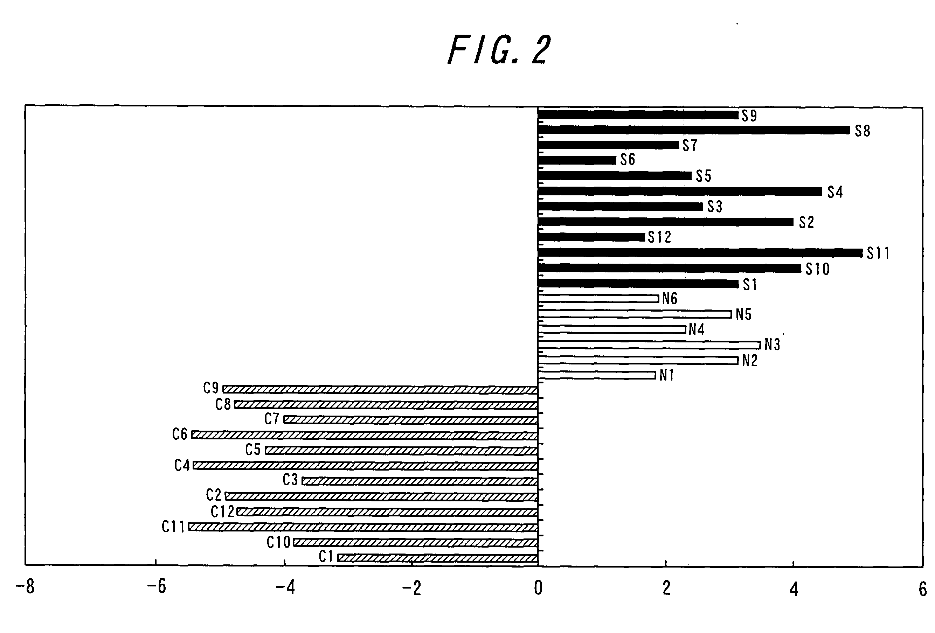

The results are shown in Table 8 and Fig. 2. As seen from Fig. 2, the mRNA levels of the genes encoding the enzymes responsible for phosphorylation of mononuclear cells were altered in schizophrenic patients (S1-S12 and N1-N6). As the result, mRNA levels in schizophrenic patients were apparently distinguishable from those of normal subjects (C1-C12).

[0111]   Table 8

| Subject | Discriminant score |
|---------|--------------------|
| C1 | −3.1574 |
| C10 | −3.84504 |
| C11 | −5.49943 |
| C12 | −4.73543 |
| C2 | −4.90849 |
| C3 | −3.73479 |
| C4 | −5.41805 |
| C5 | −4.27379 |
| C6 | −5.45122 |
| C7 | −3.9983 |
| C8 | −4.78686 |
| C9 | −4.95949 |
| N1 | 1.8629 |
| N2 | 3.14171 |
| N3. | 3.47818 |
| N4 | 2.33664 |
| N5 | 3.04534 |
| N6 | 1.88635 |
| S1 | 3.14728 |
| S10 | 4.12939 |
| S11 | 5.0998 |
| S12 | 1.68138 |
| S2 | 4.01308 |
| S3 | 2.60176 |
| S4 | 4.45117 |
| S5 | 2.4072 |
| S6 | 1.22822 |
| S7 | 2.21729 |
| S8 | 4.90192 |
| S9 | 3.13867 |

| | Mean value | S.D. |
|---|-----------|------|
| C | -4.56402 | 0.758766 |
| N | 2.625187 | 0.690093 |
| S | 3.25143 | 1.269113 |

Example 7: Investigations on patients with other psychiatric diseases

As to patients suffering from depression or panic syndrome (B1-B6) which may exhibit symptoms similar to schizophrenia, using the linear discriminant (Formula 4) shown in the Example 6, gene expression profiling was performed on genes of mononuclear cells in blood of the patients, using the DNA Chips by the method as described above. In the same manner as in Example 6, the mRNA signal intensities encoding 16 enzymes involved in phosphorylation reaction were applied to the same formula (Formula 4) used in Example 6 to provide a discriminant score Y. The results are shown in Table 9.

As shown in Table 9, in five patients except for patient B3, all of the discriminant scores were negative values, thus the patients were determined to be not suffering from schizophrenia. As to the patient B3, the Y score was +1.6 and exhibited weak positive. The result of this biological test may suggest the possibility that the patient B3 is suffering from schizophrenia. As seen from above, the discriminant according to Formula 4, paying attention to the mRNA levels of the genes encoding kinases/phosphatases, may be useful for determination of psychiatric diseases including schizophrenia.

Table 9

| Subject | Discrimant score |
|---------|------------------|
| B1 | -4.37615 |
| B2 | -4.96375 |
| B3 | 1.60492 |
| B4 | -4.34437 |
| B5 | -2.23161 |
| B6 | -1.29941 |

Example 8: Mahalanobis discriminant analysis

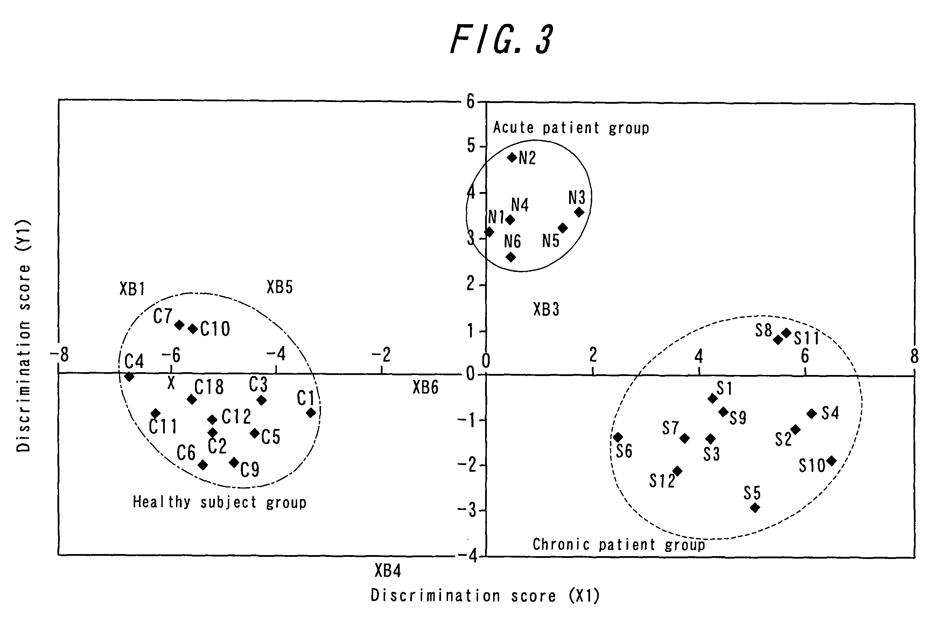

In this Example, the mRNA intensities of the genes encoding the enzymes involved in phosphorylation or dephosphorylation (kinases/phosphatases) were measured like Example 6 and two primary discriminant functions were determined using Mahalanobis cluster analysis. The discriminant scores of each sample were plotted on a two-dimensional plane, then the results were discriminated into three groups, i.e. acute patient group (N1-N6), chronic patient group (S1-S12) and normal group (C1-C12). The mRNA expression intensities (X1 to X16) were obtained for the 16 genes in the Example 6 on a DNA Chip, and the results were applied to the following two linear equations (Formula 5 and Formula 6). The coordinate consisted of (X, Y) in the Table 10 and in the Table 11, and the positions in the coordinate were calculated for the test subjects. The results from the schizophrenic patients (S1-S12 and N1-N6: the same as in Table 8) and those from the normal subjects (C1-C12) were shown in Table 10. Moreover, the results from the six patients (B1-B6: the same as in Table 9) suffering from psychiatric diseases other than schizophrenia were shown in Table 11. The results shown in Table 10 and Table 11 are plotted on a two-dimensional discrimination diagram shown in Fig.3. In Fig.3, the solid column represents the results from chronic schizophrenic patients, the open column represents those from acute schizophrenic patients, and the hatched column represents those from normal subjects.

[Formula 5]

$$X = -4.32X1\ -6.96X2\ -0.31X3\ -12.4X4\ +14.4X5\ +4.84X6\ +2.97X7\ -21.5X8$$

$$+8.11X9\ +8.30X10\ +46.8X11\ -0.89X12\ +4.26X13\ -2.48X14\ +16.7X15\ -4.97X16\ +$$

$$7.03$$

[Formula 6]

$$Y = -1.96X1\ -0.30X2\ +13.8X3\ -6.74X4\ +21.8X5\ +0.37X6\ +1.42X7\ +17.1X8$$

$$-1.31X9\ -13.4X10\ -6.13X11\ +1.50X12\ +1.26X13\ -0.90X14\ +2.48X15\ +1.99X16\ -$$

$$3.72$$

Table 10

| Subject | Discrimant score X | Discrimant score Y |
|---|---|---|
| C1 | -3.31117 | -0.86085 |
| C10 | -5.52444 | 1.02784 |
| C11 | -6.2157 | -0.87543 |
| C12 | -5.15963 | -1.02175 |
| C2 | -5.15608 | -1.3264 |
| C3 | -4.24532 | -0.561 |
| C4 | -6.69962 | -0.06117 |
| C5 | -4.37362 | -1.31594 |
| C6 | -5.35035 | -1.996 |
| C7 | -5.77044 | 1.10472 |
| C8 | -5.55691 | -0.55805 |
| C9 | -4.76531 | -1.95843 |
| N1 | 0.04866 | 3.15851 |
| N2 | 0.48305 | 4.76873 |
| N3. | 1.74519 | 3.59529 |
| N4 | 0.44795 | 3.42336 |
| N5 | 1.44364 | 3.26529 |
| N6 | 0.46093 | 2.62548 |
| S1 | 4.27598 | -0.49912 |
| S10 | 6.49481 | -1.88558 |
| S11 | 5.64561 | 0.97659 |
| S12 | 3.5928 | -2.08721 |
| S2 | 5.83667 | -1.17126 |
| S3 | 4.23527 | -1.38722 |
| S4 | 6.12612 | -0.81531 |
| S5 | 5.07678 | -2.89504 |
| S6 | 2.49265 | -1.34124 |
| S7 | 3.75289 | -1.38186 |
| S8 | 5.50818 | 0.8251 |
| S9 | 4.46141 | -0.77205 |

Table 11

| | Discrimant score X | Discrimant score Y |
|---|---|---|
| B1 | -6.84479 | < 1.94521 |
| B2 | -6.09437 | -0.11655 |
| B3 | 0.93432 | 1.47941 |
| B4 | -2.07503 | -4.63643 |
| B5 | -4.18643 | 1.96029 |
| B6 | -1.44387 | -0.24133 |

As shown by the sets of circles shown in Fig. 3, the scores from "acute schizophrenia group" "chronic schizophrenia group" and "normal group" gathered in a certain region of the graph, therefore, the three groups were completely distinguishable from each other. In addition, the blind data were obtained from the group of patients suffering from psychiatric diseases other than schizophrenia (B1-B6) and the data were also treated by the same Formulas (Formula 5 and Formula 6). The results were plotted in the same figure by the "X" marks, to reveal that the patients do not belong to the regions corresponding to the "acute schizophrenia group" "chronic schizophrenia group" and "normal group" described above. This suggests that the discriminant is also useful for the discrimination of psychiatric diseases other than schizophrenia.

The method of the invention provides a group of genes useful for objective diagnosis on whether or not a test subject is suffering from schizophrenia in non-invasive manner. Therefore, development of a DNA chip containing genes effective for diagnosis of schizophrenia may be achieved utilizing the knowledge obtained in this invention.

## Claims

1. A method for diagnosing whether a test subject suffers from schizophrenia or not, the method comprising the steps of;
   obtaining mononuclear cells in blood containing ribonucleic acid from said subject and extracting said ribonucleic acid from the blood,
   measuring gene expression profile in said test subject by a DNA micro-array or a DNA chip,
   determining whether the quantified level in said test subject exhibits statistical significant alteration or not in comparison with the gene expression profile in healthy subjects or in schizophrenic patients to diagnose whether said test subject suffers from schizophrenia or not.

2. A DNA micro-array or a DNA chip available in the method according to Claim 1.

3. The DNA micro-array or the DNA chip according to Claim 2, which immobilized with nucleic acid(s) defining gene(s) exhibiting altered expression by occurrence of schizophrenia or nucleic acid(s) defining gene(s) exhibiting altered expression by progression of schizophrenia,
   wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) defined by the gene name, the protein name which is a gene product, or the nucleic acid sequence name as described below in (1) to (152) with GenBank No. described in brackets.

   (1) Homo sapiens cDNA, 3'end/clone= IMAGE-2329930, EST wd33c06.×1 (Genbank No. AI677689)
   (2) Bcl-XI (Genbank No. Z23115)
   (3) ZNF37A mRNA for zinc finger (Genbank No. X69115)
   (4) HSCCG1 Human X chromsome mRNA for CCG1 protein inv. in cell proliferation (Genbank No. X07024)
   (5) Interferon receptor type 2 (IFNAR2) (Genbank No. L42243)
   (6) Guanine Nucleotide Exchange Factor 1 (Genbank No. HG960-HT960)
   (7) Glucosamine-6-sulphatase precursor (Genbank No. Z12173)
   (8) MACH-beta-1 protein (Caspase 8)(Genbank No. X98176)
   (9) EST 15a11 Homo sapiens cDNA/gb=W25921 /gi=1306044/ug=Hs.164036 /len=723 (Genbank No. W25921)
   (10) Ndr protein kinase (Genbank No. Z35102)
   (11) 14-3-3 protein (Genbank No. U28964)
   (12) RbAp48 mRNA encoding retinoblastoma binding protein (Genbank No. X74262)
   (13) SNAP23B protein (Genbank No. Y09568)
   (14) Inhibitory protein for potassium-induced deficiency type 1 (SKD1 homolog) (Genbank No. AF038960)
   (15) Homo sapiens cDNA, 3'end/clone=IMAGE-2509049, ETS wt31b09.×1 (Genbank No. AI955897)
   (16) Utrophin (Genbank No. X69086)
   (17) cdc2-related protein kinase (Genbank No. M80629)
   (18) Calmodulin type 1 (CALM1) (Genbank No. U12022)
   (19) Rb2/p130 protein (Genbank No. X74594)
   (20) Protein-tyrosine kinase JAK1 (Genbank No. M64174)
   (21) GTP-binding protein RAB6 (Genbank No. M28212)
   (22) Clq/MBL/SPA receptor C1qR(p) (Genbank No. U94333)
   (23) Zinc finger/leucine zipper protein (AF10)(Genbank No. U13948)
   (24) Inositol polyphosphate 4-phosphatase type I-beta (Genbank No. U96919)
   (25) Inositol polyphosphate 4-phosphatase (Genbank No. U26398)
   (26) Cytohesin binding protein HE (Genbank No. AF068836)
   (27) Cas like protein for enhancer of filamentation (HEF1) (Genbank No. L43821)
   (28) Rho GTPase-activated protein type 5 (p190-B) (Genbank No. U17032)
   (29) AMP-activated protein kinase alpha-1 (Genbank No. AB022017)

(30) Syntaxin 16 (Genbank No. AF038897)

(31) Cyclophilin-Related Protein (Genbank No. HG846-HT846)

(32) Natural killer-tumor recognition sequence (Genbank No. L04288)

(33) Integrin alpha 6B (CD49f) (Genbank No. S66213)

(34) Homo sapiens clone 24629 mRNA sequence (Genbank No. AF052160)

(35) Protein kinase C Nu (EPK2) (Genbank No. AB015982)

(36) mRNA for SYT.SSX1 translocational target region of human synovial inducible sarcomas [Partial Mutant, 3' genes, 585nt] (Genbank No. S79325)

(37) Glucose transporter pseudogene (Genbank No. M55536)

(38) Nuclear receptor intermediary activation factor 2 (TIF2) (Genbank No. X97674)

(39) CRE-BP1 transcription factor (Genbank No. U16028)

(40) Topoisomerase type II (Topo II) (Genbank No. M27504)

(41) Nuclear respiratory factor-2 subunit alpha (Genbank No. U13044)

(42) PAC clone DJ1185I07 from 7q11.23-q21 RP5-1185I07 (Genbank No. AC004990)

(43) Cyclin T2b (Genbank No. AF048732)

(44) Zinc finger protein type C3H (MBLL) (Genbank No. AF061261)

(45) MEK kinase (Mekk) (Genbank No. U29671)

(46) Rod1 (Genbank No. AB023967)

(47) HSTXK Human tyrosine kinase (TXK) (Genbank No. U07794)

(48) Serine/threonine-protein kinase PRP4h (PRP4h) (Genbank No. U48736)

(49) pre-mRNA cleavage factor I subunit Im (Genbank No. AJ001810)

(50) Homo sapiens cDNA, 3'end/clone=IMAGE-2512364, EST wt65e11.×1 (Genbank No. AI961669)

(51) Disintegrin-metalloprotease (Genbank No. Z48579)

(52) ADP-ribosylation factor no.6 (ARF6) (Genbank No. AF047432)

(53) Helicase like protein 2 containing DEAD/H box (DDX14) (Genbank No. U50553)

(54) p300/CBP-associated factor (P/CAF) (Genbank No. U57317)

(55) Ribosomal protein S6 kinase (ISPK-1) (Genbank No. U08316)

(56) Cyclin G1 (Genbank No. X77794)

(57) Guanine binding protein type q (Gaq) (Genbank No. U43083)

(58) Trinucleotide repeat CGG-DNA binding protein p20-CGGBP (CGGBP) (Genbank No. AF094481)

(59) Integrin alpha 4 subunit (CD49d) (Genbank No. L12002)

(60) Chromosome 5q21-22, clone-A3-A (Genbank No. AB002450)

(61) Unknown protein of uterine endometrium (Genbank No. X77723)

(62) Transcription factor ISGF-3 (STAT91) (Genbank No. M97935)

(63) Human PAC clone DJ525N14 from Xq23 RP3-525N14 (Genbank No. AC002086)

(64) SH2 domain protein 1A isoform B (SH2D1A) (Genbank No. AF100539)

(65) Killer cell lectin-like receptor NKG2F (Genbank No. AJ001683)

(66) Human homolog of Drosophila discs gene, isoform 2 (hdlg-2) (Genbank No. U13896)

(67) Human SNF1-like protein kinase (Genbank No. U57452)

(68) Human DNA for c-ets-1 proto-oncogene (Genbank No. X14798)

(69) EAR-1r (Genbank No. D16815)

(70) Guanine nucleotide regulatory protein (G alpha 13) (Genbank No. L22075)

(71) Retinoblastoma susceptibility protein (RB1) (Genbank No. L49229)

(72) SH-PTP3 for protein-tyrosine phosphatase (Genbank No. D13540)

(73) EST 14e9 Homo sapiens cDNA (Genbank No. W25874)

(74) MDM2-like p53-binding protein (MDMX) (Genbank No. AF007111)

(75) Homo sapiens cDNA, 5'end/clone=IMAGE-360208, EST ze27c09.r1 (Genbank No. AA013087)

(76) Erythroblastosis virus oncogene homolog 1 (ets-1) (Genbank No. J04101)

(77) HUMM9, Man9-mannosidase (Genbank No. X74837)

(78) Kinesin/heavy chain 5B (Genbank No. X65873)

(79) Interferon-inducible RNA-dependent protein kinase (Pkr) (Genbank No. U50648)

(80) Interferon regulatory factor-2 (IRF-2) (Genbank No. X15949)

(81) Homo sapiens cDNA, 3'end/clone=IMAGE-1722789, EST qd04h11.×1 (Genbank No. AI189226)

(82) Chondroitin sulfate proteoglycan PG-M (bursicon) (Genbank No. D32039)

(83) Homo sapiens mRNA; cDNA DKFZp564P0823 (from clone DKFZp564P0823) (Genbank No. AL049962)

(84) EST36b3 Homo sapiens cDNA (Genbank No. W27675)

(85) Homo sapiens cDNA, 3'end/clone=IMAGE-2489058, EST wr28g10.×1 (Genbank No. AW006742)

(86) Homo sapiens cDNA, 3'end/clone=IMAGE-815515, EST aa 38b10.s1 (Genbank No. AA457029)

(87) c-myc proto-oncogene (MYCL2) (Genbank No. J03069)

(88) Mature T cell proliferation factor c6.1B gene; MTCP1 gene (Genbank No. Z24459)

(89) Homo sapiens mRNA for KIAA0797 protein (Genbank No. AB018340)

(90) N-ras (Genbank No. X02751)

(91) WD repeat protein HAN11 (Genbank No. U94747)

(92) Homo sapiens mRNA for KIAA1048 protein (Genbank No. AB028971)

(93) Homo sapiens mRNA for KIAA0454 protein (Genbank No. AB007923)

(94) Cystine/glutamate transporter (Genbank No. AB026891)

(95) Microsomal stress 70 protein ATPase core (stch) (Genbank No. U04735)

(96) cAMP-dependent protein kinase catalytic subunit type alpha (EC 2.7.1.37) (Genbank No. X07767)

(97) Homo sapiens cDNA, 5'end/clone=IMAGE-2497327 (Genbank No AW003733)

(98) Homo sapiens cDNA, 5'end/clone=IMAGE-487691 (Genbank No. AA058762)

(99) Monoamine oxidase B (MAOB) (Genbank No. M69177)

(100) lipocortin-III (annexins A3) (Genbank No. M20560)

(101) Homo sapiens chromosome 1 specific transcript KIAA0508 (Genbank No. AB007977)

(102) Platelet-activating factor receptor (Genbank No. D10202)

(103) EST DKFZp586A2224_s1 Homo sapiens cDNA (Genbank No. AL048308)

(104) PCTAIRE-1 for serine/threonine protein kinase (Genbank No. X66363)

(105) Gelsolin; macrophage capping protein; villin (Genbank No. M94345)

(106) EST 31c9 Homo sapiens cDNA (Genbank No. W27466)

(107) Diaphanous type 2 isoform 12C protein, dia-156 protein (DIA-156) (Genbank No. Y15909)

(108) Insulin receptor precursor (Genbank No. X02160)

(109) Heregulin type 1 (HRG alpha) (Genbank No. L41827)

(110) Branched chain alpha-ketoacid dehydrogenase kinase precursor (BCKD kinase) (Genbank No. AF026548)

(111) Electron transfer flavoprotein beta subunit (Genbank No. X71129)

(112) p160 (Genbank No. U88153)

(113) Calciceurin dependently activated T cell nuclear factor (NF-Atc) (Genbank No. U08015)

(114) Homo sapiens mRNA for KIAA0563 protein (Genbank No. AB011135)

(115) Vscular smooth muscle alpha-actin (Genbank No. X13839)

(116) Rad17-like protein (RAD17) (Genbank No. AF076838)

(117) Homo sapiens cDNA, 3'end/clone=IMAGE-2394055, EST wi54d04.×1 (Genbank No. AI762213)

(118) Homo sapiens cDNA, 3 end/clone=IMAGE-979142, EST ni38e08.s1 (Genbank No. AA522537)

(119) Human T54 protein (T54) (Genbank No. U66359)

(120) Acyl-CoA dehydrogenase; SCAD gene (Genbank No. Z80345)

(121) Phosphomevalonate kinase (Genbank No. L77213)

(122) Drebrin E (Genbank No. D17530)

(123) Receptor protein-tyrosine kinase EphA4 (HEK8) (Genbank No. L36645)

(124) Tob family transducer ERBB2,2 (Genbank No. D64109)

(125) Homo sapiens cDNA, 3 end/clone=IMAGE-1657913, ESTox31b09.s1 (Genbank No. AI039144)

(126) Homogentisate 1,2-dioxygenase (Genbank No. AF000573)

(127) MFH-proliferation sequence (MASL1) (Genbank No. AB016816)

(128) Homo sapiens mRNA for KIAA0994 protein (Genbank No. AB023211)

(129) Homo sapiens cDNA, 3 end/clone=IMAGE-826408, EST aa71e09.s1 (Genbank No. AA521060)

(130) Neutrophil cytoplasmic factor type 4 (p40phox) (Genbank No. X77094)

(131) Mucin 5b (Genbank No. HG2689-HT2785)

(132) Homo sapiens cDNA, 3 end/clone=IMAGE-965972, EST nh92c11.s1 (Genbank No. AA528252)

(133) Cell adhesion protein (vitronectin) receptor alpha subunit (CD51) (Genbank No. M14648)

(134) Cluster Incl AL049435:Homo sapiens mRNA; cDNA DKFZp586B0220 (from clone DKFZp586B0220 (Genbank No. AL049435)

(135) Homo sapiens (clone S164) mRNA, 3 end of cds/cds (Genbank No. L40392)

(136) mRNA for KIAA1009 protein (Genbank No. AB023226)

(137) mRNA for KIAA0716 protein (Genbank No. AB018259)

(138) Vanin-like gene; vnn1 gene; VNN1 protein (Genbank No. AJ132099)

(139) Homo sapiens PAC clone DJ0808A01 from 7q21.1-q31.1 (Genbank No. AC004893)

(140) Homo sapiens mRNA for KIAA1050 protein (Genbank No. AB028973)

(141) Human Chromosome 16 BAC clone CIT987SK-A-270G1 (Genbank No. AF001549)

(142) Transcriptional factor TREB protein (Genbank No. X55544)

(143) Homo sapiens mRNA for KIAA0548 protein (Genbank No. AB011120)

(144) p300; transcriptional adaptor protein; E1A-binding protein (Genbank No. U01877)

(145) Integrin alpha E precursor (CD103) (L25851)

(146) Homo sapiens cDNA, 3 end/clone=IMAGE-1714897 EST qc69h01.x1 (Genbank No. AI148772)

(147) Homo sapiens mRNA full length insert cDNA clone EUROIMAGE 417629 (Genbank No. AL109724)

(148) Defensins alpha 3 (Genbank No. L12691)

(149) Homo sapiens cDNA, 5' end/clone=DKFZp564J2262-r1 (Genbank No. AL036554)

(150) Elastase/medullasin (Genbank No. M34379)

(151) Angelman Syndrome Gene, E6-AP ubiquitin protein ligase 3A (UBE3A) (Genbank No. AF002224)

(152) Skeletal muscle 165kD protein (Genbank No. X69089)

4. A method for diagnosing whether a test subject suffers from schizophrenia or not, the method comprising the steps of;

obtaining mononuclear cells in blood containing nucleic acid from said subject,

measuring the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and

determining alteration of the quantified level(s) of the gene(s) in said test subject is statistically significant in comparison with the quantified level(s) of said nucleic acid(s) defining gene(s) exhibiting altered expression by occurrence of schizophrenia or said nucleic acid(s) defining gene(s) exhibiting altered expression by progression of schizophrenia in healthy subjects or schizophrenic patients, thereby diagnosing whether said subject is suffering from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) defined by the gene name, the protein name which is a gene product, or the nucleic acid sequence name as described below in (1) to (152) with GenBank No. described in brackets.

(1) Homo sapiens cDNA, 3'end/clone= IMAGE-2329930, EST wd33c06.×1 (Genbank No. AI677689)

(2) Bcl-Xl (Genbank No. Z23115)

(3) ZNF37A mRNA for zinc finger (Genbank No. X69115)

(4) HSCCG1 Human X chromsome mRNA for CCG1 protein inv. in cell proliferation (Genbank No. X07024)

(5) Interferon receptor type 2 (IFNAR2) (Genbank No. L42243)

(6) Guanine Nucleotide Exchange Factor 1 (Genbank No. HG960-HT960)

(7) Glucosamine-6-sulphatase precursor (Genbank No. Z12173)

(8) MACH-beta-1 protein (Caspase 8)(Genbank No. X98176)

(9) EST 15a11 Homo sapiens cDNA/gb=W25921/gi=1306044/ug= Hs.164036/len=723 (Genbank No. W25921)

(10) Ndr protein kinase (Genbank No. Z35102)

(11) 14-3-3 protein (Genbank No. U28964)

(12) RbAp48 mRNA encoding retinoblastoma binding protein (Genbank No. X74262)

(13) SNAP23B protein (Genbank No. Y09568)

(14) Inhibitory protein for potassium-induced deficiency type 1 (SKD1 homolog) (Genbank No. AF038960)

(15) Homo sapiens cDNA, 3'end/clone=IMAGE-2509049, ETS wt31b09.×1 (Genbank No. AI955897)

(16) Utrophin (Genbank No. X69086)

(17) cdc2-related protein kinase (Genbank No. M80629)

(18) Calmodulin type 1 (CALM1) (Genbank No. U12022)

(19) Rb2/p130 protein (Genbank No. X74594)

(20) Protein-tyrosine kinase JAK1 (Genbank No. M64174)

(21) GTP-binding protein RAB6 (Genbank No. M28212)

(22) Clq/MBL/SPA receptor C1qR(p) (Genbank No. U94333)

(23) Zinc finger/leucine zipper protein (AF10)(Genbank No. U13948)

(24) Inositol polyphosphate 4-phosphatase type I-beta (Genbank No. U96919)

(25) Inositol polyphosphate 4-phosphatase (Genbank No. U26398)

(26) Cytohesin binding protein HE (Genbank No. AF068836)

(27) Cas like protein for enhancer of filamentation (HEF1) (Genbank No. L43821)

(28) Rho GTPase-activated protein type 5 (p190-B) (Genbank No. U17032)

(29) AMP-activated protein kinase alpha-1 (Genbank No. AB022017)

(30) Syntaxin 16 (Genbank No. AF038897)

(31) Cyclophilin-Related Protein (Genbank No. HG846-HT846)

(32) Natural killer-tumor recognition sequence (Genbank No. L04288)

(33) Integrin alpha 6B (CD49f) (Genbank No. S66213)

(34) Homo sapiens clone 24629 mRNA sequence (Genbank No. AF052160)

(35) Protein kinase C Nu (EPK2) (Genbank No. AB015982)

(36) mRNA for SYT.SSX1 translocational target region of human synovial inducible sarcomas [Partial Mutant, 3' genes, 585nt] (Genbank No. S79325)

(37) Glucose transporter pseudogene (Genbank No. M55536)

(38) Nuclear receptor intermediary activation factor 2 (TIF2) (Genbank No. X97674)

(39) CRE-BP1 transcription factor (Genbank No. U16028)

(40) Topoisomerase type II (Topo II) (Genbank No. M27504)

(41) Nuclear respiratory factor-2 subunit alpha (Genbank No. U13044)

(42) PAC clone DJ1185I07 from 7q11.23-q21 RP5-1185I07 (Genbank No. AC004990)

(43) Cyclin T2b (Genbank No. AF048732)

(44) Zinc finger protein type C3H (MBLL) (Genbank No. AF061261)

(45) MEK kinase (Mekk) (Genbank No. U29671)

(46) Rod1 (Genbank No. AB023967)

(47) HSTXK Human tyrosine kinase (TXK) (Genbank No. U07794)

(48) Serine/threonine-protein kinase PRP4h (PRP4h) (Genbank No. U48736)

(49) pre-mRNA cleavage factor I subunit Im (Genbank No. AJ001810)

(50) Homo sapiens cDNA, 3'end/clone=IMAGE-2512364, EST wt65e11.×1 (Genbank No. AI961669)

(51) Disintegrin-metalloprotease (Genbank No. Z48579)

(52) ADP-ribosylation factor no.6 (ARF6) (Genbank No. AF047432)

(53) Helicase like protein 2 containing DEAD/H box (DDX14) (Genbank No. U50553)

(54) p300/CBP-associated factor (P/CAF) (Genbank No. U57317)

(55) Ribosomal protein S6 kinase (ISPK-1) (Genbank No. U08316)

(56) Cyclin G1 (Genbank No. X77794)

(57) Guanine binding protein type q (Gaq) (Genbank No. U43083)

(58) Trinucleotide repeat CGG-DNA binding protein p20-CGGBP (CGGBP) (Genbank No. AF094481)

(59) Integrin alpha 4 subunit (CD49d) (Genbank No. L12002)

(60) Chromosome 5q21-22, clone-A3-A (Genbank No. AB002450)

(61) Unknown protein of uterine endometrium (Genbank No. X77723)

(62) Transcription factor ISGF-3 (STAT91) (Genbank No. M97935)

(63) Human PAC clone DJ525N14 from Xq23 RP3-525N14 (Genbank No. AC002086)

(64) SH2 domain protein 1A isoform B (SH2D1A) (Genbank No. AF100539)

(65) Killer cell lectin-like receptor NKG2F (Genbank No. AJ001683)

(66) Human homolog of Drosophila discs gene, isoform 2 (hdlg-2) (Genbank No. U13896)

(67) Human SNF1-like protein kinase (Genbank No. U57452)

(68) Human DNA for c-ets-1 proto-oncogene (Genbank No. X14798)

(69) EAR-1r (Genbank No. D16815)

(70) Guanine nucleotide regulatory protein (G alpha 13) (Genbank No. L22075)

(71) Retinoblastoma susceptibility protein (RB1) (Genbank No. L49229)

(72) SH-PTP3 for protein-tyrosine phosphatase (Genbank No. D13540)

(73) EST 14e9 Homo sapiens cDNA (Genbank No. W25874)

(74) MDM2-like p53-binding protein (MDMX) (Genbank No. AF007111)

(75) Homo sapiens cDNA, 5'end/clone=IMAGE-360208, EST ze27c09.r1 (Genbank No. AA013087)

(76) Erythroblastosis virus oncogene homolog 1 (ets-1) (Genbank No. J04101)

(77) HUMM9, Man9-mannosidase (Genbank No. X74837)

(78) Kinesin/heavy chain 5B (Genbank No. X65873)

(79) Interferon-inducible RNA-dependent protein kinase (Pkr) (Genbank No. U50648)

(80) Interferon regulatory factor-2 (IRF-2) (Genbank No. X15949)

(81) Homo sapiens cDNA, 3'end/clone=IMAGE-1722789, EST qd04h11.×1 (Genbank No. AI189226)

(82) Chondroitin sulfate proteoglycan PG-M (bursicon) (Genbank No. D32039)

(83) Homo sapiens mRNA; cDNA DKFZp564P0823 (from clone DKFZp564P0823) (Genbank No. AL049962)

(84) EST36b3 Homo sapiens cDNA (Genbank No. W27675)

(85) Homo sapiens cDNA, 3'end/clone=IMAGE-2489058, EST wr28g10.×1 (Genbank No. AW006742)

(86) Homo sapiens cDNA, 3'end/clone=IMAGE-815515, EST aa 38b10.s1 (Genbank No. AA457029)

(87) c-myc proto-oncogene (MYCL2) (Genbank No. J03069)

(88) Mature T cell proliferation factor c6.1B gene; MTCP1 gene (Genbank No. Z24459)

(89) Homo sapiens mRNA for KIAA0797 protein (Genbank No. AB018340)

(90) N-ras (Genbank No. X02751)

(91) WD repeat protein HAN11 (Genbank No. U94747)

(92) Homo sapiens mRNA for KIAA1048 protein (Genbank No. AB028971)

(93) Homo sapiens mRNA for KIAA0454 protein (Genbank No. AB007923)

(94) Cystine/glutamate transporter (Genbank No. AB026891)

(95) Microsomal stress 70 protein ATPase core (stch) (Genbank No. U04735)

(96) cAMP-dependent protein kinase catalytic subunit type alpha (EC 2.7.1.37) (Genbank No. X07767)

(97) Homo sapiens cDNA, 5'end/clone=IMAGE-2497327 (Genbank No AW003733)

(98) Homo sapiens cDNA, 5'end/clone=IMAGE-487691 (Genbank No. AA058762)

(99) Monoamine oxidase B (MAOB) (Genbank No. M69177)

(100) lipocortin-III (annexins A3) (Genbank No. M20560)

(101) Homo sapiens chromosome 1 specific transcript KIAA0508 (Genbank No. AB007977)

(102) Platelet-activating factor receptor (Genbank No. D10202)

(103) EST DKFZp586A2224_s1 Homo sapiens cDNA (Genbank No. AL048308)

(104) PCTAIRE-1 for serine/threonine protein kinase (Genbank No. X66363)

(105) Gelsolin; macrophage capping protein; villin (Genbank No. M94345)

(106) EST 31c9 Homo sapiens cDNA (Genbank No. W27466)

(107) Diaphanous type 2 isoform 12C protein, dia-156 protein (DIA-156) (Genbank No. Y15909)

(108) Insulin receptor precursor (Genbank No. X02160)

(109) Heregulin type 1 (HRG alpha) (Genbank No. L41827)

(110) Branched chain alpha-ketoacid dehydrogenase kinase precursor (BCKD kinase) (Genbank No. AF026548)

(111) Electron transfer flavoprotein beta subunit (Genbank No. X71129)

(112) p160 (Genbank No. U88153)

(113) Calciceurin dependently activated T cell nuclear factor (NF-Atc) (Genbank No. U08015)

(114) Homo sapiens mRNA for KIAA0563 protein (Genbank No. AB011135)

(115) Vscular smooth muscle alpha-actin (Genbank No. X13839)

(116) Rad17-like protein (RAD17) (Genbank No. AF076838)

(117) Homo sapiens cDNA, 3'end/clone=IMAGE-2394055, EST wi54d04.×1 (Genbank No. AI762213)

(118) Homo sapiens cDNA, 3 end/clone=IMAGE-979142, EST ni38e08.s1 (Genbank No. AA522537)

(119) Human T54 protein (T54) (Genbank No. U66359)

(120) Acyl-CoA dehydrogenase; SCAD gene (Genbank No. Z80345)

(121) Phosphomevalonate kinase (Genbank No. L77213)

(122) Drebrin E (Genbank No. D17530)

(123) Receptor protein-tyrosine kinase EphA4 (HEK8) (Genbank No. L36645)

(124) Tob family transducer ERBB2,2 (Genbank No. D64109)

(125) Homo sapiens cDNA, 3 end/clone=IMAGE-1657913, ESTox31b09.s1 (Genbank No. AI039144)

(126) Homogentisate 1,2-dioxygenase (Genbank No. AF000573)

(127) MFH-proliferation sequence (MASL1) (Genbank No. AB016816)

(128) Homo sapiens mRNA for KIAA0994 protein (Genbank No. AB023211)

(129) Homo sapiens cDNA, 3 end/clone=IMAGE-826408, EST aa71e09.s1 (Genbank No. AA521060)

(130) Neutrophil cytoplasmic factor type 4 (p40phox) (Genbank No. X77094)

(131) Mucin 5b (Genbank No. HG2689-HT2785)

(132) Homo sapiens cDNA, 3 end/clone=IMAGE-965972, EST nh92c11.s1 (Genbank No. AA528252)

(133) Cell adhesion protein (vitronectin) receptor alpha subunit (CD51) (Genbank No. M14648)

(134) Cluster Incl AL049435:Homo sapiens mRNA; cDNA DKFZp586B0220 (from clone DKFZp586B0220 (Genbank No. AL049435)

(135) Homo sapiens (clone S164) mRNA, 3 end of cds /cds (Genbank No. L40392)

(136) mRNA for KIAA1009 protein (Genbank No. AB023226)

(137) mRNA for KIAA0716 protein (Genbank No. AB018259)

(138) Vanin-like gene; vnn1 gene; VNN1 protein (Genbank No. AJ132099)

(139) Homo sapiens PAC clone DJ0808A01 from 7q21.1-q31.1 (Genbank No. AC004893)

(140) Homo sapiens mRNA for KIAA1050 protein (Genbank No. AB028973)

(141) Human Chromosome 16 BAC clone CIT987SK-A-270G1 (Genbank No. AF001549)

(142) Transcriptional factor TREB protein (Genbank No. X55544)

(143) Homo sapiens mRNA for KIAA0548 protein (Genbank No. AB011120)

(144) p300; transcriptional adaptor protein; E1A-binding protein (Genbank No. U01877)

(145) Integrin alpha E precursor (CD103) (L25851)

(146) Homo sapiens cDNA, 3 end/clone=IMAGE-1714897 EST qc69h01.x1 (Genbank No. AI148772)

(147) Homo sapiens mRNA full length insert cDNA clone EUROIMAGE 417629 (Genbank No. AL109724)

(148) Defensins alpha 3 (Genbank No. L12691)

(149) Homo sapiens cDNA, 5' end/clone=DKFZp564J2262-r1 (Genbank No. AL036554)

(150) Elastase/medullasin (Genbank No. M34379)

(151) Angelman Syndrome Gene, E6-AP ubiquitin protein ligase 3A (UBE3A) (Genbank No. AF002224)

(152) Skeletal muscle 165kD protein (Genbank No. X69089)

5. The method according to Claim 4, wherein expression levels of 2 to 50 kinds of genes derived from said nucleic acid(s) defining gene(s) exhibiting altered expression by occurrence of schizophrenia or said nucleic acid(s) defining gene(s) exhibiting altered expression by progression of schizophrenia are utilized as an index for diagnosing whether the test subject suffers from schizophrenia or not.

6. The method according to Claim 4, wherein expression levels of 2 to 20 kinds of genes derived from said nucleic acid(s) defining gene(s) exhibiting altered expression by occurrence of schizophrenia or said nucleic acid(s) defining gene(s) exhibiting altered expression by progression of schizophrenia are utilized as an index for diagnosing whether the test subject suffers from schizophrenia or not.

7. The method according to Claim 4, wherein expression levels of 2 to 10 kinds of genes derived from said nucleic acid(s) defining gene(s) exhibiting altered expression by occurrence of schizophrenia or said nucleic acid(s) defining gene(s) exhibiting altered expression by progression of schizophrenia are utilized as an index for diagnosing whether the test subject suffers from schizophrenia or not.

8. The method according to Claim 4, wherein expression levels of 1 kind of gene derived from said nucleic acid(s) defining gene(s) exhibiting altered expression by occurrence of schizophrenia or said nucleic acid(s) defining gene(s) exhibiting altered expression by progression of schizophrenia is utilized as an index for diagnosing whether the test subject suffers from schizophrenia or not.

9. A method for analyzing whether or not the expression level(s) of nucleic acid(s) defining gene(s) exhibiting altered expression by occurrence of schizophrenia or nucleic acid(s) defining gene(s) exhibiting altered expression by progression of schizophrenia in a test subject is statistically excluded from the range of the expression level(s) of the nucleic acid(s) in schizophrenic patients, the method comprising the steps of;

obtaining mononuclear cells in blood containing nucleic acid from said test subject,

measuring the content of at least one nucleic acid selected from the group consisting of the nucleic acid(s) (containing its fragment and a nucleic acid complementary to the nucleic acid) defining gene(s) exhibiting altered expression by occurrence of schizophrenia or the nucleic acid(s) (containing its fragment and a nucleic acid complementary to the nucleic acid) defining gene(s) exhibiting altered expression by progression of schizophrenia in said mononuclear cells, and

comparing the determined level(s) in said test subject with the determined level(s) of said nucleic acid(s) defining gene(s) exhibiting altered expression by occurrence of schizophrenia or said nucleic acid(s) defining gene(s) exhibiting altered expression by progression of schizophrenia in healthy subjects or in schizophrenic patients to analyze whether the alteration of said determined level in said test subject is statistically significant or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) defined by the gene name, the protein name which is a gene product, or the nucleic acid sequence name as described below in (1) to (152) with GenBank No. described in brackets.

(1) Homo sapiens cDNA, 3'end/clone= IMAGE-2329930, EST wd33c06.×1 (Genbank No. AI677689)

(2) Bcl-Xl (Genbank No. Z23115)

(3) ZNF37A mRNA for zinc finger (Genbank No. X69115)

(4) HSCCG1 Human X chromsome mRNA for CCG1 protein inv. in cell proliferation (Genbank No. X07024)

(5) Interferon receptor type 2 (IFNAR2) (Genbank No. L42243)

(6) Guanine Nucleotide Exchange Factor 1 (Genbank No. HG960-HT960)

(7) Glucosamine-6-sulphatase precursor (Genbank No. Z12173)

(8) MACH-beta-1 protein (Caspase 8)(Genbank No. X98176)

(9) EST 15a11 Homo sapiens cDNA/gb=W25921/gi=1306044/ug =Hs.164036 /len=723 (Genbank No. W25921)

(10) Ndr protein kinase (Genbank No. Z35102)

(11) 14-3-3 protein (Genbank No. U28964)

(12) RbAp48 mRNA encoding retinoblastoma binding protein (Genbank No. X74262)

(13) SNAP23B protein (Genbank No. Y09568)

(14) Inhibitory protein for potassium-induced deficiency type 1 (SKD1 homolog) (Genbank No. AF038960)

(15) Homo sapiens cDNA, 3'end/clone=IMAGE-2509049, ETS wt31b09.×1 (Genbank No. AI955897)

(16) Utrophin (Genbank No. X69086)

(17) cdc2-related protein kinase (Genbank No. M80629)

(18) Calmodulin type 1 (CALM1) (Genbank No. U12022)

(19) Rb2/p130 protein (Genbank No. X74594)

(20) Protein-tyrosine kinase JAK1 (Genbank No. M64174)

(21) GTP-binding protein RAB6 (Genbank No. M28212)

(22) Clq/MBL/SPA receptor C1qR(p) (Genbank No. U94333)

(23) Zinc finger/leucine zipper protein (AF10)(Genbank No. U13948)

(24) Inositol polyphosphate 4-phosphatase type I-beta (Genbank No. U96919)

(25) Inositol polyphosphate 4-phosphatase (Genbank No. U26398)

(26) Cytohesin binding protein HE (Genbank No. AF068836)

(27) Cas like protein for enhancer of filamentation (HEF1) (Genbank No. L43821)

(28) Rho GTPase-activated protein type 5 (p190-B) (Genbank No. U17032)

(29) AMP-activated protein kinase alpha-1 (Genbank No. AB022017)

(30) Syntaxin 16 (Genbank No. AF038897)

(31) Cyclophilin-Related Protein (Genbank No. HG846-HT846)

(32) Natural killer-tumor recognition sequence (Genbank No. L04288)

(33) Integrin alpha 6B (CD49f) (Genbank No. S66213)

(34) Homo sapiens clone 24629 mRNA sequence (Genbank No. AF052160)

(35) Protein kinase C Nu (EPK2) (Genbank No. AB015982)

(36) mRNA for SYT.SSX1 translocational target region of human synovial inducible sarcomas [Partial Mutant, 3' genes, 585nt] (Genbank No. S79325)

(37) Glucose transporter pseudogene (Genbank No. M55536)

(38) Nuclear receptor intermediary activation factor 2 (TIF2) (Genbank No. X97674)

(39) CRE-BP1 transcription factor (Genbank No. U16028)

(40) Topoisomerase type II (Topo II) (Genbank No. M27504)

(41) Nuclear respiratory factor-2 subunit alpha (Genbank No. U13044)

(42) PAC clone DJ1185I07 from 7q11.23-q21 RP5-1185I07 (Genbank No. AC004990)

(43) Cyclin T2b (Genbank No. AF048732)

(44) Zinc finger protein type C3H (MBLL) (Genbank No. AF061261)

(45) MEK kinase (Mekk) (Genbank No. U29671)

(46) Rod1 (Genbank No. AB023967)

(47) HSTXK Human tyrosine kinase (TXK) (Genbank No. U07794)

(48) Serine/threonine-protein kinase PRP4h (PRP4h) (Genbank No. U48736)

(49) pre-mRNA cleavage factor I subunit Im (Genbank No. AJ001810)

(50) Homo sapiens cDNA, 3'end/clone=IMAGE-2512364, EST wt65e11.×1 (Genbank No. AI961669)

(51) Disintegrin-metalloprotease (Genbank No. Z48579)

(52) ADP-ribosylation factor no.6 (ARF6) (Genbank No. AF047432)

(53) Helicase like protein 2 containing DEAD/H box (DDX14) (Genbank No. U50553)

(54) p300/CBP-associated factor (P/CAF) (Genbank No. U57317)

(55) Ribosomal protein S6 kinase (ISPK-1) (Genbank No. U08316)

(56) Cyclin G1 (Genbank No. X77794)

(57) Guanine binding protein type q (Gaq) (Genbank No. U43083)

(58) Trinucleotide repeat CGG-DNA binding protein p20-CGGBP (CGGBP) (Genbank No. AF094481)

(59) Integrin alpha 4 subunit (CD49d) (Genbank No. L12002)

(60) Chromosome 5q21-22, clone-A3-A (Genbank No. AB002450)

(61) Unknown protein of uterine endometrium (Genbank No. X77723)

(62) Transcription factor ISGF-3 (STAT91) (Genbank No. M97935)

(63) Human PAC clone DJ525N14 from Xq23 RP3-525N14 (Genbank No. AC002086)

(64) SH2 domain protein 1A isoform B (SH2D1A) (Genbank No. AF100539)

(65) Killer cell lectin-like receptor NKG2F (Genbank No. AJ001683)

(66) Human homolog of Drosophila discs gene, isoform 2 (hdlg-2) (Genbank No. U13896)

(67) Human SNF1-like protein kinase (Genbank No. U57452)

(68) Human DNA for c-ets-1 proto-oncogene (Genbank No. X14798)

(69) EAR-1r (Genbank No. D16815)

(70) Guanine nucleotide regulatory protein (G alpha 13) (Genbank No. L22075)

(71) Retinoblastoma susceptibility protein (RB1) (Genbank No. L49229)

(72) SH-PTP3 for protein-tyrosine phosphatase (Genbank No. D13540)

(73) EST 14e9 Homo sapiens cDNA (Genbank No. W25874)

(74) MDM2-like p53-binding protein (MDMX) (Genbank No. AF007111)

(75) Homo sapiens cDNA, 5'end/clone=IMAGE-360208, EST ze27c09.r1 (Genbank No. AA013087)

(76) Erythroblastosis virus oncogene homolog 1 (ets-1) (Genbank No. J04101)

(77) HUMM9, Man9-mannosidase (Genbank No. X74837)

(78) Kinesin/heavy chain 5B (Genbank No. X65873)

(79) Interferon-inducible RNA-dependent protein kinase (Pkr) (Genbank No. U50648)

(80) Interferon regulatory factor-2 (IRF-2) (Genbank No. X15949)

(81) Homo sapiens cDNA, 3'end/clone=IMAGE-1722789, EST qd04h11.$\times$1 (Genbank No. AI189226)

(82) Chondroitin sulfate proteoglycan PG-M (bursicon) (Genbank No. D32039)

(83) Homo sapiens mRNA; cDNA DKFZp564P0823 (from clone DKFZp564P0823) (Genbank No. AL049962)

(84) EST36b3 Homo sapiens cDNA (Genbank No. W27675)

(85) Homo sapiens cDNA, 3'end/clone=IMAGE-2489058, EST wr28g10.$\times$1 (Genbank No. AW006742)

(86) Homo sapiens cDNA, 3'end/clone=IMAGE-815515, EST aa 38b10.s1 (Genbank No. AA457029)

(87) c-myc proto-oncogene (MYCL2) (Genbank No. J03069)

(88) Mature T cell proliferation factor c6.1B gene; MTCP1 gene (Genbank No. 224459)

(89) Homo sapiens mRNA for KIAA0797 protein (Genbank No. AB018340)

(90) N-ras (Genbank No. X02751)

(91) WD repeat protein HAN11 (Genbank No. U94747)

(92) Homo sapiens mRNA for KIAA1048 protein (Genbank No. AB028971)

(93) Homo sapiens mRNA for KIAA0454 protein (Genbank No. AB007923)

(94) Cystine/glutamate transporter (Genbank No. AB026891)

(95) Microsomal stress 70 protein ATPase core (stch) (Genbank No. U04735)

(96) cAMP-dependent protein kinase catalytic subunit type alpha (EC 2.7.1.37) (Genbank No. X07767)

(97) Homo sapiens cDNA, 5'end/clone=IMAGE-2497327 (Genbank No AW003733)

(98) Homo sapiens cDNA, 5'end/clone=IMAGE-487691 (Genbank No. AA058762)

(99) Monoamine oxidase B (MAOB) (Genbank No. M69177)

(100) lipocortin-III (annexins A3) (Genbank No. M20560)

(101) Homo sapiens chromosome 1 specific transcript KIAA0508 (Genbank No. AB007977)

(102) Platelet-activating factor receptor (Genbank No. D10202)

(103)EST DKFZp586A2224_s1 Homo sapiens cDNA (Genbank No. AL048308)

(104) PCTAIRE-1 for serine/threonine protein kinase (Genbank No. X66363)

(105) Gelsolin; macrophage capping protein; villin (Genbank No. M94345)

(106) EST 31c9 Homo sapiens cDNA (Genbank No. W27466)

(107) Diaphanous type 2 isoform 12C protein, dia-156 protein (DIA-156) (Genbank No. Y15909)

(108) Insulin receptor precursor (Genbank No. X02160)

(109) Heregulin type 1 (HRG alpha) (Genbank No. L41827)

(110) Branched chain alpha-ketoacid dehydrogenase kinase precursor (BCKD kinase) (Genbank No. AF026548)

(111) Electron transfer flavoprotein beta subunit (Genbank No. X71129)

(112) p160 (Genbank No. U88153)

(113) Calciceurin dependently activated T cell nuclear factor (NF-Atc) (Genbank No. U08015)

(114) Homo sapiens mRNA for KIAA0563 protein (Genbank No. AB011135)

(115) Vscular smooth muscle alpha-actin (Genbank No. X13839)

(116) Rad17-like protein (RAD17) (Genbank No. AF076838)

(117) Homo sapiens cDNA, 3'end/clone=IMAGE-2394055, EST wi54d04.$\times$1 (Genbank No. AI762213)

(118) Homo sapiens cDNA, 3 end/clone=IMAGE-979142, EST ni38e08.s1 (Genbank No. AA522537)

(119) Human T54 protein (T54) (Genbank No. U66359)

(120) Acyl-CoA dehydrogenase; SCAD gene (Genbank No. Z80345)

(121) Phosphomevalonate kinase (Genbank No. L77213)

(122) Drebrin E (Genbank No. D17530)

(123) Receptor protein-tyrosine kinase EphA4 (HEK8) (Genbank No. L36645)

(124) Tob family transducer ERBB2,2 (Genbank No. D64109)

(125) Homo sapiens cDNA, 3 end/clone=IMAGE-1657913, ESTox31b09.s1 (Genbank No. AI039144)

(126) Homogentisate 1,2-dioxygenase (Genbank No. AF000573)

(127) MFH-proliferation sequence (MASL1) (Genbank No. AB016816)

(128) Homo sapiens mRNA for KIAA0994 protein (Genbank No. AB023211)

(129) Homo sapiens cDNA, 3 end/clone=IMAGE-826408, EST aa71e09.s1 (Genbank No. AA521060)

(130) Neutrophil cytoplasmic factor type 4 (p40phox) (Genbank No. X77094)

(131) Mucin 5b (Genbank No. HG2689-HT2785)

(132) Homo sapiens cDNA, 3 end/clone=IMAGE-965972, EST nh92c11.s1 (Genbank No. AA528252)

(133) Cell adhesion protein (vitronectin) receptor alpha subunit (CD51) (Genbank No. M14648)

(134) Cluster Incl AL049435:Homo sapiens mRNA; cDNA DKFZp586B0220 (from clone DKFZp586B0220 (Genbank No. AL049435)

(135) Homo sapiens (clone S164) mRNA, 3 end of cds/cds (Genbank No. L40392)

(136) mRNA for KIAA1009 protein (Genbank No. AB023226)

(137) mRNA for KIAA0716 protein (Genbank No. AB018259)

(138) Vanin-like gene; vnn1 gene; VNN1 protein (Genbank No. AJ132099)

(139) Homo sapiens PAC clone DJ0808A01 from 7q21.1-q31.1 (Genbank No. AC004893)

(140) Homo sapiens mRNA for KIAA1050 protein (Genbank No. AB028973)

(141) Human Chromosome 16 BAC clone CIT987SK-A-270G1 (Genbank No. AF001549)

(142) Transcriptional factor TREB protein (Genbank No. X55544)

(143) Homo sapiens mRNA for KIAA0548 protein (Genbank No. AB011120)

(144) p300; transcriptional adaptor protein; E1A-binding protein (Genbank No. U01877)

(145) Integrin alpha E precursor (CD103) (L25851)

(146) Homo sapiens cDNA, 3 end/clone=IMAGE-1714897 EST qc69h01.x1 (Genbank No. AI148772)

(147) Homo sapiens mRNA full length insert cDNA clone EUROIMAGE 417629 (Genbank No. AL109724)

(148) Defensins alpha 3 (Genbank No. L12691)

(149) Homo sapiens cDNA, 5' end/clone=DKFZp564J2262-r1 (Genbank No. AL036554)

(150) Elastase/medullasin (Genbank No. M34379)

(151) Angelman Syndrome Gene, E6-AP ubiquitin protein ligase 3A (UBE3A) (Genbank No. AF002224)

(152) Skeletal muscle 165kD protein (Genbank No. X69089)

10. The method according to Claim 9, wherein expression levels of 2 to 50 kinds of genes derived from said nucleic acid(s) defining gene(s) exhibiting altered expression by occurrence of schizophrenia or said nucleic acid(s) defining gene(s) exhibiting altered expression by progression of schizophrenia are utilized as an index for analyzing whether or not the expression level(s) of nucleic acid(s) defining gene(s) exhibiting altered expression by occurrence of schizophrenia or nucleic acid(s) defining gene(s) exhibiting altered expression by progression of schizophrenia is statistically excluded from the range of expression level(s) in schizophrenic patients.

11. The method according to Claim 9, wherein expression levels of 2 to 20 kinds of genes derived from said nucleic acid(s) defining gene(s) exhibiting altered expression by occurrence of schizophrenia or said nucleic acid(s) defining gene(s) exhibiting altered expression by progression of schizophrenia are utilized as an index for analyzing whether or not the expression level(s) of nucleic acid(s) defining gene(s) exhibiting altered expression by occurrence of schizophrenia or nucleic acid(s) defining gene(s) exhibiting altered expression by progression of schizophrenia is statistically excluded from the range of the expression level(s) in schizophrenic patients.

12. The method according to Claim 9, wherein expression levels of 2 to 10 kinds of genes derived from said nucleic acid(s) defining gene(s) exhibiting altered expression by occurrence of schizophrenia or said nucleic acid(s) defining gene(s) exhibiting altered expression by progression of schizophrenia are utilized as an index for analyzing whether or not the expression level(s) of nucleic acid(s) defining gene(s) exhibiting altered expression by occurrence of schizophrenia or nucleic acid(s) defining gene(s) exhibiting altered expression by progression of schizophrenia is statistically excluded from the range of the expression level(s) in schizophrenic patients.

13. The method according to Claim 9, wherein expression level of 1 kind of gene derived from said nucleic acid(s) defining gene(s) exhibiting altered expression by occurrence of schizophrenia or said nucleic acid(s) defining gene(s) exhibiting altered expression by progression of schizophrenia is utilized as an index for analyzing whether or not the expression level of nucleic acid(s) defining gene(s) exhibiting altered expression by occurrence of schizophrenia or nucleic acid(s) defining gene(s) exhibiting altered expression by progression of schizophrenia is statistically excluded from the range of the expression level in schizophrenic patients.

**14.** A method for diagnosing whether a test subject suffers from schizophrenia or not, the method comprising the steps of;

obtaining mononuclear cells in blood containing ribonucleic acid from said subject and extracting said ribonucleic acid from the blood,

immobilizing at least one nucleic acid in said mononuclear cells onto a DNA micro-array or a DNA chip as a probe, the nucleic acid being selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia,

determining the expression level(s) of said nucleic acid(s) defining gene(s) exhibiting altered expression by occurrence of schizophrenia or nucleic acid(s) defining gene(s) exhibiting altered expression by progression of schizophrenia all together using said DNA micro-array or said DNA chip immobilized with the nucleic acid, and

comparing the determined level(s) with the determined level(s) of said nucleic acid(s) defining gene(s) exhibiting altered expression by occurrence of schizophrenia or said nucleic acid(s) defining gene(s) exhibiting altered expression by progression of schizophrenia in healthy subjects or in schizophrenic patients to determine whether the alteration of said determined level(s) in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) defined by the gene name, the protein name which is a gene product, or the nucleic acid sequence name as described below in (1) to (152) with GenBank No. described in brackets.

(1) Homo sapiens cDNA, 3'end/clone= IMAGE-2329930, EST wd33c06.×1 (Genbank No. AI677689)
(2) Bcl-Xl (Genbank No. Z23115)
(3) ZNF37A mRNA for zinc finger (Genbank No. X69115)
(4) HSCCG1 Human X chromsome mRNA for CCG1 protein inv. in cell proliferation (Genbank No. X07024)
(5) Interferon receptor type 2 (IFNAR2) (Genbank No. L42243)
(6) Guanine Nucleotide Exchange Factor 1 (Genbank No. HG960-HT960)
(7) Glucosamine-6-sulphatase precursor (Genbank No. Z12173)
(8) MACH-beta-1 protein (Caspase 8)(Genbank No. X98176)
(9) EST 15a11 Homo sapiens cDNA /gb=W25921/gi=1306044/ug= Hs.164036/len=723 (Genbank No. W25921)
(10) Ndr protein kinase (Genbank No. Z35102)
(11) 14-3-3 protein (Genbank No. U28964)
(12) RbAp48 mRNA encoding retinoblastoma binding protein (Genbank No. X74262)
(13) SNAP23B protein (Genbank No. Y09568)
(14) Inhibitory protein for potassium-induced deficiency type 1 (SKD1 homolog) (Genbank No. AF038960)
(15) Homo sapiens cDNA, 3'end/clone=IMAGE-2509049, ETS wt31b09.x1 (Genbank No. AI955897)
(16) Utrophin (Genbank No. X69086)
(17) cdc2-related protein kinase (Genbank No. M80629)
(18) Calmodulin type 1 (CALM1) (Genbank No. U12022)
(19) Rb2/p130 protein (Genbank No. X74594)
(20) Protein-tyrosine kinase JAK1 (Genbank No. M64174)
(21) GTP-binding protein RAB6 (Genbank No. M28212)
(22) Clq/MBL/SPA receptor C1qR(p) (Genbank No. U94333)
(23) Zinc finger/leucine zipper protein (AF10)(Genbank No. U13948)
(24) Inositol polyphosphate 4-phosphatase type 1-beta (Genbank No. U96919)
(25) Inositol polyphosphate 4-phosphatase (Genbank No. U26398)
(26) Cytohesin binding protein HE (Genbank No. AF068836)
(27) Cas like protein for enhancer of filamentation (HEF1) (Genbank No. L43821)
(28) Rho GTPase-activated protein type 5 (p190-B) (Genbank No. U17032)
(29) AMP-activated protein kinase alpha-1 (Genbank No. AB022017)
(30) Syntaxin 16 (Genbank No. AF038897)
(31) Cyclophilin-Related Protein (Genbank No. HG846-HT846)

(32) Natural killer-tumor recognition sequence (Genbank No. L04288)

(33) Integrin alpha 6B (CD49f) (Genbank No. S66213)

(34) Homo sapiens clone 24629 mRNA sequence (Genbank No. AF052160)

(35) Protein kinase C Nu (EPK2) (Genbank No. AB015982)

(36) mRNA for SYT.SSX1 translocational target region of human synovial inducible sarcomas [Partial Mutant, 3' genes, 585nt] (Genbank No. S79325)

(37) Glucose transporter pseudogene (Genbank No. M55536)

(38) Nuclear receptor intermediary activation factor 2 (TIF2) (Genbank No. X97674)

(39) CRE-BP1 transcription factor (Genbank No. U16028)

(40) Topoisomerase type II (Topo II) (Genbank No. M27504)

(41) Nuclear respiratory factor-2 subunit alpha (Genbank No. U13044)

(42) PAC clone DJ1185I07 from 7q11.23-q21 RP5-1185I07 (Genbank No. AC004990)

(43) Cyclin T2b (Genbank No. AF048732)

(44) Zinc finger protein type C3H (MBLL) (Genbank No. AF061261)

(45) MEK kinase (Mekk) (Genbank No. U29671)

(46) Rod1 (Genbank No. AB023967)

(47) HSTXK Human tyrosine kinase (TXK) (Genbank No. U07794)

(48) Serine/threonine-protein kinase PRP4h (PRP4h) (Genbank No. U48736)

(49) pre-mRNA cleavage factor I subunit Im (Genbank No. AJ001810)

(50) Homo sapiens cDNA, 3'end/clone=IMAGE-2512364, EST wt65e11.×1 (Genbank No. AI961669)

(51) Disintegrin-metalloprotease (Genbank No. Z48579)

(52) ADP-ribosylation factor no. 6 (ARF6) (Genbank No. AF047432)

(53) Helicase like protein 2 containing DEAD/H box (DDX14) (Genbank No. U50553)

(54) p300/CBP-associated factor (P/CAF) (Genbank No. U57317)

(55) Ribosomal protein S6 kinase (ISPK-1) (Genbank No. U08316)

(56) Cyclin G1 (Genbank No. X77794)

(57) Guanine binding protein type q (Gaq) (Genbank No. U43083)

(58) Trinucleotide repeat CGG-DNA binding protein p20-CGGBP (CGGBP) (Genbank No. AF094481)

(59) Integrin alpha 4 subunit (CD49d) (Genbank No. L12002)

(60) Chromosome 5q21-22, clone-A3-A (Genbank No. AB002450)

(61) Unknown protein of uterine endometrium (Genbank No. X77723)

(62) Transcription factor ISGF-3 (STAT91) (Genbank No. M97935)

(63) Human PAC clone DJ525N14 from Xq23 RP3-525N14 (Genbank No. AC002086)

(64) SH2 domain protein 1A isoform B (SH2D1A) (Genbank No. AF100539)

(65) Killer cell lectin-like receptor NKG2F (Genbank No. AJ001683)

(66) Human homolog of Drosophila discs gene, isoform 2 (hdlg-2) (Genbank No. U13896)

(67) Human SNF1-like protein kinase (Genbank No. U57452)

(68) Human DNA for c-ets-1 proto-oncogene (Genbank No. X14798)

(69) EAR-1r (Genbank No. D16815)

(70) Guanine nucleotide regulatory protein (G alpha 13) (Genbank No. L22075)

(71) Retinoblastoma susceptibility protein (RB1) (Genbank No. L49229)

(72) SH-PTP3 for protein-tyrosine phosphatase (Genbank No. D13540)

(73) EST 14e9 Homo sapiens cDNA (Genbank No. W25874)

(74) MDM2-like p53-binding protein (MDMX) (Genbank No. AF007111)

(75) Homo sapiens cDNA, 5'end/clone=IMAGE-360208, EST ze27c09.r1 (Genbank No. AA013087)

(76) Erythroblastosis virus oncogene homolog 1 (ets-1) (Genbank No. J04101)

(77) HUMM9, Man9-mannosidase (Genbank No. X74837)

(78) Kinesin/heavy chain 5B (Genbank No. X65873)

(79) Interferon-inducible RNA-dependent protein kinase (Pkr) (Genbank No. U50648)

(80) Interferon regulatory factor-2 (IRF-2) (Genbank No. X15949)

(81) Homo sapiens cDNA, 3'end/clone=IMAGE-1722789, EST qd04h11.×1 (Genbank No. AI189226)

(82) Chondroitin sulfate proteoglycan PG-M (bursicon) (Genbank No. D32039)

(83) Homo sapiens mRNA; cDNA DKFZp564P0823 (from clone DKFZp564P0823) (Genbank No. AL049962)

(84) EST36b3 Homo sapiens cDNA (Genbank No. W27675)

(85) Homo sapiens cDNA, 3'end/clone=IMAGE-2489058, EST wr28g10.×1 (Genbank No. AW006742)

(86) Homo sapiens cDNA, 3'end/clone=IMAGE-815515, EST aa 38b10.s1 (Genbank No. AA457029)

(87) c-myc proto-oncogene (MYCL2) (Genbank No. J03069)

(88) Mature T cell proliferation factor c6.1B gene; MTCP1 gene (Genbank No. Z24459)

(89) Homo sapiens mRNA for KIAA0797 protein (Genbank No. AB018340)

(90) N-ras (Genbank No. X02751)

(91) WD repeat protein HAN11 (Genbank No. U94747)

(92) Homo sapiens mRNA for KIAA1048 protein (Genbank No. AB028971)

(93) Homo sapiens mRNA for KIAA0454 protein (Genbank No. AB007923)

(94) Cystine/glutamate transporter (Genbank No. AB026891)

(95) Microsomal stress 70 protein ATPase core (stch) (Genbank No. U04735)

(96) cAMP-dependent protein kinase catalytic subunit type alpha (EC 2.7.1.37) (Genbank No. X07767)

(97) Homo sapiens cDNA, 5'end/clone=IMAGE-2497327 (Genbank No AW003733)

(98) Homo sapiens cDNA, 5'end/clone=IMAGE-487691 (Genbank No. AA058762)

(99) Monoamine oxidase B (MAOB) (Genbank No. M69177)

(100) lipocortin-III (annexins A3) (Genbank No. M20560)

(101) Homo sapiens chromosome 1 specific transcript KIAA0508 (Genbank No. AB007977)

(102) Platelet-activating factor receptor (Genbank No. D10202)

(103)EST DKFZp586A2224_s1 Homo sapiens cDNA (Genbank No. AL048308)

(104) PCTAIRE-1 for serine/threonine protein kinase (Genbank No. X66363)

(105) Gelsolin; macrophage capping protein; villin (Genbank No. M94345)

(106) EST 31c9 Homo sapiens cDNA (Genbank No. W27466)

(107) Diaphanous type 2 isoform 12C protein, dia-156 protein (DIA-156) (Genbank No. Y15909)

(108) Insulin receptor precursor (Genbank No. X02160)

(109) Heregulin type 1 (HRG alpha) (Genbank No. L41827)

(110) Branched chain alpha-ketoacid dehydrogenase kinase precursor (BCKD kinase) (Genbank No. AF026548)

(111) Electron transfer flavoprotein beta subunit (Genbank No. X71129)

(112) p160 (Genbank No. U88153)

(113) Calciceurin dependently activated T cell nuclear factor (NF-Atc) (Genbank No. U08015)

(114) Homo sapiens mRNA for KIAA0563 protein (Genbank No. AB011135)

(115) Vscular smooth muscle alpha-actin (Genbank No. X13839)

(116) Rad17-like protein (RAD17) (Genbank No. AF076838)

(117) Homo sapiens cDNA, 3'end/clone=IMAGE-2394055, EST wi54d04.×1 (Genbank No. AI762213)

(118) Homo sapiens cDNA, 3 end/clone=IMAGE-979142, EST ni38e08.s1 (Genbank No. AA522537)

(119) Human T54 protein (T54) (Genbank No. U66359)

(120) Acyl-CoA dehydrogenase; SCAD gene (Genbank No. Z80345)

(121) Phosphomevalonate kinase (Genbank No. L77213)

(122) Drebrin E (Genbank No. D17530)

(123) Receptor protein-tyrosine kinase EphA4 (HEK8) (Genbank No. L36645)

(124) Tob family transducer ERBB2,2 (Genbank No. D64109)

(125) Homo sapiens cDNA, 3 end/clone=IMAGE-1657913, ESTox31b09.s1 (Genbank No. AI039144)

(126) Homogentisate 1,2-dioxygenase (Genbank No. AF000573)

(127) MFH-proliferation sequence (MASL1) (Genbank No. AB016816)

(128) Homo sapiens mRNA for KIAA0994 protein (Genbank No. AB023211)

(129) Homo sapiens cDNA, 3 end /clone=IMAGE-826408, EST aa71e09.s1 (Genbank No. AA521060)

(130) Neutrophil cytoplasmic factor type 4 (p40phox) (Genbank No. X77094)

(131) Mucin 5b (Genbank No. HG2689-HT2785)

(132) Homo sapiens cDNA, 3 end/clone=IMAGE-965972, EST nh92c11.s1 (Genbank No. AA528252)

(133) Cell adhesion protein (vitronectin) receptor alpha subunit (CD51) (Genbank No. M14648)

(134) Cluster Incl AL049435:Homo sapiens mRNA; cDNA DKFZp586B0220 (from clone DKFZp586B0220 (Genbank No. AL049435)

(135) Homo sapiens (clone S164) mRNA, 3 end of cds /cds (Genbank No. L40392)

(136) mRNA for KIAA1009 protein (Genbank No. AB023226)

(137) mRNA for KIAA0716 protein (Genbank No. AB018259)

(138) Vanin-like gene; vnn1 gene; VNN1 protein (Genbank No. AJ132099)

(139) Homo sapiens PAC clone DJ0808A01 from 7q21.1-q31.1 (Genbank No. AC004893)

(140) Homo sapiens mRNA for KIAA1050 protein (Genbank No. AB028973)

(141) Human Chromosome 16 BAC clone CIT987SK-A-270G1 (Genbank No. AF001549)

(142) Transcriptional factor TREB protein (Genbank No. X55544)

(143) Homo sapiens mRNA for KIAA0548 protein (Genbank No. AB011120)

(144) p300; transcriptional adaptor protein; E1A-binding protein (Genbank No. U01877)

(145) Integrin alpha E precursor (CD103) (L25851)

(146) Homo sapiens cDNA, 3 end/clone=IMAGE-1714897 EST qc69h01.x1 (Genbank No. AI148772)

(147) Homo sapiens mRNA full length insert cDNA clone EUROIMAGE 417629 (Genbank No. AL109724)

(148) Defensins alpha 3 (Genbank No. L12691)

(149) Homo sapiens cDNA, 5' end/clone=DKFZp564J2262-r1 (Genbank No. AL036554)

(150) Elastase/medullasin (Genbank No. M34379)

(151) Angelman Syndrome Gene, E6-AP ubiquitin protein ligase 3A (UBE3A) (Genbank No. AF002224)

(152) Skeletal muscle 165kD protein (Genbank No. X69089)

**15.** A method for diagnosing whether a test subject suffers from schizophrenia or not, the method comprising the steps of;

obtaining mononuclear cells in blood containing ribonucleic acid from said subject and extracting said ribonucleic acid from the blood,

measuring the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and

comparing multiplicative values of the probabilities obtained from the statistical distributions or deviations of the quantified value(s) of said nucleic acid(s) defining gene(s) exhibiting altered expression by occurrence of schizophrenia or said nucleic acid(s) defining gene(s) exhibiting altered expression by progression of schizophrenia in said test subject with the multiplicative values of the probabilities in healthy subjects or in schizophrenic patients to determine whether the alteration of the content of the gene in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) defined by the gene name, the protein name which is a gene product, or the nucleic acid sequence name as described below in (1) to (152) with GenBank No. described in brackets.

(1) Homo sapiens cDNA, 3'end/clone= IMAGE-2329930, EST wd33c06.×1 (Genbank No. AI677689)

(2) Bcl-Xl (Genbank No. Z23115)

(3) ZNF37A mRNA for zinc finger (Genbank No. X69115)

(4) HSCCG1 Human X chromsome mRNA for CCG1 protein inv. in cell proliferation (Genbank No. X07024)

(5) Interferon receptor type 2 (IFNAR2) (Genbank No. L42243)

(6) Guanine Nucleotide Exchange Factor 1 (Genbank No. HG960-HT960)

(7) Glucosamine-6-sulphatase precursor (Genbank No. Z12173)

(8) MACH-beta-1 protein (Caspase 8)(Genbank No. X98176)

(9) EST 15a11 Homo sapiens cDNA/gb=W25921/gi=1306044/ug= Hs.164036/len=723 (Genbank No. W25921)

(10) Ndr protein kinase (Genbank No. Z35102)

(11) 14-3-3 protein (Genbank No. U28964)

(12) RbAp48 mRNA encoding retinoblastoma binding protein (Genbank No. X74262)

(13) SNAP23B protein (Genbank No. Y09568)

(14) Inhibitory protein for potassium-induced deficiency type 1 (SKD1 homolog) (Genbank No. AF038960)

(15) Homo sapiens cDNA, 3'end/clone=IMAGE-2509049, ETS wt31b09.×1 (Genbank No. AI955897)

(16) Utrophin (Genbank No. X69086)

(17) cdc2-related protein kinase (Genbank No. M80629)

(18) Calmodulin type 1 (CALM1) (Genbank No. U12022)

(19) Rb2/p130 protein (Genbank No. X74594)

(20) Protein-tyrosine kinase JAK1 (Genbank No. M64174)

(21) GTP-binding protein RAB6 (Genbank No. M28212)

(22) Clq/MBL/SPA receptor C1qR(p) (Genbank No. U94333)

(23) Zinc finger/leucine zipper protein (AF10)(Genbank No. U13948)

(24) Inositol polyphosphate 4-phosphatase type I-beta (Genbank No. U96919)

(25) Inositol polyphosphate 4-phosphatase (Genbank No. U26398)

(26) Cytohesin binding protein HE (Genbank No. AF068836)

(27) Cas like protein for enhancer of filamentation (HEF1) (Genbank No. L43821)

(28) Rho GTPase-activated protein type 5 (p190-B) (Genbank No. U17032)

(29) AMP-activated protein kinase alpha-1 (Genbank No. AB022017)

(30) Syntaxin 16 (Genbank No. AF038897)

(31) Cyclophilin-Related Protein (Genbank No. HG846-HT846)

(32) Natural killer-tumor recognition sequence (Genbank No. L04288)

(33) Integrin alpha 6B (CD49f) (Genbank No. S66213)

(34) Homo sapiens clone 24629 mRNA sequence (Genbank No. AF052160)

(35) Protein kinase C Nu (EPK2) (Genbank No. AB015982)

(36) mRNA for SYT.SSX1 translocational target region of human synovial inducible sarcomas [Partial Mutant, 3' genes, 585nt] (Genbank No. S79325)

(37) Glucose transporter pseudogene (Genbank No. M55536)

(38) Nuclear receptor intermediary activation factor 2 (TIF2) (Genbank No. X97674)

(39) CRE-BP1 transcription factor (Genbank No. U16028)

(40) Topoisomerase type II (Topo II) (Genbank No. M27504)

(41) Nuclear respiratory factor-2 subunit alpha (Genbank No. U13044)

(42) PAC clone DJ1185I07 from 7q11.23-q21 RP5-1185I07 (Genbank No. AC004990)

(43) Cyclin T2b (Genbank No. AF048732)

(44) Zinc finger protein type C3H (MBLL) (Genbank No. AF061261)

(45) MEK kinase (Mekk) (Genbank No. U29671)

(46) Rod1 (Genbank No. AB023967)

(47) HSTXK Human tyrosine kinase (TXK) (Genbank No. U07794)

(48) Serine/threonine-protein kinase PRP4h (PRP4h) (Genbank No. U48736)

(49) pre-mRNA cleavage factor I subunit Im (Genbank No. AJ001810)

(50) Homo sapiens cDNA, 3'end/clone=IMAGE-2512364, EST wt65e11.$\times$1 (Genbank No. AI961669)

(51) Disintegrin-metalloprotease (Genbank No. Z48579)

(52) ADP-ribosylation factor no.6 (ARF6) (Genbank No. AF047432)

(53) Helicase like protein 2 containing DEAD/H box (DDX14) (Genbank No. U50553)

(54) p300/CBP-associated factor (P/CAF) (Genbank No. U57317)

(55) Ribosomal protein S6 kinase (ISPK-1) (Genbank No. U08316)

(56) Cyclin G1 (Genbank No. X77794)

(57) Guanine binding protein type q (Gaq) (Genbank No. U43083)

(58) Trinucleotide repeat CGG-DNA binding protein p20-CGGBP (CGGBP) (Genbank No. AF094481)

(59) Integrin alpha 4 subunit (CD49d) (Genbank No. L12002)

(60) Chromosome 5q21-22, clone-A3-A (Genbank No. AB002450)

(61) Unknown protein of uterine endometrium (Genbank No. X77723)

(62) Transcription factor ISGF-3 (STAT91) (Genbank No. M97935)

(63) Human PAC clone DJ525N14 from Xq23 RP3-525N14 (Genbank No. AC002086)

(64) SH2 domain protein 1A isoform B (SH2D1A) (Genbank No. AF100539)

(65) Killer cell lectin-like receptor NKG2F (Genbank No. AJ001683)

(66) Human homolog of Drosophila discs gene, isoform 2 (hdlg-2) (Genbank No. U13896)

(67) Human SNF1-like protein kinase (Genbank No. U57452)

(68) Human DNA for c-ets-1 proto-oncogene (Genbank No. X14798)

(69) EAR-1r (Genbank No. D16815)

(70) Guanine nucleotide regulatory protein (G alpha 13) (Genbank No. L22075)

(71) Retinoblastoma susceptibility protein (RB1) (Genbank No. L49229)

(72) SH-PTP3 for protein-tyrosine phosphatase (Genbank No. D13540)

(73) EST 14e9 Homo sapiens cDNA (Genbank No. W25874)

(74) MDM2-like p53-binding protein (MDMX) (Genbank No. AF007111)

(75) Homo sapiens cDNA, 5'end/clone=IMAGE-360208, EST ze27c09.r1 (Genbank No. AA013087)

(76) Erythroblastosis virus oncogene homolog 1 (ets-1) (Genbank No. J04101)

(77) HUMM9, Man9-mannosidase (Genbank No. X74837)

(78) Kinesin/heavy chain 5B (Genbank No. X65873)

(79) Interferon-inducible RNA-dependent protein kinase (Pkr) (Genbank No. U50648)

(80) Interferon regulatory factor-2 (IRF-2) (Genbank No. X15949)

(81) Homo sapiens cDNA, 3'end/clone=IMAGE-1722789, EST qd04h11.$\times$1 (Genbank No. AI189226)

(82) Chondroitin sulfate proteoglycan PG-M (bursicon) (Genbank No. D32039)

(83) Homo sapiens mRNA; cDNA DKFZp564P0823 (from clone DKFZp564P0823) (Genbank No. AL049962)

(84) EST36b3 Homo sapiens cDNA (Genbank No. W27675)

(85) Homo sapiens cDNA, 3'end/clone=IMAGE=2489058, EST wr28g10.$\times$1 (Genbank No. AW006742)

(86) Homo sapiens cDNA, 3'end/clone=IMAGE-815515, EST aa 38b10.s1 (Genbank No. AA457029)

(87) c-myc proto-oncogene (MYCL2) (Genbank No. J03069)

(88) Mature T cell proliferation factor c6.1B gene; MTCP1 gene (Genbank No. Z24459)

(89) Homo sapiens mRNA for KIAA0797 protein (Genbank No. AB018340)

(90) N-ras (Genbank No. X02751)

(91) WD repeat protein HAN11 (Genbank No. U94747)

(92) Homo sapiens mRNA for KIAA1048 protein (Genbank No. AB028971)

(93) Homo sapiens mRNA for KIAA0454 protein (Genbank No. AB007923)

(94) Cystine/glutamate transporter (Genbank No. AB026891)

(95) Microsomal stress 70 protein ATPase core (stch) (Genbank No. U04735)

(96) cAMP-dependent protein kinase catalytic subunit type alpha (EC 2.7.1.37) (Genbank No. X07767)

(97) Homo sapiens cDNA, 5'end/clone=IMAGE-2497327 (Genbank No AW003733)

(98) Homo sapiens cDNA, 5'end/clone=IMAGE-487691 (Genbank No. AA058762)

(99) Monoamine oxidase B (MAOB) (Genbank No. M69177)

(100) lipocortin-III (annexins A3) (Genbank No. M20560)

(101) Homo sapiens chromosome 1 specific transcript KIAA0508 (Genbank No. AB007977)

(102) Platelet-activating factor receptor (Genbank No. D10202)

(103) EST DKFZp586A2224_s1 Homo sapiens cDNA (Genbank No. AL048308)

(104) PCTAIRE-1 for serine/threonine protein kinase (Genbank No. X66363)

(105) Gelsolin; macrophage capping protein; villin (Genbank No. M94345)

(106) EST 31c9 Homo sapiens cDNA (Genbank No. W27466)

(107) Diaphanous type 2 isoform 12C protein, dia-156 protein (DIA-156) (Genbank No. Y15909)

(108) Insulin receptor precursor (Genbank No. X02160)

(109) Heregulin type 1 (HRG alpha) (Genbank No. L41827)

(110) Branched chain alpha-ketoacid dehydrogenase kinase precursor (BCKD kinase) (Genbank No. AF026548)

(111) Electron transfer flavoprotein beta subunit (Genbank No. X71129)

(112) p160 (Genbank No. U88153)

(113) Calciceurin dependently activated T cell nuclear factor (NF-Atc) (Genbank No. U08015)

(114) Homo sapiens mRNA for KIAA0563 protein (Genbank No. AB011135)

(115) Vscular smooth muscle alpha-actin (Genbank No. X13839)

(116) Rad17-like protein (RAD17) (Genbank No. AF076838)

(117) Homo sapiens cDNA, 3'end/clone=IMAGE-2394055, EST wi54d04.×1 (Genbank No. AI762213)

(118) Homo sapiens cDNA, 3 end/clone=IMAGE-979142, EST ni38e08.s1 (Genbank No. AA522537)

(119) Human T54 protein (T54) (Genbank No. U66359)

(120) Acyl-CoA dehydrogenase; SCAD gene (Genbank No. Z80345)

(121) Phosphomevalonate kinase (Genbank No. L77213)

(122) Drebrin E (Genbank No. D17530)

(123) Receptor protein-tyrosine kinase EphA4 (HEK8) (Genbank No. L36645)

(124) Tob family transducer ERBB2,2 (Genbank No. D64109)

(125) Homo sapiens cDNA, 3 end/clone=IMAGE-1657913, ESTox31b09.s1 (Genbank No. AI039144)

(126) Homogentisate 1,2-dioxygenase (Genbank No. AF000573)

(127) MFH-proliferation sequence (MASL1) (Genbank No. AB016816)

(128) Homo sapiens mRNA for KIAA0994 protein (Genbank No. AB023211)

(129) Homo sapiens cDNA, 3 end/clone=IMAGE-826408, EST aa71e09.s1 (Genbank No. AA521060)

(130) Neutrophil cytoplasmic factor type 4 (p40phox) (Genbank No. X77094)

(131) Mucin 5b (Genbank No. HG2689-HT2785)

(132) Homo sapiens cDNA, 3 end/clone=IMAGE-965972, EST nh92c11.s1 (Genbank No. AA528252)

(133) Cell adhesion protein (vitronectin) receptor alpha subunit (CD51) (Genbank No. M14648)

(134) Cluster Incl AL049435:Homo sapiens mRNA; cDNA DKFZp586B0220 (from clone DKFZp586B0220 (Genbank No. AL049435)

(135) Homo sapiens (clone S164) mRNA, 3 end of cds /cds (Genbank No. L40392)

(136) mRNA for KIAA1009 protein (Genbank No. AB023226)

(137) mRNA for KIAA0716 protein (Genbank No. AB018259)

(138) Vanin-like gene; vnn1 gene; VNN1 protein (Genbank No. AJ132099)

(139) Homo sapiens PAC clone DJ0808A01 from 7q21.1-q31.1 (Genbank No. AC004893)

(140) Homo sapiens mRNA for KIAA1050 protein (Genbank No. AB028973)

(141) Human Chromosome 16 BAC clone CIT987SK-A-270G1 (Genbank No. AF001549)

(142) Transcriptional factor TREB protein (Genbank No. X55544)

(143) Homo sapiens mRNA for KIAA0548 protein (Genbank No. AB011120)

(144) p300; transcriptional adaptor protein; E1A-binding protein (Genbank No. U01877)

(145) Integrin alpha E precursor (CD103) (L25851)

(146) Homo sapiens cDNA, 3 end/clone=IMAGE-1714897 EST qc69h01.x1 (Genbank No. AI148772)

(147) Homo sapiens mRNA full length insert cDNA clone EUROIMAGE 417629 (Genbank No. AL109724)

(148) Defensins alpha 3 (Genbank No. L12691)

(149) Homo sapiens cDNA, 5' end/clone=DKFZp564J2262-r1 (Genbank No. AL036554)

(150) Elastase/medullasin (Genbank No. M34379)

(151) Angelman Syndrome Gene, E6-AP ubiquitin protein ligase 3A (UBE3A) (Genbank No. AF002224)

(152) Skeletal muscle 165kD protein (Genbank No. X69089)

**16.** A method for diagnosing whether a test subject suffers from schizophrenia or not, the method comprising the steps of;

obtaining mononuclear cells in blood containing ribonucleic acid from said subject and extracting said ribonucleic acid from the blood,

determining the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and

comparing the discriminant value(s) obtained by linear weighted addition of the quantified value(s) of said nucleic acid(s) defining gene(s) exhibiting altered expression by occurrence of schizophrenia or said nucleic acid (s) defining gene(s) exhibiting altered expression by progression of schizophrenia with the discriminant value(s) in healthy subjects or schizophrenic patients obtained by linear weighted addition to determine whether the alteration of the discriminant value(s) of the gene in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) defined by the gene name, the protein name which is a gene product, or the nucleic acid sequence name as described below in (1) to (152) with GenBank No. described in brackets.

(1) Homo sapiens cDNA, 3'end/clone= IMAGE-2329930, EST wd33c06.×1 (Genbank No. AI677689)

(2) Bcl-XI (Genbank No. Z23115)

(3) ZNF37A mRNA for zinc finger (Genbank No. X69115)

(4) HSCCG1 Human X chromsome mRNA for CCG1 protein inv. in cell proliferation (Genbank No. X07024)

(5) Interferon receptor type 2 (IFNAR2) (Genbank No. L42243)

(6) Guanine Nucleotide Exchange Factor 1 (Genbank No. HG960-HT960)

(7) Glucosamine-6-sulphatase precursor (Genbank No. Z12173)

(8) MACH-beta-1 protein (Caspase 8)(Genbank No. X98176)

(9) EST 15a11 Homo sapiens cDNA/gb=W25921/gi=1306044/ug= Hs.164036/len=723 (Genbank No. W25921)

(10) Ndr protein kinase (Genbank No. Z35102)

(11) 14-3-3 protein (Genbank No. U28964)

(12) RbAp48 mRNA encoding retinoblastoma binding protein (Genbank No. X74262)

(13) SNAP23B protein (Genbank No. Y09568)

(14) Inhibitory protein for potassium-induced deficiency type 1 (SKD1 homolog) (Genbank No. AF038960)

(15) Homo sapiens cDNA, 3'end/clone=IMAGE-2509049, ETS wt31b09.×1 (Genbank No. AI955897)

(16) Utrophin (Genbank No. X69086)

(17) cdc2-related protein kinase (Genbank No. M80629)

(18) Calmodulin type 1 (CALM1) (Genbank No. U12022)

(19) Rb2/p130 protein (Genbank No. X74594)

(20) Protein-tyrosine kinase JAK1 (Genbank No. M64174)

(21) GTP-binding protein RAB6 (Genbank No. M28212)

(22) Clq/MBL/SPA receptor C1qR(p) (Genbank No. U94333)

(23) Zinc finger/leucine zipper protein (AF10)(Genbank No. U13948)

(24) Inositol polyphosphate 4-phosphatase type I-beta (Genbank No. U96919)

(25) Inositol polyphosphate 4-phosphatase (Genbank No. U26398)

(26) Cytohesin binding protein HE (Genbank No. AF068836)

(27) Cas like protein for enhancer of filamentation (HEF1) (Genbank No. L43821)

(28) Rho GTPase-activated protein type 5 (p190-B) (Genbank No. U17032)

(29) AMP-activated protein kinase alpha-1 (Genbank No. AB022017)

(30) Syntaxin 16 (Genbank No. AF038897)

(31) Cyclophilin-Related Protein (Genbank No. HG846-HT846)

(32) Natural killer-tumor recognition sequence (Genbank No. L04288)

(33) Integrin alpha 6B (CD49f) (Genbank No. S66213)

(34) Homo sapiens clone 24629 mRNA sequence (Genbank No. AF052160)

(35) Protein kinase C Nu (EPK2) (Genbank No. AB015982)

(36) mRNA for SYT.SSX1 translocational target region of human synovial inducible sarcomas [Partial Mutant, 3' genes, 585nt] (Genbank No. S79325)

(37) Glucose transporter pseudogene (Genbank No. M55536)

(38) Nuclear receptor intermediary activation factor 2 (TIF2) (Genbank No. X97674)

(39) CRE-BP1 transcription factor (Genbank No. U16028)

(40) Topoisomerase type II (Topo II) (Genbank No. M27504)

(41) Nuclear respiratory factor-2 subunit alpha (Genbank No. U13044)

(42) PAC clone DJ1185I07 from 7q11.23-q21 RP5-1185I07 (Genbank No. AC004990)

(43) Cyclin T2b (Genbank No. AF048732)

(44) Zinc finger protein type C3H (MBLL) (Genbank No. AF061261)

(45) MEK kinase (Mekk) (Genbank No. U29671)

(46) Rod1 (Genbank No. AB023967)

(47) HSTXK Human tyrosine kinase (TXK) (Genbank No. U07794)

(48) Serine/threonine-protein kinase PRP4h (PRP4h) (Genbank No. U48736)

(49) pre-mRNA cleavage factor I subunit Im (Genbank No. AJ001810)

(50) Homo sapiens cDNA, 3'end/clone=IMAGE-2512364, EST wt65e11.$\times$1 (Genbank No. AI961669)

(51) Disintegrin-metalloprotease (Genbank No. Z48579)

(52) ADP-ribosylation factor no.6 (ARF6) (Genbank No. AF047432)

(53) Helicase like protein 2 containing DEAD/H box (DDX14) (Genbank No. U50553)

(54) p300/CBP-associated factor (P/CAF) (Genbank No. U57317)

(55) Ribosomal protein S6 kinase (ISPK-1) (Genbank No. U08316)

(56) Cyclin G1 (Genbank No. X77794)

(57) Guanine binding protein type q (Gaq) (Genbank No. U43083)

(58) Trinucleotide repeat CGG-DNA binding protein p20-CGGBP (CGGBP) (Genbank No. AF094481)

(59) Integrin alpha 4 subunit (CD49d) (Genbank No. L12002)

(60) Chromosome 5q21-22, clone-A3-A (Genbank No. AB002450)

(61) Unknown protein of uterine endometrium (Genbank No. X77723)

(62) Transcription factor ISGF-3 (STAT91) (Genbank No. M97935)

(63) Human PAC clone DJ525N14 from Xq23 RP3-525N14 (Genbank No. AC002086)

(64) SH2 domain protein 1A isoform B (SH2D1A) (Genbank No. AF100539)

(65) Killer cell lectin-like receptor NKG2F (Genbank No. AJ001683)

(66) Human homolog of Drosophila discs gene, isoform 2 (hdlg-2) (Genbank No. U13896)

(67) Human SNF1-like protein kinase (Genbank No. U57452)

(68) Human DNA for c-ets-1 proto-oncogene (Genbank No. X14798)

(69) EAR-1r (Genbank No. D16815)

(70) Guanine nucleotide regulatory protein (G alpha 13) (Genbank No. L22075)

(71) Retinoblastoma susceptibility protein (RB1) (Genbank No. L49229)

(72) SH-PTP3 for protein-tyrosine phosphatase (Genbank No. D13540)

(73) EST 14e9 Homo sapiens cDNA (Genbank No. W25874)

(74) MDM2-like p53-binding protein (MDMX) (Genbank No. AF007111)

(75) Homo sapiens cDNA, 5'end/clone=IMAGE-360208, EST ze27c09.r1 (Genbank No. AA013087)

(76) Erythroblastosis virus oncogene homolog 1 (ets-1) (Genbank No. J04101)

(77) HUMM9, Man9-mannosidase (Genbank No. X74837)

(78) Kinesin/heavy chain 5B (Genbank No. X65873)

(79) Interferon-inducible RNA-dependent protein kinase (Pkr) (Genbank No. U50648)

(80) Interferon regulatory factor-2 (IRF-2) (Genbank No. X15949)

(81) Homo sapiens cDNA, 3'end/clone=IMAGE-1722789, EST qd04h11.$\times$1 (Genbank No. AI189226)

(82) Chondroitin sulfate proteoglycan PG-M (bursicon) (Genbank No. D32039)

(83) Homo sapiens mRNA; cDNA DKFZp564P0823 (from clone DKFZp564P0823) (Genbank No. AL049962)

(84) EST36b3 Homo sapiens cDNA (Genbank No. W27675)

(85) Homo sapiens cDNA, 3'end/clone=IMAGE-2489058, EST wr28g10.×1 (Genbank No. AW006742)

(86) Homo sapiens cDNA, 3'end/clone=IMAGE-815515, EST aa 38b10.s1 (Genbank No. AA457029)

(87) c-myc proto-oncogene (MYCL2) (Genbank No. J03069)

(88) Mature T cell proliferation factor c6.1B gene; MTCP1 gene (Genbank No. Z24459)

(89) Homo sapiens mRNA for KIAA0797 protein (Genbank No. AB018340)

(90) N-ras (Genbank No. X02751)

(91) WD repeat protein HAN11 (Genbank No. U94747)

(92) Homo sapiens mRNA for KIAA1048 protein (Genbank No. AB028971)

(93) Homo sapiens mRNA for KIAA0454 protein (Genbank No. AB007923)

(94) Cystine/glutamate transporter (Genbank No. AB026891)

(95) Microsomal stress 70 protein ATPase core (stch) (Genbank No. U04735)

(96) cAMP-dependent protein kinase catalytic subunit type alpha (EC 2.7.1.37) (Genbank No. X07767)

(97) Homo sapiens cDNA, 5'end/clone=IMAGE-2497327 (Genbank No AW003733)

(98) Homo sapiens cDNA, 5'end/clone=IMAGE-487691 (Genbank No. AA058762)

(99) Monoamine oxidase B (MAOB) (Genbank No. M69177)

(100) lipocortin-III (annexins A3) (Genbank No. M20560)

(101)Homo sapiens chromosome 1 specific transcript KIAA0508 (Genbank No. AB007977)

(102) Platelet-activating factor receptor (Genbank No. D10202)

(103) EST DKFZp586A2224_s1 Homo sapiens cDNA (Genbank No. AL048308)

(104) PCTAIRE-1 for serine/threonine protein kinase (Genbank No. X66363)

(105) Gelsolin; macrophage capping protein; villin (Genbank No. M94345)

(106) EST 31c9 Homo sapiens cDNA (Genbank No. W27466)

(107) Diaphanous type 2 isoform 12C protein, dia-156 protein (DIA-156) (Genbank No. Y15909)

(108) Insulin receptor precursor (Genbank No. X02160)

(109) Heregulin type 1 (HRG alpha) (Genbank No. L41827)

(110) Branched chain alpha-ketoacid dehydrogenase kinase precursor (BCKD kinase) (Genbank No. AF026548)

(111) Electron transfer flavoprotein beta subunit (Genbank No. X71129)

(112) p160 (Genbank No. U88153)

(113) Calciceurin dependently activated T cell nuclear factor (NF-Atc) (Genbank No. U08015)

(114) Homo sapiens mRNA for KIAA0563 protein (Genbank No. AB011135)

(115) Vscular smooth muscle alpha-actin (Genbank No. X13839)

(116) Rad17-like protein (RAD17) (Genbank No. AF076838)

(117) Homo sapiens cDNA, 3'end/clone=IMAGE-2394055, EST wi54d04.×1 (Genbank No. AI762213)

(118) Homo sapiens cDNA, 3 end/clone=IMAGE-979142, EST ni38e08.s1 (Genbank No. AA522537)

(119) Human T54 protein (T54) (Genbank No. U66359)

(120) Acyl-CoA dehydrogenase; SCAD gene (Genbank No. Z80345)

(121) Phosphomevalonate kinase (Genbank No. L77213)

(122) Drebrin E (Genbank No. D17530)

(123) Receptor protein-tyrosine kinase EphA4 (HEK8) (Genbank No. L36645)

(124) Tob family transducer ERBB2,2 (Genbank No. D64109)

(125) Homo sapiens cDNA, 3 end/clone=IMAGE-1657913, ESTox31b09.s1 (Genbank No. AI039144)

(126) Homogentisate 1,2-dioxygenase (Genbank No. AF000573)

(127) MFH-proliferation sequence (MASL1) (Genbank No. AB016816)

(128) Homo sapiens mRNA for KIAA0994 protein (Genbank No. AB023211)

(129) Homo sapiens cDNA, 3 end/clone=IMAGE-826408, EST aa71e09.s1 (Genbank No. AA521060)

(130) Neutrophil cytoplasmic factor type 4 (p40phox) (Genbank No. X77094)

(131) Mucin 5b (Genbank No. HG2689-HT2785)

(132) Homo sapiens cDNA, 3 end/clone=IMAGE-965972, EST nh92c11.s1 (Genbank No. AA528252)

(133) Cell adhesion protein (vitronectin) receptor alpha subunit (CD51) (Genbank No. M14648)

(134) Cluster Incl AL049435:Homo sapiens mRNA; cDNA DKFZp586B0220 (from clone DKFZp586B0220 (Genbank No. AL049435)

(135) Homo sapiens (clone S164) mRNA, 3 end of cds/cds (Genbank No. L40392)

(136) mRNA for KIAA1009 protein (Genbank No. AB023226)

(137) mRNA for KIAA0716 protein (Genbank No. AB018259)

(138) Vanin-like gene; vnn1 gene; VNN1 protein (Genbank No. AJ132099)

(139) Homo sapiens PAC clone DJ0808A01 from 7q21.1-q31.1 (Genbank No. AC004893)

(140) Homo sapiens mRNA for KIAA1050 protein (Genbank No. AB028973)

(141) Human Chromosome 16 BAC clone CIT987SK-A-270G1 (Genbank No. AF001549)

(142) Transcriptional factor TREB protein (Genbank No. X55544)

(143) Homo sapiens mRNA for KIAA0548 protein (Genbank No. AB011120)

(144) p300; transcriptional adaptor protein; E1A-binding protein (Genbank No. U01877)

(145) Integrin alpha E precursor (CD103) (L25851)

(146) Homo sapiens cDNA, 3 end/clone=IMAGE-1714897 EST qc69h01.x1 (Genbank No. AI148772)

(147) Homo sapiens mRNA full length insert cDNA clone EUROIMAGE 417629 (Genbank No. AL109724)

(148) Defensins alpha 3 (Genbank No. L12691)

(149) Homo sapiens cDNA, 5' end/clone=DKFZp564J2262-r1 (Genbank No. AL036554)

(150) Elastase/medullasin (Genbank No. M34379)

(151) Angelman Syndrome Gene, E6-AP ubiquitin protein ligase 3A (UBE3A) (Genbank No. AF002224)

(152) Skeletal muscle 165kD protein (Genbank No. X69089)

**17.** A method for diagnosing whether a test subject suffers from schizophrenia or not, the method comprising the steps of;

obtaining mononuclear cells in blood containing ribonucleic acid from said subject and extracting said ribonucleic acid from the blood,

determining the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells, and

comparing the discriminant value(s) obtained by linear weighted addition of the quantified value(s) of said nucleic acid(s) defining gene(s) exhibiting altered expression by occurrence of schizophrenia or said nucleic acid (s) defining gene(s) exhibiting altered expression by progression of schizophrenia with the discriminant value(s) in healthy subjects or schizophrenic patients obtained by linear weighted addition to determine whether the alteration of the discriminant value(s) of the gene in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) encoding enzyme(s) catalyzing transfer of phosphate group(s) (kinase or phosphatase) and defined by the gene name, the protein name which is a gene product, or the nucleic acid sequence name as described below in (1) to (16) with GenBank No described in brackets.

(1) Ndr protein kinase (Genbank No. Z35102)

(2) Protein-tyrosine kinase JAK1 (Genbank No. M64174)

(3) Inositol polyphosphate 4-phosphatase type I-beta (Genbank No. U96919)

(4) AMP-activated protein kinase alpha-1 (Genbank No. AB022017)

(5) Protein kinase C Nu (EPK2) (Genbank No. AB015982)

(6) MEK kinase (Mekk) (Genbank No. U29671)

(7) HSTXK Human tyrosine kinase (TXK) (Genbank No. U07794)

(8) Serine/threonine-protein kinase PRP4h (PRP4h) (Genbank No. U48736)

(9) Ribosomal protein S6 kinase (ISPK-1) (Genbank No. U08316)

(10) Human SNF1-like protein kinase (Genbank No. U57452)

(11) SH-PTP3 for protein-tyrosine phosphatase (Genbank No. D13540)

(12) Interferon-inducible RNA-dependent protein kinase (Pkr) (Genbank No. U50648)

(13) cAMP-dependent protein kinase catalytic subunit type alpha (EC 2.7.1.37) (Genbank No. X07767)

(14) PCTAIRE-1 for serine/threonine protein kinase (Genbank No. X66363)

(15) Branched chain alpha-ketoacid dehydrogenase kinase precursor (BCKD kinase) (Genbank No. AF026548)

(16) Phosphomevalonate kinase (Genbank No. L77213)

**18.** A method to discriminate a subject suffering from schizophrenia from a subject suffering from other psychiatric diseases, the method comprising the steps of;

obtaining mononuclear cells in blood containing ribonucleic acid from said subject and extracting said ribonucleic acid from the blood,

determining the content of at least one nucleic acid selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia in said mononuclear cells,

comparing the discriminant value(s) obtained by linear weighted addition of the quantified value(s) of said nucleic acid(s) defining gene(s) exhibiting altered expression by occurrence of schizophrenia or said nucleic acid(s) defining gene(s) exhibiting altered expression by progression of schizophrenia with the discriminant value(s) in healthy subjects or schizophrenic patients obtained by linear weighted addition to determine whether the alteration of the discriminant value(s) of the gene in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) encoding enzyme(s) catalyzing transfer of phosphate group(s) (kinase or phosphatase) and defined by the gene name, the protein name which is a gene product, or the nucleic acid sequence name as described below in (1) to (16) with GenBank No described in brackets.

(1) Ndr protein kinase (Genbank No. Z35102)
(2) Protein-tyrosine kinase JAK1 (Genbank No. M64174)
(3) Inositol polyphosphate 4-phosphatase type I-beta (Genbank No. U96919)
(4) AMP-activated protein kinase alpha-1 (Genbank No. AB022017)
(5) Protein kinase C Nu (EPK2) (Genbank No. AB015982)
(6) MEK kinase (Mekk) (Genbank No. U29671)
(7) HSTXK Human tyrosine kinase (TXK) (Genbank No. U07794)
(8) Serine/threonine-protein kinase PRP4h (PRP4h) (Genbank No. U48736)
(9) Ribosomal protein S6 kinase (ISPK-1) (Genbank No. U08316)
(10) Human SNF1-like protein kinase (Genbank No. U57452)
(11) SH-PTP3 for protein-tyrosine phosphatase (Genbank No. D13540)
(12) Interferon-inducible RNA-dependent protein kinase (Pkr) (Genbank No. U50648)
(13) cAMP-dependent protein kinase catalytic subunit type alpha (EC 2.7.1.37) (Genbank No. X07767)
(14) PCTAIRE-1 for serine/threonine protein kinase (Genbank No. X66363)
(15) Branched chain alpha-ketoacid dehydrogenase kinase precursor (BCKD kinase) (Genbank No. AF026548)
(16) Phosphomevalonate kinase (Genbank No. L77213)

19. A method for diagnosing whether a test subject suffers from schizophrenia or not, the method comprising the steps of;

obtaining mononuclear cell in blood containing ribonucleic acid from said subject and extracting said ribonucleic acid from the blood,

immobilizing at least one nucleic acid in said mononuclear cells onto a DNA micro-array or a DNA chip as a probe, the nucleic acid being selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia,

determining the expression level(s) of said nucleic acid(s) defining gene(s) exhibiting altered expression by occurrence of schizophrenia or nucleic acid(s) defining gene(s) exhibiting altered expression by progression of schizophrenia all together using said DNA micro-array or said DNA chip immobilized with the nucleic acid(s), and

comparing the determined level(s) with the determined level(s) of said nucleic acid(s) defining gene(s) exhibiting altered expression by occurrence of schizophrenia or said nucleic acid(s) defining gene(s) exhibiting altered expression by progression of schizophrenia in healthy subjects or in schizophrenic patients to determine whether the alteration of said determined level(s) in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) encoding enzyme(s) catalyzing transfer of phosphate group(s) (kinase or phosphatase) and defined by the gene name, the protein name which is a gene product, or the nucleic acid sequence name as described below in (1) to (16) with GenBank No described in brackets.

(1) Ndr protein kinase (Genbank No. Z35102)

(2) Protein-tyrosine kinase JAK1 (Genbank No. M64174)

(3) Inositol polyphosphate 4-phosphatase type I-beta (Genbank No. U96919)

(4) AMP-activated protein kinase alpha-1 (Genbank No. AB022017)

(5) Protein kinase C Nu (EPK2) (Genbank No. AB015982)

(6) MEK kinase (Mekk) (Genbank No. U29671)

(7) HSTXK Human tyrosine kinase (TXK) (Genbank No. U07794)

(8) Serine/threonine-protein kinase PRP4h (PRP4h) (Genbank No. U48736)

(9) Ribosomal protein S6 kinase (ISPK-1) (Genbank No. U08316)

(10) Human SNF1-like protein kinase (Genbank No. U57452)

(11) SH-PTP3 for protein-tyrosine phosphatase (Genbank No. D13540)

(12) Interferon-inducible RNA-dependent protein kinase (Pkr) (Genbank No. U50648)

(13) cAMP-dependent protein kinase catalytic subunit type alpha (EC 2.7.1.37) (Genbank No. X07767)

(14) PCTAIRE-1 for serine/threonine protein kinase (Genbank No. X66363)

(15) Branched chain alpha-ketoacid dehydrogenase kinase precursor (BCKD kinase) (Genbank No. AF026548)

(16) Phosphomevalonate kinase (Genbank No. L77213)

20. A method for diagnosing whether a test subject suffers from schizophrenia or not, the method comprising the steps of;

obtaining mononuclear cell in blood containing ribonucleic acid from said subject and extracting said ribonucleic acid from the blood,

immobilizing at least one nucleic acid in said mononuclear cells onto a DNA micro-array or a DNA chip as a probe, the nucleic acid being selected from the group consisting of nucleic acid(s) (containing its fragment and a nucleic acid complementary to the nucleic acid) defining gene(s) exhibiting altered expression by occurrence of schizophrenia or nucleic acid(s) (containing its fragment and a nucleic acid complementary to the nucleic acid) defining gene(s) exhibiting altered expression by progression of schizophrenia,

determining the expression level(s) of said nucleic acid(s) defining gene(s) exhibiting altered expression by occurrence of schizophrenia or nucleic acid(s) defining gene(s) exhibiting altered expression by progression of schizophrenia all together using said DNA micro-array or said DNA chip immobilized with the nucleic acid(s), and

comparing the determined level(s) with the determined level(s) of said nucleic acid(s) defining gene(s) exhibiting altered expression by occurrence of schizophrenia or said nucleic acid(s) defining gene(s) exhibiting altered expression by progression of schizophrenia in healthy subjects or in schizophrenic patients to determine whether the alteration of said determined level(s) in said test subject is statistically significant or not, thereby diagnosing whether said test subject suffers from schizophrenia or not,

wherein said nucleic acid(s) defining gene(s) exhibiting altered expression by occurrence of schizophrenia or said nucleic acid(s) defining gene(s) exhibiting altered expression by progression of schizophrenia is selected from nucleic acid(s) encoding enzyme(s) catalyzing transfer of phosphate group(s) (kinase or phosphatase) and defined by the gene name, the protein name which is a gene product, or the nucleic acid sequence name as described below in (1) to (16) with GenBank No described in brackets.

(1) Ndr protein kinase (Genbank No. Z35102)

(2) Protein-tyrosine kinase JAK1 (Genbank No. M64174)

(3) Inositol polyphosphate 4-phosphatase type I-beta (Genbank No. U96919)

(4) AMP-activated protein kinase alpha-1 (Genbank No. AB022017)

(5) Protein kinase C Nu (EPK2) (Genbank No. AB015982)

(6) MEK kinase (Mekk) (Genbank No. U29671)

(7) HSTXK Human tyrosine kinase (TXK) (Genbank No. U07794)

(8) Serine/threonine-protein kinase PRP4h (PRP4h) (Genbank No. U48736)

(9) Ribosomal protein S6 kinase (ISPK-1) (Genbank No. U08316)

(10) Human SNF1-like protein kinase (Genbank No. U57452)

(11) SH-PTP3 for protein-tyrosine phosphatase (Genbank No. D13540)

(12) Interferon-inducible RNA-dependent protein kinase (Pkr) (Genbank No. U50648)

(13) cAMP-dependent protein kinase catalytic subunit type alpha (EC 2.7.1.37) (Genbank No. X07767)

(14) PCTAIRE-1 for serine/threonine protein kinase (Genbank No. X66363)

(15) Branched chain alpha-ketoacid dehydrogenase kinase precursor (BCKD kinase) (Genbank No. AF026548)

(16) Phosphomevalonate kinase (Genbank No. L77213)

# FIG. 1

※ Accuracy of determination : 96.2%

FIG. 2

EP 1 548 129 A1

# FIG. 3

EP 1 548 129 A1

# INTERNATIONAL SEARCH REPORT

| International application No. |
|---|
| PCT/JP03/12361 |

| A. CLASSIFICATION OF SUBJECT MATTER |
|---|
| Int.Cl$^7$ C12Q1/68, G01N33/53 |

According to International Patent Classification (IPC) or to both national classification and IPC

| B. FIELDS SEARCHED |
|---|
| Minimum documentation searched (classification system followed by classification symbols) |
| Int.Cl$^7$ C12Q1/68, G01N33/53 |

Documentation searched other than minimum documentation to the extent that such documents are included in the fields searched

Electronic data base consulted during the international search (name of data base and, where practicable, search terms used)
BIOSIS, MEDLINE, WPIDS

## C. DOCUMENTS CONSIDERED TO BE RELEVANT

| Category* | Citation of document, with indication, where appropriate, of the relevant passages | Relevant to claim No. |
|---|---|---|
| X | JOHNSTON-WILSON, N.L. et al., Emerging techno logies for large-scale screening of human tissues and fluids in the study of severe psychiatric disease., Int.J.Neuropsychopharmacol., 2001, March, Vol.4(1), pages 83 to 92 | 2,3,9-13 |
| X | PRASAD, S. et al., Molecular genetics of schizo phrenia: past, present and future., J.Biosci., 2002, February, Vol.27(1 Suppl 1), pages 35 to 52 | 2,3,9-13 |
| A | MARCOTTE, E.R. et al., DNA microarrays in neuropsy chopharmacology., Trends.Pharmacol.Sci., 2001, August, Vol.22(8), pages 426 to 436 | 2,3,9-13 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 11 November, 2003 (11.11.03) | 25 November, 2003 (25.11.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**EP 1 548 129 A1**

International application No.

PCT/JP03/12361

C (Continuation).    DOCUMENTS CONSIDERED TO BE RELEVANT

| Category* | Citation of document, with indication, where appropriate, of the relevant passages | Relevant to claim No. |
|---|---|---|
| A | KAWANISHI, Y. et al., Pharmacogenomics and schizo phrenia., Eur.J.Pharmacol., 27 December, 2000 (27.12.00), Vol.410(2-3), pages 227 to 241 | 2,3,9-13 |

Form PCT/ISA/210 (continuation of second sheet) (July 1998)

**INTERNATIONAL SEARCH REPORT**

International application No.

PCT/JP03/12361

**Box I    Observations where certain claims were found unsearchable (Continuation of item 2 of first sheet)**

This international search report has not been established in respect of certain claims under Article 17(2)(a) for the following reasons:

1. ☒ Claims Nos.: 1, 4 to 8, 14 to 20

    because they relate to subject matter not required to be searched by this Authority, namely:

The inventions as set forth in claims 1, 4 to 8, 14 to 20 pertain to diagnostic methods to be practiced on the human body and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:

    because they relate to parts of the international application that do not comply with the prescribed requirements to such an extent that no meaningful international search can be carried out, specifically:

3. ☐ Claims Nos.:

    because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.

                       ☐   No protest accompanied the payment of additional search fees.

Form PCT/ISA/210 (continuation of first sheet (1)) (July 1998)